# EUROPÄISCHE PATENTSCHRIFT

(11) **EP 1 341 786 B1**
(45) Veröffentlichungstag und Bekanntmachung des Hinweises auf die Patenterteilung: **24.12.2008**
(21) Anmeldenummer: 01998547.2
(22) Anmeldetag: 28.11.2001
(51) Int. Cl.: C07D 409/12, C07D 407/12, C07D 405/12, C07D 307/68, C07D 307/42, A61K 31/443, A61K 31/4436, A61K 31/4155, A61K 31/341, C07D 307/54, C07D 333/24, A61K 31/44, C07D 333/28, C07D 307/56, A61P 29/02

(54) **SUBSTITUIERTE AMINO-FURAN-2-YL-ESSIGSAURE- UND AMINO-THIEN-2-YL-ESSIGSAURE-DERIVATE UND IHRE VERWENDUNG ZUR BEHANDLUNG VON MIGRAINE BZW. SCHMERZ**
SUBSTITUTED DERIVATIVES OF AMINOFURAN-2-YL-ACETIC ACID AND AMINOTHIEN-2-YL-ACETIC ACID AND THE USE THEREOF FOR TREATING MIGRAINES AND PAIN
DERIVES D'ACIDE AMINO-FURANNE-2-YL-ACETIQUE ET D'ACIDE AMINO-THIEN-2-YL-ACETIQUE SUBSTITUES ET LEUR UTILISATION POUR LE TRAITEMENT DE LA MIGRAINE ET DE LA DOULEUR

(30) Priorität: 30.11.2000 DE 10059864
(43) Veröffentlichungstag der Anmeldung: 10.09.2003
(73) Patentinhaber: Grünenthal GmbH, 52078 Aachen (DE)
(72) Erfinder: SANDERMANN, Corinna, 61381 Friedrichsdorf (DE); ENGLBERGER, Werner, 52223 Stolberg (DE); PRZEWOSNY, Michael, 52064 Aachen (DE)
(86) Internationale Anmeldenummer: PCT/EP2001/013910
(87) Internationale Veröffentlichungsnummer: WO 2002/044171

(56) Entgegenhaltungen:
- WO-A-01/96323
- WO-A-98/00398
- DATABASE CA [Online] CHEMICAL ABSTRACTS SERVICE, COLUMBUS, OHIO, US; PARK, YONG SUN ET AL: "Enantioselective Syntheses of.alpha.-,.beta.-, and.gamma.-Aryl Amino Acids and Esters" retrieved from STN Database accession no. 126:238630 XP002192099 & J. ORG. CHEM. (1997), 62(6), 1574-1575,

## Beschreibung

Die Erfindung betrifft substituierte Amino-furan-2-yl-essigsäure- und substituierte Amino-thien-2-yl-essigsäure-Derivate, Verfahren zu ihrer Herstellung, sie enthaltende Arzneimittel, Verwendung dieser Verbindungen zur Herstellung von Medikamenten zur Behandlung von u.a. Schmerz bzw. Migräne und sie enthaltende pharmazeutische Zusammensetzungen.

Die Behandlung chronischer und nicht chronischer Schmerzzustände hat in der Medizin eine große Bedeutung. Es besteht ein weltweiter Bedarf an gut wirksamen Therapien für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist.

Klassische Opioide wie Morphin sind bei der Therapie starker bis stärkster Schmerzen gut wirksam. Ihr Einsatz wird jedoch durch die bekannten Nebenwirkungen, wie z.B. Atemdepression, Erbrechen, Sedierung, Obstipation und Toleranzentwicklung, limitiert. Außerdem sind sie bei neuropathischen oder inzidentiellen Schmerzen, unter denen insbesondere Tumorpatienten leiden, weniger wirksam.

Opioide entfalten ihre analgetische Wirkung durch Bindung an zellmebranständige Rezeptoren, die zu der Familie der so genannten G - Protein-gekoppelten Rezeptoren gehören. Neben diesen gibt es weitere Rezeptoren sowie lonenkanäle, die wesentlich an dem System der Schmerzentstehung und der Schmerzweiterleitung beteiligt sind, beispielsweise der N-Methyl-D-Aspartat-Ionenkanal (NMDA-Ionenkanal), über den ein wesentlicher Teil der Kommunikation von Synapsen abläuft und durch den der Calcium-Ionenaustausch zwischen einer neuronalen Zelle und ihrer Umgebung gesteuert wird (s. z.B. P. D. Leeson, L. L. Iversen, J. Med. Chem. 37 (1994) 4053-4067).

Wichtige Erkenntnisse über die physiologische Bedeutung von ionenkanalselektiven Substanzen sind durch die Entwicklung der "patch-clamp"-Technik ermöglicht worden, mit deren Hilfe sich die Wirkung von NMDA-Antagonisten (d.h. Antagonisten des NMDA-Ionenkanals) auf den Calciumhaushalt im Zellinneren nachweisen läßt.

Der vorliegenden Erfindung liegt die Aufgabe zugrunde, neue Verbindungen, die sich für die Schmerztherapie eignen, zur Verfügung zu stellen. Darüber hinaus ist es wünschenswert, daß diese Substanzen jene Nebenwirkungen, die üblicherweise bei der Anwendung von Opioiden wie Morphin auftreten, wie z.B. Übelkeit, Erbrechen, Abhängigkeit, Atemdepression oder Obstipation, möglichst nicht oder nur in geringem Maße hervorrufen.

Diese Aufgabe wird gelöst durch Verbindungen der allgemeinen Struktur (I) sowie durch ihre pharmazeutisch annehmbaren Salze 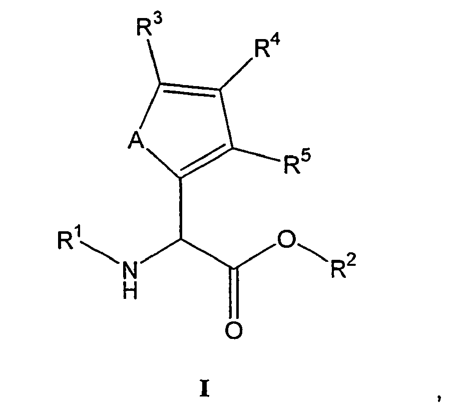 worin
- A: Sauerstoff oder Schwefel bedeutet,
- R¹: Aryl oder Heterocyclyl² bedeutet,
- R²: H, Methyl, Ethyl, n-Propyl, 2-Propyl, n-Butyl, tert-Butyl, n-Hexyl bedeutet,
- R³, R⁴ und R⁵: unabhängig voneinander H, OH, SH, Br, Cl, C₁₋₆-Alkyl, 2-Furyl, 3-Furyl, 2-Thienyl, 3-Thienyl, -SiR⁶R⁷R⁸, -CH₂OH, -CH₂-O-(C=O)-CH₃, -(CH₂)-Sₚ-(CH₂)_{q}-R¹⁰, p = 1 oder 2 und q = 0 oder 1, -(CH₂)ᵣ-CO₂R¹¹ mit r = 0 oder 1 oder -COR¹³ bedeuten,
- R⁶, R⁷ und R⁸: unabhängig voneinander Methyl, tert-Butyl oder Phenyl bedeuten,
- R¹⁰: H, Methyl, Ethyl, 2-Furyl, 2-Thienyl oder -(C=O)-CH₃ bedeutet,
- R¹¹: H, Methyl, Ethyl oder tert-Butyl bedeutet,
- R¹³: Methyl bedeutet,
- wobei Aryl: für 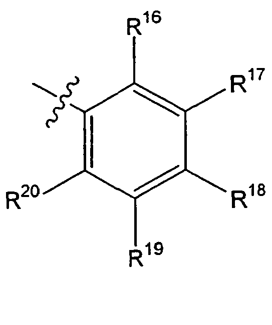 steht, Heterocyclyl² für 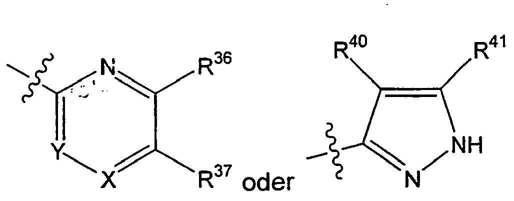 steht, R¹⁶, R¹⁷, R¹⁸, R¹⁹ und R²⁰ unabhängig voneinander H, OR²⁸, S(O)ₜR²⁹ mit t = 0, F, Cl, Br, I, -CN, -NO₂, Methyl, Ethyl, n-Propyl, 2-Propyl, n-Butyl, 2-Butyl, iso-Butyl, tert-Butyl, n-Pentyl, Neopentyl, Isopentyl, n-Hexyl, iso-Hexyl, CF₃ oder -CO₂R³¹ bedeuten,
- R²⁸, R²⁹ und R³¹: unabhängig voneinander H, Methyl, Ethyl, -CF₃ oder Phenyl bedeuten,
- X-Y: für CR³⁸-CR³⁹, CR³⁸-N oder N-CR³⁹ steht,
- R³⁶, R³⁷, R³⁸ und R³⁹: unabhängig voneinander H, F, Cl, Br, I, -CN, -NO₂, -OH, -SH, C₁₋₆-Alkyl oder -CF₃ bedeuten, und
- R⁴⁰ und R⁴¹: unabhängig voneinander H, F, Cl, Br, I, -CN, -OH, -O-C₁₋₆-Alkyl, -SH, -S-C₁₋₆-Alkyl, C₁₋₈-Alkyl, CO₂-C₁₋₆-Alkyl oder -N=N-Aryl bedeuten.

Demnach sind die erfindungsgemäßen Verbindungen der allgemeinen Struktur (I) entweder Amino-furan-2-yl-essigsäure-Derivate der allgemeinen Struktur (I-A) oder Amino-thien-2-yl-essigsäure-Derivate der allgemeinen Struktur (I-B):

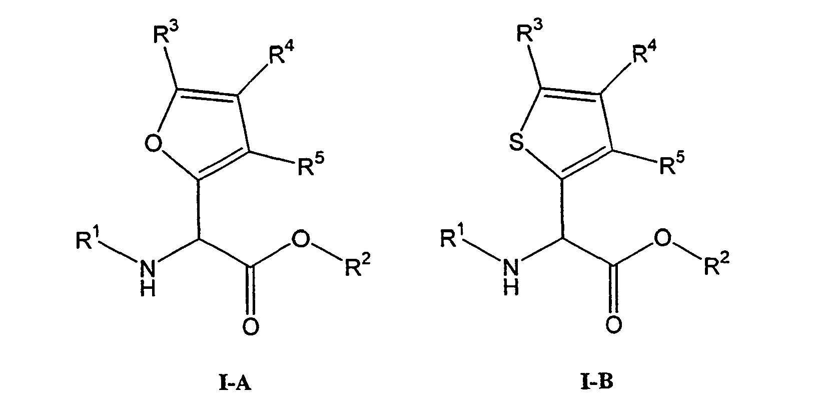

Es hat sich gezeigt, daß diese Verbindungen der allgemeinen Struktur (I-A) bzw. (I-B) selektiv an die Glycin-Bindungsstelle des NMDA-Ionenkanals binden und sich somit zur Behandlung von Schmerzzuständen eignen. Dies trifft auch auf die aus dem Stand der Technik als solche bekannte (4-Methoxy-phenylamino)-thien-2-yl-essigsäure (N. A. Petasis et al., Tetrahedron (1997), 16463-16470) zu, für die im Stand der Technik keine medizinische Indikation offenbart ist. Daher ist auch diese Verbindung Gegenstand der vorliegenden Erfindung, soweit ihre Verwendung in einem Arzneimittel, zur Herstellung eines Medikaments insbesondere zur Behandlung von Schmerz und eine sie enthaltende pharmazeutische Zusammensetzung betroffen sind.

Pharmazeutisch annehmbare Salze im Sinne dieser Erfindung sind solche Salze der erfindungsgemäßen Verbindungen gemäß der allgemeinen Struktur (I), die bei pharmazeutischer Verwendung physiologisch - insbesondere bei Anwendung am Säugetier und/oder Menschen - verträglich sind. Solche pharmazeutisch annehmbaren Salze können beispielsweise mit anorganischen oder organischen Säuren gebildet werden, oder für den Fall, daß die erfindungsgemäßen Verbindungen Säuren, insbesondere Carbonsäuren, sind, mit Basen gebildet werden.

Vorzugsweise werden die pharmazeutisch annehmbaren Salze der erfindungsgemäßen Verbindungen gemäß der allgemeinen Struktur (I) mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure gebildet. Handelt es sich bei den erfindungsgemäßen Verbindungen um Säuren, insbesondere Carbonsäuren, können die pharmazeutisch annehmbaren Salze auch durch Umsetzung mit Basen, wie z.B. Natriumhydrogencarbonat oder Natriumcarbonat, gebildet werden. Bei den gebildeten Salzen handelt es sich u.a. um Hydrochloride, Hydrobromide, Phosphate, Carbonate, Hydrogencarbonate, Formiate, Acetate, Oxalate, Succinate, Tartrate, Fumarate, Citrate und Glutaminate bzw. um Natrium-Salze. Ebenfalls bevorzugt sind Solvate und insbesondere die Hydrate der erfindungsgemäßen Verbindungen, die z.B. durch Kristallisation aus wäßriger Lösung erhalten werden können.

Die erfindungsgemäßen Verbindungen der allgemeinen Struktur (I) weisen stets mindestens ein Asymmetriezentrum auf, das in der untenstehenden Formel mit * gekennzeichnet ist:

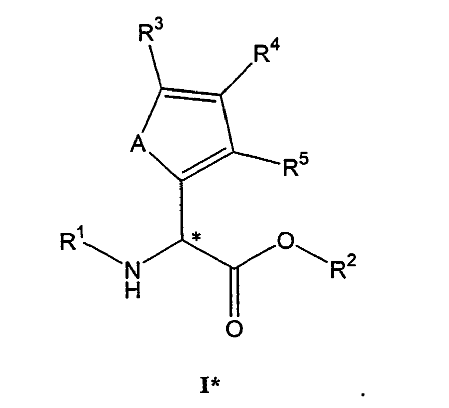

Die erfindungsgemäßen Verbindungen der allgemeinen Formel (I) können daher in Form ihrer Racemate, in Form der reinen Enantiomeren und/oder - sofern ein weiteres Asymmetriezentrum vorliegt - in Form der reinen Diastereomeren oder in Form von Mischungen dieser Enantiomeren bzw. Diastereomeren vorliegen, und zwar sowohl in Substanz als auch als pharmazeutisch annehmbare Salze dieser Verbindungen. Die Mischungen können in jedem beliebigen Mischungsverhältnis der Stereoisomeren vorliegen.

Ganz besonders bevorzugte Verbindungen der allgemeinen Struktur (I) sind jene, die dadurch gekennzeichnet sind, daß
- R¹: Aryl oder Heterocyclyl² bedeutet,
- R²: H, Methyl, Ethyl oder tert-Butyl bedeutet,
- R³: H, Cl, Methyl, Ethyl, n-Propyl, 2-Propyl, n-Butyl, tert-Butyl, -CH₂OH, -CH₂SH, -CH₂-S-CH₃, -CH₂-S-CH₂-Furan-2-yl, -CH₂-O-(C=O)-CH₃, -CH₂-S-(C=O)-CH₃, -CH₂-S-S-CH₃, -CH₂-S-S-CH₂-Furan2-yl, -CH₂-CO₂Methyl oder -CH₂-CO₂Ethyl bedeutet,
- R⁴: H, Br, Methyl, Ethyl, -CH₂OH, -CO₂Methyl, -CO₂Ethyl oder - COMethyl bedeutet,
- R⁵: H, Methyl oder Ethyl bedeutet,
- wobei Aryl: für 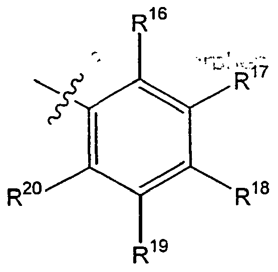 steht,
- Heterocyclyl²: für 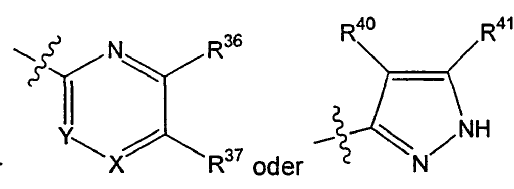 steht,
- R¹⁶: Butyl H, -O-Phenyl, F, Cl, Br, Methyl, Ethyl, n-Propyl, 2-Propyl oder tert-bedeutet,
- R¹⁷: H, Cl, Methyl, Ethyl oder CF₃ bedeutet,
- R¹⁸: Propyl, H, F, Cl, Br, I, -CN, -O-CH₃, -O-CF₃, -O-Phenyl, Methyl, Ethyl, n-2-Propyl, n-Butyl, 2-Butyl oder tert-Butyl bedeutet,
- R¹⁹: H, Cl, Br, Methyl oder Ethyl bedeutet,
- R²⁰: H oder Methyl bedeutet,
- X-Y: für CR³⁸-CR³⁹, CR³⁸-N oder N-CR³⁹ steht,
- R³⁶: H, Methyl oder Ethyl bedeutet
- R³⁷: H, NO₂, Cl, Br, Methyl oder CF₃ bedeutet;
- R³⁸: H bedeutet,
- R³⁹: H, Cl oder Br bedeutet,
- R⁴⁰: und H, -N=N-Phenyl, -CN, CO₂H, CO₂-Methyl oder CO₂-Ethyl bedeutet,
- R⁴¹: H, OH, SH, S-Methyl, Methyl, Ethyl, n-Propyl, 2-Propyl, n-Butyl oder tert-Butyl bedeutet.

Unter diesen ganz besonders bevorzugten Verbindungen sind solche insbesondere bevorzugt, in denen
- R¹: 4-Trifluormethoxy-phenyl, 2-Phenoxy-phenyl, 4-Phenoxy-phenyl, 2-Chlor-phenyl, 4-Chlor-phenyl, 4-Iod-phenyl, 4-Cyano-phenyl, 2-Methylphenyl, 2-Ethyl-phenyl, 4-Ethyl-phenyl, 2-(2-Propyl)-phenyl, 4-(2-Butyl)-phenyl, 4-tert-Butyl-phenyl, 2,4-Dichlorphenyl, 2,3-Dichlorphenyl, 3,5-Dichlorphenyl, 2,4-Dibrom-phenyl, 4-Chlor-2-fluor-phenyl, 2-Chlor-4-fluorphenyl, 4-Brom-2-chlor-phenyl, 2-Chlor-4-Iod-phenyl, 3-Chlor-2-methylphenyl, 4-Chlor-2-methyl-phenyl, 5-Chlor-2-methyl-phenyl, 2-Chlor-4-methyl-phenyl, 4-Chlor-3-trifluormethyl-phenyl, 2,4-Dibrom-5-methyl-phenyl, 5-Nitro-pyridin-2-yl, 3,5-Dibrom-pyridin-2-yl, 3,5-Dichlor-pyridin-2-yl, 3-Chlor-5-Trifluormethyl-pyridin-2-yl, 3,5-Dibrom-6-methyl-pyridin-2-yl, Pyrazin-2-yl, 5-Brom-pyrimidin-2-yl, 4-Carboxyethyl-pyrazol-3-yl, 4-Cyano-pyrazol-3-yl, 5-tert-Butyl-pyrazol-3-yl, 5-Hydroxy-4-(4-Phenylazo)-pyrazol-3-yl oder 4-Cyano-5-thiomethyl-pyrazol-3-yl bedeutet,
- R²: H oder Ethyl bedeutet,
- R³: H, Cl, Methyl, Ethyl, n-Propyl, tert-Butyl, -CH₂OH, -CH₂SH, -CH₂S-CH₃, -CH₂-S-CH₂-Furan-2-yl, -CH₂-O-(C=O)-CH₃, -CH₂-S-(C=O)-CH₃, - CH₂-S-S-CH₃, -CH₂-S-S-CH₂-Furan2-yl oder -CH₂-CO₂Ethyl bedeutet,
- R⁴: H, Br, Methyl, -CH₂OH, -CO₂Methyl, -CO₂Ethyl oder -C(=O)CH₃ bedeutet und
- R⁵: H oder Methyl bedeutet.

Außerordentlich bevorzugte erfindungsgemäße Verbindungen sind solche, in denen R¹ 3,5-Dichlorphenyl bedeutet und R² H bedeutet.

Beispielhafte und vorteilhafte Verbindungen der vorliegenden Erfindung sind aus der Gruppe ausgewählt, die
- (5-Methylsulfanylmethyl-furan-2-yl)-(5-nitro-pyridin-2-ylamino)-essigsäure
- (5-Brompyrimidin-2-ylamino)-(5-methylsulfanylmethyl-furan-2-yl)-essigsäure
- 5-[Carboxy-(3,5-dichlorpyridin-2-ylamino)-methyl]-2-methyl-furan-3-carbonsäureethylester
- 5-[Carboxy-(3,5-dibrompyridin-2-ylamino)-methyl]-2-methyl-furan-3-carbonsäureethylester
- 5-[Carboxy-(3,5-dibrom-6-methyl-pyridin-2-ylamino)-methyl]-2-methyl-furan-3-carbonsäureethylester
- (3,5-Dibrom-6-methyl-pyridin-2-ylamino)-(4-hydroxymethyl-furan-2-yl)-essigsäure
- (3,5-Dichlor-pyridin-2-ylamino)-(4-methyl-thiophen-2-yl)-essigsäure
- (2,4-Dibrom-5-methyl-phenylamino)-(4-methyl-thiophen-2-yl)-essigsäure
- (4-Methyl-thiophen-2-yl)-(5-nitro-pyridin-2-ylamino)-essigsäure
- (3,5-Dichlor-pyridin-2-ylamino)-furan-2-yl-essigsäure
- (3,5-Dibrompyridin-2-ylamino)-furan-2-yl-essigsäure
- (3,5-Dibrom-6-methyl-pyridin-2-ylamino)-furan-2-yl-essigsäure
- (3-Chlor-5-trifluormethyl-pyridin-2-ylamino)-furan-2-yl-essigsäure
- (5-Brompyrimidin-2-ylamino)-furan-2-yl-essigsäure
- 5-[(3,5-Dichlor-phenylamino)-ethoxycarbonyl-methyl]-2-methyl-furan-3-carbonsäuremethylester
- (5-Hydroxy-4-phenylazo-1H-pyrazol-3-ylamino)-(5-methylsulfanylmethyl-furan-2-yl)-essigsäureethylester
- 3-{[Ethoxycarbonyl-(4-ethoxycarbonyl-5-methyl-furan-2-yl)-methyl]-amino}-1H-pyrazol-4-carbosäureethylester
- 5-[(4-Cyano-5-methylsulfanyl-1H-pyrazol-3-ylamino)-ethoxycarbonyl-methyl]-2-methyl-furan-3-carbonsäureethylester
- 5-[(4-Cyano-1H-pyrazol-3-ylamino)-ethoxycarbonyl-methyl]-2-methyl-furan-3-carbonsäureethylester
- 5-[(4-Brom-2-chlor-phenylamino)-ethoxycarbonyl-methyl]-2-methyl-furan-3-carbonsäureethylester
- 5-[(4-Cyano-phenylamino)-ethoxycarbonyl-methyl]-2-methyl-furan-3-carbonsäureethylester
- (4-Hydroxymethyl-furan-2-yl)-(5-hydroxy-4-phenylazo-1H-pyrazol-3-ylamino)-essigsäureethylester
- (4-Cyano-5-methylsulfanyl-1H-pyrazol-3-ylamino)-(4-hydroxymethyl-furan-2-yl)-essigsäureethylester
- (4-Brom-2-chlor-phenylamino)-(4-hydroxymethyl-furan-2-yl)-essigsäureethylester
- (3,5-Dichlor-phenylamino)-(4-hydroxymethyl-furan-2-yl)-essigsäureethylester
- (5-Chlor-2-methyl-phenylamino)-[5-(furan-2-ylmethylsulfanylmethyl)-furan-2-yl]-essigsäureethylester
- (2,4-Dibrom-phenylamino)-(4-methyl-thiophen-2-yl)-essigsäure
- (5-Chlor-2-methyl-phenylamino)-(5-methylsulfanylmethyl-furan-2-yl)-essigsäure
- (2-Ethyl-phenylamino)-(5-methylsulfanylmethyl-furan-2-yl)-essigsäure
- (4-sec-Butyl-phenylamino)-(5-methylsulfanylmethyl-furan-2-yl)-essigsäure
- (5-Methylsulfanylmethyl-furan-2-yl)-(4-trifluormethoxy-phenylamino)-essigsäure
- (2-Isopropyl-phenylamino)-(5-methylsulfanylmethyl-furan-2-yl)-essigsäure
- (2,4-Dibrom-phenylamino)-(5-methylsulfanylmethyl-furan-2-yl)-essigsäure
- (4-*tert*-Butyl-phenylamino)-(5-methylsulfanylmethyl-furan-2-yl)-essigsäure
- (5-Chlor-2-methyl-phenylamino)-[5-(furan-2-ylmethylsulfanylmethyl)-furan-2-yl]-essigsäure
- (2-Ethyl-phenylamino)-[5-(furan-2-ylmethylsulfanylmethyl)-furan-2-yl]-essigsäure
- (4-sec-Butyl-phenylamino)-[5-(furan-2-ylmethylsulfanylmethyl)-furan-2-yl]-essigsäure
- [5-(Furan-2-ylmethylsulfanylmethyl)-furan-2-yl]-(2-isopropyl-phenylamino)-essigsäure
- (4-*tert*-Butyl-phenylamino)-[5-(furan-2-ylmethylsulfanylmethyl)-furan-2-yl]-essigsäure
- 5-[Ethoxycarbonyl-(4-iod-phenylamino)-methyl]-2-methyl-furan-3-carbonsäuremethylester
- 5-[(4-Chlor-2-methyl-phenylamino)-ethoxycarbonyl-methyl]-2-methyl-furan-3-carbonsäuremethylester
- 5-[Ethoxycarbonyl-(4-phenoxy-phenylamino)-methyl]-2-methyl-furan-3-carbonsäuremethylester
- 5-[Ethoxycarbonyl-(4-iod-phenylamino)-methyl]-2-methyl-furan-3-carbonsäureethylester
- 5-[(2-Chlor-phenylamino)-ethoxycarbonyl-methyl]-2-methyl-furan-3-carbonsäureethylester
- 5-[(4-Chlor-2-methyl-phenylamino)-ethoxycarbonyl-methyl]-2-methyl-furan-3-carbonsäureethylester
- 5-[(2-Chlor-4-fluor-phenylamino)-ethoxycarbonyl-methyl]-2-methyl-furan-3-carbonsäureethylester
- 5-[Ethoxycarbonyl-(4-phenoxy-phenylamino)-methyl]-2-methyl-furan-3-carbonsäureethylester
- 5-[(2,3-Dichlor-phenylamino)-ethoxycarbonyl-methyl]-2-methyl-furan-3-carbonsäureethylester
- (4-Hydroxymethyl-furan-2-yl)-(4-iod-phenylamino)-essigsäureethylester
- (2,4-Dichlor-phenylamino)-(4-hydroxymethyl-furan-2-yl)-essigsäureethylester
- (4-Chlor-2-methyl-phenylamino)-(4-hydroxymethyl-furan-2-yl)-essigsäureethylester
- (4-Hydroxymethyl-furan-2-yl)-(4-phenoxy-phenylamino)-essigsäureethylester
- (2,3-Dichlor-phenylamino)-(4-hydroxymethyl-furan-2-yl)-essigsäureethylester
- (2,3-Dichlor-phenylamino)-furan-2-yl-essigsäureethylester
- 5-[Carboxy-(3,5-dibrom-6-methyl-pyridin-2-ylamino)-methyl]-2-methyl-furan-3-carbonsäuremethylester
- (5-Methylsulfanylmethyl-furan-2-yl)-(pyrazin-2-ylamino)-essigsäure
- (3,5-Dibrom-pyridin-2-ylamino)-(5-methylsulfanylmethyl-furan-2-yl)-essigsäure
- (3,5-Dibrom-6-methyl-pyridin-2-ylamino)-(5-methylsulfanylmethyl-furan-2-yl)-essigsäure
- 5-[(4-Brom-2-chlor-phenylamino)-carboxy-methyl]-2-methyl-furan-3-carbonsäuremethylester
- 5-[Carboxy-(4-cyano-phenylamino)-methyl]-2-methyl-furan-3-carbonsäuremethylester
- 5-[Carboxy-(3,5-dichlor-phenylamino)-methyl]-2-methyl-furan-3-carbonsäuremethylester
- 5-[Carboxy-(2-phenoxy-phenylamino)-methyl]-2,4-dimethyl-furan-3-carbonsäureethylester
- 5-[(4-Brom-2-chlor-phenylamino)-carboxy-methyl]-2,4-dimethyl-furan-3-carbonsäureethylester
- 5-[Carboxy-(4-cyano-phenylamino)-methyl]-2,4-dimethyl-furan-3-carbonsäureethylester
- 5-[Carboxy-(3,5-dichlor-phenylamino)-methyl]-2,4-dimethyl-furan-3-carbonsäureethylester
- 5-[(4-Brom-2-chlor-phenylamino)-carboxy-methyl]-2-methyl-furan-3-carbonsäureethylester
- 5-[Carboxy-(4-cyano-phenylamino)-methyl]-2-methyl-furan-3-carbonsäureethylester
- 5-[Carboxy-(3,5-dichlor-phenylamino)-methyl]-2-methyl-furan-3-carbonsäureethylester
- (5-*tert*-Butyl-1H-pyrazol-3-ylamino)-(4-hydroxymethyl-furan-2-yl)-essigsäure
- (4-Brom-2-chlor-phenylamino)-(3-methyl-thiophen-2-yl)-essigsäure
- (4-Cyano-phenylamino)-(3-methyl-thiophen-2-yl)-essigsäure
- (3,5-Dichlor-phenylamino)-(3-methyl-thiophen-2-yl)-essigsäure
- (3,5-Dichlor-phenylamino)-furan-2-yl-essigsäure
- [5-(Furan-2-ylmethylsulfanylmethyl)-furan-2-yl]-(4-phenoxy-phenylamino)-essigsäureethylester
- (5-Ethoxycarbonylmethyl-thiophen-2-yl)-(5-hydroxy-4-phenylazo-1H-pyrazol-3-ylamino)-essigsäureethylester
- {5-[1-(4-Cyano-1H-pyrazol-3-ylamino)-3-methoxy-2-oxo-propyl]-thiophen-2-yl}-essigsäureethylester
- (4-Ethyl-phenylamino)-(5-methylsulfanylmethyl-furan-2-yl)-essigsäure
- (3-Chlor-2-methyl-phenylamino)-(5-methylsulfanylmethyl-furan-2-yl)-essigsäure
- (4-Chlor-phenylamino)-(5-methylsulfanylmethyl-furan-2-yl)-essigsäure
- (5-Methylsulfanylmethyl-furan-2-yl)-o-tolylamino-essigsäure
- 5-[Carboxy-(4-chlor-3-trifluormethyl-phenylamino)-methyl]-2-methyl-furan-3-carbonsäureethylester
- 5-[Carboxy-(2-chlor-phenylamino)-methyl]-2-methyl-furan-3-carbonsäureethylester
- 5-[Carboxy-(2-chlor-4-fluor-phenylamino)-methyl]-2-methyl-furan-3-carbonsäureethylester
- 5-[Carboxy-(4-chlor-2-fluor-phenylamino)-methyl]-2-methyl-furan-3-carbonsäureethylester
- 5-[Carboxy-(2,3-dichlor-phenylamino)-methyl]-2-methyf-furan-3-carbonsäureethylester
- (2,4-Dichlor-phenylamino)-(4-methyl-thiophen-2-yl)-essigsäure
- (4-Chlor-3-trifluormethyl-phenylamino)-(4-methyl-thiophen-2-yl)-essigsäure
- (2,4-Dichlor-phenylamino)-furan-2-yl-essigsäure
- (4-Chlor-3-trifluormethyl-phenylamino)-furan-2-yl-essigsäure
- (4-lod-phenylamino)-(5-methylsulfanylmethyl-furan-2-yl)-essigsäure
- (2,4-Dichlor-phenylamino)-(5-methylsulfanylmethyl-furan-2-yl)-essigsäure
- (4-Chlor-3-trifluormethyl-phenylamino)-(5-methylsulfanylmethyl-furan-2-yl)-essigsäure
- (2-Chlor-phenylamino)-(5-methylsulfanylmethyl-furan-2-yl)-essigsäure
- (4-Chlor-2-methyl-phenylamino)-(5-methylsulfanylmethyl-furan-2-yl)-essigsäure
- (2-Chlor-4-fluor-phenylamino)-(5-methylsulfanylmethyl-furan-2-yl)-essigsäure
- (2-Chlor-4-methyl-phenylamino)-(5-methylsulfanylmethyl-furan-2-yl)-essigsäure
- (2,3-Dichlor-phenylamino)-(5-methylsulfanylmethyl-furan-2-yl)-essigsäure
- [5-(Furan-2-ylmethylsulfanylmethyl)-furan-2-yl]-(4-iodo-phenylamino)-essigsäure
- (4-Chlor-3-trifluormethyl-phenylamino)-[5-(furan-2-ylmethylsulfanylmethyl)-furan-2-yl]-essigsäure
- (4-Chlor-2-methyl-phenylamino)-[5-(furan-2-ylmethylsulfanylmethyl)-furan-2-yl]-essigsäure
- (2-Chlor-4-methyl-phenylamino)-[5-(furan-2-ylmethylsulfanylmethyl)-furan-2-yl]-essigsäure
- (4-Chlor-3-trifluormethyl-phenylamino)-(5-ethoxycarbonylmethyl-thiophen-2-yl)-essigsäure
- (4-Acetyl-3,5-dimethyl-furan-2-yl)-(2-chlor-4-methyl-phenylamino)-essigsäure
- (4-Acetyl-3,5-dimethyl-furan-2-yl)-(2,3-dichlor-phenylamino)-essigsäure
- (3,5-Dichlor-phenylamino)-(5-methylsulfanylmethyl-furan-2-yl)-essigsäure
- [2,2']Bithiophenyl-5-yl-(3,5-dichlor-phenylamino-essigsäure
- (3,5-Dichlor-phenylamino)-(5-methyldisulfanylmethyl-furan-2-yl)-essigsäure
- (3-Chlor-phenylamino)-(5-mercaptomethyl-furan-2-yl)-essigsäure
- (3,4-Dichlor-phenylamino)-(5-mercaptomethyl-furan-2-yl)-essigsäure
- (3,5-Dichlor-phenylamino)-(5-mercaptomethyl-furan-2-yl)-essigsäure
- (3,5-Dichlor-phenylamino)-(5-methyl-thiophen-2-yl)-essigsäure
- (3,5-Dichlor-phenylamino)-(5-hydroxymethyl-thiophen-2-yl)-essigsäure
- (5-Acetylsulfanylmethyl-furan-2-yl)-(3,5-dichlor-phenylamino)-essigsäure
- (3,5-Dichlor-phenylamino)-(5-ethyl-thiophen-2-yl)-essigsäure
- (3,5-Dichlor-phenylamino)-(5-n-propyl-thiophen-2-yl)-essigsäure
- (3,5-Dichlor-phenylamino)-[5-(furan-2-ylmethylsulfanylmethyl)-furan-2-yl]-essigsäure
- (3-Chlor-phenylamino)-(5-mercaptomethyl-furan-2-y!)-essigsäure
- (3,4-Dichlor-phenylamino)-(5-mercaptomethyl-furan-2-yl)-essigsäure
- (3,5-Dichlorphenylamino)-(thiophen-2-yl)-essigsäureethylester
- (4-Bromfuran-2-yl-(3,5-dichlor-phenylamino)-essigsäureethylester
- (3,5-Dichlor-phenylamino)-(5-propylthiophen-2-yl)-essigsäureethylester
- (3,5-Dichlor-phenylamino)-(3-methylthiophen-2-yl)-essigsäureethylester
- (5-tert-Butylfuran-2-yl)-(3,5-dichlor-phenylamino)-essigsäure
- (3,5-Dichlorphenylamino)-[5-(furan-2-ylmethyldisulfanylmethyl)-furan-2-yl]-essigsäure
- (3,5-Dichlorphenylamino)-(5-methyldisulfanylmethylfuran-2-yl)-essigsäure
- (3,5-Dichlorphenylamino)-[5-(furan-2-ylmethylsulfanylmethyl)-furan-2-yl]-essigsäure
- 5-Acetoxymethylfuran-2-yl-(3,5-dichlorphenylamino)-essigsäure
- (3,5-Dichlorphenylamino)-thiophen-3-yl-essigsäure
- (3,5-Dichlorphenylamino)-(5-methylthiophen-2-yl)-essigsäure
- (3,5-Dichlorphenylamino)-(4-methylthiophen-2-yl)-essigsäure
- (5-Chlorthiophen-2-yl)-(3,5-dichlorphenylamino)-essigsäure
sowie deren pharmazeutisch annehmbare Salze enthält.

Die erfindungsgemäßen Verbindungen der allgemeinen Struktur (I) 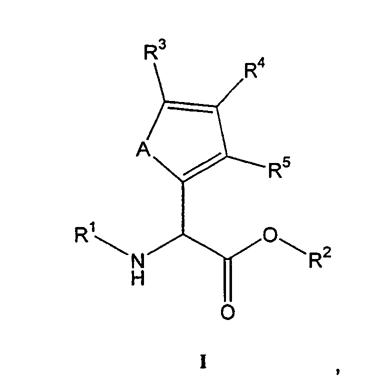 worin A, R¹, R², R³, R⁴ und R⁵ die oben definierten Bedeutungen haben, sind nach einem Verfahren zugänglich, das ebenfalls ein Gegenstand der vorliegenden Erfindung und dadurch gekennzeichnet ist, daß ein Amin der allgemeinen Struktur (II)

R¹-NH² II,

worin R¹ wie oben für die allgemeine Struktur (I) definiert ist, mit einem Glyoxylsäurederivat der allgemeinen Struktur (III) 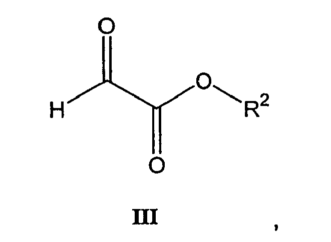 worin R² wie oben für die allgemeine Struktur (I) definiert ist,
und einem Furan der allgemeinen Struktur (IV-A) oder einem Thiophen der allgemeinen Struktur (IV-B) 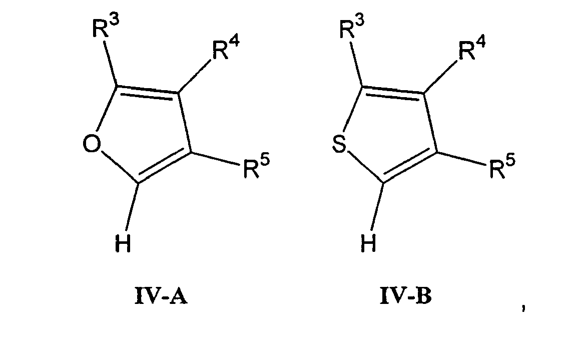 worin R³, R⁴ und R⁵ wie oben für die allgemeine Struktur (I) definiert sind, unter Einwirken einer Säure umgesetzt wird. Wird das Furan-Derivat (IV-A) eingesetzt, erhält man als Produkt das entsprechende Amino-furan-2-yl-essigsäure-Derivat der allgemeinen Struktur (I-A), wird hingegen das Thiophen-Derivat (IV-B) eingesetzt, erhält man als Produkt der erfindungsgemäßen 3-Komponenten-Reaktion das entsprechende Amino-thien-2-yl-essigsäure-Derivat der allgemeinen Struktur (I-B).

Für die Herstellung der Furanylessigsäuren (IV-A) und Thienylessigsäuren (IV-B), also den Verbindungen der allgemeinen Struktur (IV), für die R² H bedeutet, wird die Reaktionskomponente Glyoxylsäure (III) bevorzugt in Form ihres Hydrats eingesetzt.

Das erfindungsgemäße Verfahren kann in Gegenwart geringer Mengen einer anorganischen oder insbesondere organischen Säure, z.B. Trifluoressigsäure, bevorzugt in katalytischen Mengen von etwa 1-10 Mol% (bezogen auf das Edukt (II)), ausgeführt werden. Wird als GlyoxylsäureDerivat der allgemeinen Struktur (III) die Glyoxylsäure selbst (d.h. die Verbindung (III) mit R² = H) bzw. ihr Hydrat in geringem Überschuß von ca. 1,01 bis 1,5, bevorzugt 1,05 bis 1,25 Mol-Äquivalenten (bezogen auf die Verbindung (II)) eingesetzt, kann die Umsetzung auch ohne Zusatz einer Säure oder eines weiteren Reagenzes durch bloßes Mischen der Ausgangsverbindungen (II), (III) und (IV-A) bzw. (IV-B), bevorzugt in einem organischen Lösungsmittel, z.B. Acetonitril, und anschließendes Rühren bei Temperaturen zwischen 0 °C und 100 °C - ggf. auch als Eintopf-Verfahren - ausgeführt werden. Ebenfalls vorteilhaft ist es, das Furan-Derivat (IV-A) bzw. Thiophen-Derivat (IV-B) im Überschuß von beispielsweise 1,5 bis 4,5 Mol-Aquivalenten, insbesondere 2,5 bis 3,5 Mol-Äquivalenten (bezogen auf Edukt (II)), einzusetzen.

Um eine möglichst vollständige Umsetzung der Edukte zu den erfindungsgemäßen Verbindungen der allgemeinen Struktur (I) zu erreichen, ist für die erfindungsgemäße 3-Komponenten-Reaktion eine Reaktionszeit von 8 h bis etwa 18 h, insbesondere 14 h, zweckmäßig.

Alternativ kann die erfindungsgemäße 3-Komponenten-Reaktion auch unter Einwirken von Mikrowellenstrahlung ausgeführt werden, womit sich die Reaktionszeit für eine weitgehend vollständige Umsetzung auf wenige Minuten, z.B. auf 0,5 min bis 5 min, verkürzt. Bei dieser Verfahrensführung wird die Reaktionstemperatur vorzugsweise bei 15 °C bis 60 °C, insbesondere ca. 50 °C, gewählt. Für die Mikrowellen-Bestrahlung eignet sich beispielsweise eine Labor-Mikrowelle der Firma MLS-GmbH (D-88299 Leutkirch, Auenweg 37, Deutschland), Modell MLS ETHOS 600 mit einer Leistung von ca. 800 W. Zweckmäßig erfolgt die Umsetzung bespielsweise in einem druckstabilen Teflongefäß.

Das erfindungsgemäße Verfahren kann auch in semi- oder vollautomatisierter Form als Parallelsynthese einer Gruppe von erfindungsgemäßen Verbindungen der allgemeinen Struktur (I) durchgeführt werden. Mit Hilfe dieser Technik und durch Umsetzung der Verbindungen (II), (III) und (IV-A) bzw. (IV-B) läßt sich auch eine Substanzbibliothek in Gestalt eines "array of compounds" aufbauen. Diese Substanzbibliothek enthält die Bibliotheksmitglieder, die die Reaktionsprodukte der Umsetzung der Verbindungen (II), (III) und (IV-A) bzw. (IV-B) sind, als einzelne reine Verbindungen. Mit Hilfe dieser Substanzbibliothek kann beispielsweise ein medizinisches Screening in einem oder mehreren in-vitro-Screening-Verfahren in automatisierter Form durchgeführt werden.

Die im erfindungsgemäßen Verfahren eingesetzten Amine der allgemeinen Struktur (II), die Glyoxylsäure-Derivate der allgemeinen Struktur (III), die Furan-Derivate der allgemeinen Struktur (IV-A) und die Thiophen-Derivate der allgemeinen Struktur (IV-B) sind kommerziell erhältlich (von Acros, Geel; Avocado, Port of Heysham; Aldrich, Deisenhofen; Fluka, Seelze; Lancaster, Mülheim; Maybridge, Tintagel; Merck, Darmstadt; Sigma, Deisenhofen; TCI, Japan) oder können nach im Stand der Technik allgemein bekannten Verfahren hergestellt werden.

Die erfindungsgemäßen Verbindungen der allgemeinen Struktur (I) können sowohl in Substanz als auch als Salz isoliert werden. Die erfindungsgemäße Verbindung der allgemeinen Struktur (I) wird üblicherweise nach erfolgter Umsetzung gemäß dem oben beschriebenen erfindungsgemäßen Verfahren und anschließender herkömmlicher Aufarbeitung erhalten. Die so gewonnene oder in-situ ohne Isolierung gebildete Verbindung der allgemeinen Struktur (I) kann dann beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in das korrespondierende Salz überführt werden. Soweit es sich bei den erfindungsgemäßen Verbindungen der allgemeinen Formel (I) um Säuren, insbesondere Carbonsäuren handelt, kann die Salzbildung durch Zugabe einer physiologisch verträglichen Base, z.B. NaHCO₃ oder Natriumcarbonat, herbeigeführt werden; für die Carbonsäuren ist insbesondere die Bildung des Natriumsalzes bevorzugt. Bei den gebildeten Salzen handelt es sich u.a. um Hydrochloride, Hydrobromide, Phosphate, Carbonate, Hydrogencarbonate, Formiate, Acetate, Oxalate, Succinate, Tartrate, Fumarate, Citrate und Glutaminate. Die besonders bevorzugte Hydrochloridbildung kann auch durch Versetzen der in einem geeigneten organischen Lösungsmittel, wie z.B. Butan-2-on (Methylethylketon), gelösten Base mit Trimethylsilylchlorid (TMSCI), vorteilhaft in Gegenwart von Wasser, herbeigeführt werden.

Soweit die Verbindungen der allgemeinen Struktur (I) in dem erfindungsgemäßen Herstellungsverfahren als Racemate oder als Mischungen ihrer verschiedenen Enantiomeren und/oder Diastereomeren erhalten werden, können diese Mischungen nach im Stand der Technik wohlbekannten Verfahren aufgetrennt werden. Geeignete Methoden sind u.a. chromatographische Trennverfahren, insbesondere Flüssigkeitschromatographie-Verfahren unter Normal- oder erhöhtem Druck, bevorzugt MPLC- und HPLC-Verfahren, sowie Verfahren der fraktionierten Kristallisation. Dabei können insbesondere einzelne Enantiomeren z.B. mittels HPLC an chiraler Phase oder mittels Kristallisation von mit chiralen Säuren, etwa (+)-Weinsäure, (-)-Weinsäure oder (+)-10-Camphersulfonsäure, oder - sofern es sich um Säuren handelt - mit chiralen Basen, etwa Brucin oder (-)-Ephedrin, gebildeten diastereomeren Salzen voneinander getrennt werden.

Ein weiterer Gegenstand der vorliegenden Erfindung ist ein Arzneimittel, enthaltend mindestens eine wie oben definierte Verbindung der allgemeinen Struktur (I), oder eines ihrer pharmazeutischen Salze, insbesondere das Hydrochlorid-Salz. Vorzugsweise enthält das erfindungsgemäße Arzneimittel mindestens eine der oben beispielhaft genannten Verbindungen in Substanz oder als pharmazeutisch annehmbares Salz und gegebenenfalls weitere Wirk- und Hilfsstoffe.

Diese erfindungsgemäßen Arzneimittel werden vorzugsweise in der Therapie von Schmerzzuständen, wie z.B. akuter Schmerz, chronischer Schmerz bzw. neuropathischer Schmerz, eingesetzt. Es hat sich auch gezeigt, daß die erfindungsgemäßen Arzneimittel auch zur Behandlung von Migräne, inflammatorischen und/oder allergischen Reaktionen, Depressionen, Drogen- und/oder Alkoholmißbrauch, Gastritis, Diarrhoe, Harninkontinenz, cardiovaskulären Erkrankungen, Atemwegserkrankungen, Husten, seelischen Erkrankungen, neurodegenerativen Erkrankungen, Epilepsie, Schizophrenie, Morbus Alzheimer, Morbus Huntington, Morbus Parkinson, cerebralen Ischämien, cerebralen Infarkten, durch erhöhten Aminosäurespiegel bedingten Psychosen, Schlaganfällen, Hirnödemen, Unterversorgungszuständen des zentralen Nervensystems, Hypoxie, Anoxie, AIDS-Demens, Encephalomyelitis, Tourette-Syndrom, perinataler Asphyxie oder zur Anxiolyse erfolgreich eingesetzt werden können.

Ein weiterer Gegenstand der vorliegenden Anmeldung ist die Verwendung von mindestens einem substituierten Amino-furan-2-yl-essigsäure-Derivat der Formel (I-A) oder substituiertem Amino-thien-2-yl-essigsäure-Derivat der Formel (I-B) zur Herstellung eines Medikaments zur Behandlung von Schmerz, Migräne, inflammatorischen und/oder allergischen Reaktionen, Depressionen, Drogen- und/oder Alkoholmißbrauch, Gastritis, Diarrhoe, Harninkontinenz, cardiovaskulären Erkrankungen, Atemwegserkrankungen, Husten, seelischen Erkrankungen, neurodegenerativen Erkrankungen, Epilepsie, Schizophrenie, Morbus Alzheimer, Morbus Huntington, Morbus Parkinson, cerebralen Ischämien, cerebralen Infarkten, durch erhöhten Aminosäurespiegel bedingten Psychosen, Schlaganfällen, Hirnödemen, Unterversorgungszuständen des zentralen Nervensystems, Hypoxie, Anoxie, AIDS-Demens, Encephalomyelitis, Tourette-Syndrom, perinataler Asphyxie oder zur Anxiolyse.

Ferner sind pharmazeutische Zusammensetzungen Gegenstand der vorliegenden Erfindung, die mindestens eine Verbindung der wie oben definierten allgemeinen Struktur (I) in Substanz oder eines ihrer pharmazeutisch annehmbaren Salze sowie einen oder mehrere pharmazeutische Hilfsstoffe enthalten.

Die erfindungsgemäßen Arzneimittel, Medikamente und pharmazeutischen Zusammensetzungen können als flüssige, halbfeste oder feste Arzneiformen und in Form von z.B. Injektionslösungen, Tropfen, Säften, Sirupen, Sprays, Suspensionen, Granulaten, Tabletten, Pellets, transdermale therapeutische Systeme, Kapseln, Pflastern, Zäpfchen, Salben, Cremes, Lotionen, Gelen, Emulsionen oder Aerosolen vorliegen und verabreicht werden und enthalten neben mindestens einer erfindungsgemäßen Verbindung der allgemeinen Struktur (I) je nach galenischer Form und in Abhängigkeit vom Applikationsweg pharmazeutische Hilfsstoffe, wie z.B. Trägermaterialien, Füllstoffe, Lösungsmittel, Verdünnungsmittel, oberflächenaktive Stoffe, Farbstoffe, Konservierungsstoffe, Sprengmittel, Gleitmittel, Schmiermittel, Aromen und/oder Bindemittel. Diese Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokusnußöl, Erdnußöl, Sojabohnenöl, Lecithin, Natriumlactat, Polyoxyethylen- und -propylenfettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crospovidon, Agar und Bentonit.

Die Auswahl der Hilfsstoffe sowie die einzusetzenden Mengen derselben hängt davon ab, ob das Arzneimittel/Medikament oral, subkutan, parenteral, intravenös, vaginal, pulmonal, intraperitoneal, transdermal, intramuskulär, nasal, buccal, rectal oder örtlich, zum Beispiel auf Infektionen an der Haut, der Schleimhäute und an den Augen, appliziert werden soll. Für die orale Applikation eignen sich u.a. Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Pulver zur Inhalation sowie Sprays.

Erfindungsgemäße Verbindungen der allgemeinen Struktur I in einem Depot in gelöster Form oder in einem Pflaster, gegebenenfalls unter Zusatz von die Hautpenetration fördernden Mitteln, sind geeignete perkutane Applikationszubereitungen. Rektal, transmucosal, parenteral, oral oder perkutan anwendbare Zubereitungsformen können die erfindungsgemäßen Verbindungen der allgemeinen Struktur (I) verzögert freisetzen.

Die Herstellung der erfindungsgemäßen Arzneimittel und pharmazeutischen Zusammensetzungen erfolgt mit Hilfe von im Stand der Technik der pharmazeutischen Formulierung wohlbekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels, d.h. eine Verbindung der allgemeinen Struktur (I) oder eines ihrer pharmazeutisch annehmbaren Salze, mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder pharmazeutisch akzeptable Gummis, und pharmazeutischen Verdünnungsmitteln, wie z.B. Wasser, granuliert werden, um eine feste Zusammensetzung zu bilden, die eine erfindungsgemäße Verbindung oder ein pharmazeutisch annehmbares Salz davon in homogener Verteilung enthält. Unter einer homogenen Verteilung wird hier verstanden, daß der Wirkstoff gleichmäßig über die gesamte Zusammensetzung verteilt ist, so daß diese ohne weiteres in gleich wirksame Einheitsdosis-Formen, wie Tabletten, Pillen oder Kapseln, unterteilt werden kann. Die feste Zusammensetzung wird anschließend in Einheitsdosis-Formen unterteilt. Die Tabletten oder Pillen des erfindungsgemäßen Arzneimittels bzw. der erfindungsgemäßen Zusammensetzungen können auch überzogen oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

Die an den Patienten zu verabreichende Wirkstoffmenge variiert und ist abhängig vom Gewicht, dem Alter und der Krankheitsgeschichte des Patienten, sowie von der Applikationsart, der Indikation und dem Schweregrad der Erkrankung. Üblicherweise werden 0,1 bis 5000 mg/kg, insbesondere 1 bis 500 mg/kg, vorzugsweise 2 bis 250 mg/kg Körpergewicht wenigstens einer erfindungsgemäßen Verbindung der allgemeinen Struktur (I) appliziert.

Nachfolgend wird die vorliegende Erfindung durch Beispiele weiter erläutert, ohne sie darauf zu beschränken.

### Beispiele

### Vorbemerkungen

Die eingesetzten Chemikalien und Lösungsmittel wurden von Acros, Geel; Avocado, Port of Heysham; Aldrich, Deisenhofen; Fluka, Seelze; Lancaster, Mülheim; Maybridge, Tintagel; Merck, Darmstadt; Sigma, Deisenhofen bzw. TCI, Japan durch Kauf erworben oder nach üblichen und im Stand der Technik bekannten Verfahren synthetisiert.

Dünnschichtchromatographische Untersuchungen wurden mit HPTLC-Fertigplatten, Kieselgel 60 F 254, der Firma E. Merck, Darmstadt, durchgeführt.

ESI-Massenspektren wurden aufgenommen mit einem LCQ Classic Massenspektrometer der Firma Finnigan, die ¹H-NMR-Spektren wurden mit einem 300-MHz- Avance-DPX-300-NMR-Gerät oder einem 600-MHz-Avance-DRX-600-NMR-Gerät der Firma Bruker aufgenommen

### Allgemeine Arbeitsvorschrift 1 (AAV 1)

Ein Rundbodenröhrchen aus Glas (Durchmesser 16 mm, Länge 125 mm) mit Gewinde wurde mit einem Magnetrührkern versehen und automatisiert mit einem Schraubdeckel mit Septum verschlossen. Das Röhrchen wurde von einem Roboter in einen auf 20 °C temperierten Rührblock gestellt. Nacheinander wurden die folgenden Reagenzien von einem Roboter hinzupipettiert:
- 1 ml einer Lösung, die Trifluoressigsäure und die Aminkomponente (II) - jeweils 0,1M - enthält, in Acetonitril
- 1 ml einer 0,11 M Glyoxylsäurederivat-(III)-Lösung in Acetonitril
- 1 ml einer 0,3 M Furan(IV-A)- oder Thiophen(IV-B)-Lösung in Acetonitril

Das Reaktionsgemisch wurde bei 40°C in einem Rührblock 600 min lang gerührt. Danach wurde die Reaktionslösung abfiltriert. Das Röhrchen wurde dabei zweimal mit 1,5 ml einer 7,5 %igen NaHCO₃-Lösung gespült.

Anschließend wurde das Rack mit den Proben manuell auf eine Aufarbeitungsanlage gestellt. Das Reaktionsgemisch wurde auf einem Vortexer mit 2 ml Ethylacetat versetzt und geschüttelt. Zur Ausbildung der Phasengrenze wurde in der Zentrifuge kurz zentrifugiert. Die Phasengrenze wurde optisch detektiert und die organische Phase abpipettiert. Im nächsten Schritt wurde die wäßrige Phase erneut mit 2 ml Ethylacetat versetzt, geschüttelt, zentrifugiert und die organische Phase abpipettiert. Die vereinigten organischen Phasen werden über 2,4 g MgSO₄ (granuliert) getrocknet. Das Lösungsmittel wurde in einer Vakuumzentrifuge entfernt. Jede Probe wurde mit ESI-MS und/oder NMR analysiert.

Durch die automatisierte Synthese wurde eine Gleichbehandlung aller Proben sowie eine ausgesprochen konstante Reaktionsführung gewährleistet.

Die synthetisierten und isolierten Verbindungen sind in Tabelle 1 wiedergegeben.

**Tabelle 1**

| **Beispiel-Nr,** | **Verbindung** | **berechnete Molmasse** | **gefundene Molmasse** |
|---|---|---|---|
| 1 | (5-Methylsulfanylmethyl-furan-2-yl)-(5-nitro-pyridin-2-ylamino)-essigsäure | 323,32 | 323,9; 278,1 (M-CO₂) |
| 2 | (5-Brompyrimidin-2-ylamino)-(5-methylsulfanylmethyl-furan-2-yl)-essigsäure | 358,21 | 356,9/359,0 |
| 3 | 5-[Carboxy-(3,5-dichlorpyridin-2-ylamino)-methyl]-2-methyl-furan-3-carbonsäureethylester | 373,19 | 372,9/374,9 |
| 4 | 5-[Carboxy-(3,5-dibrompyridin-2-ylamino)-methyl]-2-methyl-furan-3-carbonsäureethylester | 462,1 | 460,7/462,8 /464,8 |
| 5 | 5-[Carboxy-(3,5-dibrom-6-methyl-pyridin-2-ylamino)-methyl]-2-methyl-furan-3-carbonsäureethylester | 476,13 | 474,9/476,9 /478,8 |
| 6 | (3,5-Dibrom-6-methyl-pyridin-2-ylamino)-(4-hydroxymethyl-furan-2-yl)-essigsäure | 420,06 | 420,9/422,9 |
| 7 | (3,5-Dichlor-pyridin-2-ylamino)-(4-methyl-thiophen-2-yl)-essigsäure | 317,19 | 316,9/318,9 |
| 8 | (2,4-Dibrom-5-methyl-phenylamino)-(4-methyl-thiophen-2-yl)-essigsäure | 419,14 | 420,8/422,8 |
| 9 | (4-Methyl-thiophen-2-yl)-(5-nitro-pyridin-2-ylamino)-essigsäure | 293,3 | 293,9; 248,2 (M-CO₂) |
| 10 | (3,5-Dichlor-pyridin-2-ylamino)-furan-2-yl-essigsäure | 287,1 | 286,9/289,0 |
| 11 | (3,5-Dibrompyridin-2-ylamino)-furan-2-yl-essigsäure | 376,01 | 376,9/378,9 |
| 12 | (3,5-Dibrom-6-methyl-pyridin-2-ylamino)-furan-2-yl-essigsäure | 390,04 | 390,9/392,9 |
| 13 | (3-Chlor-5-trifluormethyl-pyridin-2-ylamino)-furan-2-yl-essigsäure | 320,65 | 320,9/322,9 |
| 14 | (5-Brompyrimidin-2-ylamino)-furan-2-yl-essigsäure | 298,1 | 297,0/299,0 |
| 15 | 5-[(3,5-Dichlor-phenylamino)-ethoxycarbonylmethyl]-2-methyl-furan-3-carbonsäuremethylester | 386,23 | 385,9/387,9 |
| 16 | (5-Hydroxy-4-phenylazo-1H-pyrazol-3-ylamino)-(5-methylsulfanylmethyl-furan-2-yl)-essigsäureethylester | 415,47 | 416,0 |
| 17 | 3-{[Ethoxycarbonyl-(4-ethoxycarbonyl-5-methyl-furan-2-yl)-methyl]-amino}-1 H-pyrazol-4-carbosäureethylester | 393,39 | 393,9 |
| 18 | 5-[(4-Cyano-5-methylsulfanyl-1H-pyrazol-3-ylamino)-ethoxycarbonyl-methyl]-2-methyl-furan-3-carbonsäureethylester | 392,43 | 392,9 |
| 19 | 5-[(4-Cyano-1H-pyrazol-3-ylamino)-ethoxycarbonyl-methyl]-2-methyl-furan-3-carbonsäureethylester | 346,34 | 346,9 |
| 20 | 5-[(4-Brom-2-chlor-phenylamino)-ethoxycarbonyl-methyl]-2-methyl-furan-3-carbonsäureethylester | 444,71 | 443,9/445,9 |
| 21 | 5-[(4-Cyano-phenylamino)-ethoxycarbonyl-methyl]-2-methyl-furan-3-carbonsäureethylester | 356,37 | 357,0 |
| 22 | (4-Hydroxymethyl-furan-2-yl)-(5-hydroxy-4-phenylazo-1H-pyrazol-3-ylamino)-essigsäureethylester | 385,37 | 386,0 |
| 23 | (4-Cyano-5-methylsulfanyl-1H-pyrazol-3-ylamino)-(4-hydroxymethyl-furan-2-yl)-essigsäureethylester | 336,36 | 336,9 |
| 24 | (4-Brom-2-chlor-phenylamino)-(4-hydroxymethyl-furan-2-yl)-essigsäureethylester | 388,64 | 387,9/389,9 |
| 25 | (3,5-Dichlor-phenylamino)-(4-hydroxymethyl-furan-2-yl)-essigsäureethylester | 344,19 | 344,0/346,0 |
| 26 | (5-Chlor-2-methyl-phenylamino)-[5-(furan-2-ylmethylsulfanylmethyl)-furan-2-yl]-essigsäureethylester | 419,92 | 420,0 |
| 27 | (2,4-Dibrom-phenylamino)-(4-methyl-thiophen-2-yl)-essigsäure | 405,11 | (M-CO₂) 358,2/360,2 /362,2 |
| 28 | (5-Chlor-2-methyl-phenylamino)-(5-methylsulfanylmethyl-furan-2-yl)-essigsäure | 325,81 | 326,0 |
| 29 | (2-Ethyl-phenylamino)-(5-methylsulfanylmethyl-furan-2-yl)-essigsäure | 305,39 | 306,0 |
| 30 | (4-sec-Butyl-phenylamino)-(5-methylsulfanylmethyl-furan-2-yl)-essigsäure | 333,44 | 334,1 |
| 31 | (5-Methylsulfanylmethyl-furan-2-yl)-(4-trifluormethoxy-phenylamino)-essigsäure | 361,33 | 362,0/ 316,2 (M-CO₂) |
| 32 | (2-Isopropyl-phenylamino)-(5-methylsulfanylmethyl-furan-2-yl)-essigsäure | 319,42 | 320,1 |
| 33 | (2,4-Dibrom-phenylamino)-(5-methylsulfanylmethyl-furan-2-yl)-essigsäure | 435,14 | (M-CO₂) 390,0/392,0 |
| 34 | (4-*tert*-Butyl-phenylamino)-(5-methylsulfanylmethyl-furan-2-yl)-essigsäure | 333,44 | 334,0 |
| 35 | (5-Chlor-2-methyl-phenylamino)-[5-(furan-2-ylmethylsulfanylmethyl)-furan-2-yl]-essigsäure | 391,87 | 392,0/394,0 |
| 36 | (2-Ethyl-phenylamino)-[5-(furan-2-ylmethylsulfanylmethyl)-furan-2-yl]-essigsäure | 371,45 | 372,1 |
| 37 | (4-sec-Butyl-phenylamino)-[5-(furan-2-ylmethylsulfanylmethyl)-furan-2-yl]-essigsäure | 399,5 | 400,1 |
| 38 | [5-(Furan-2-ylmethylsulfanylmethyl)-furan-2-yl]-(2-isopropyl-phenylamino)-essigsäure | 385,48 | 386,2 |
| 39 | (4-*tert*-Butyl-phenylamino)-[5-(furan-2-ylmethylsulfanylmethyl)-furan-2-yl]-essigsäure | 399,5 | 400,1 |
| 40 | 5-[Ethoxycarbonyl-(4-iod-phenylamino)-methyl]-2-methyl-furan-3-carbonsäuremethylester | 443,23 | 442,1/444,0 /445,0 |
| 41 | 5-[(4-Chlor-2-methyl-phenylamino)-ethoxycarbonyl-methyl]-2-methyl-furan-3-carbonsäuremethylester | 365,81 | 366,1/368,1 |
| 42 | 5-[Ethoxycarbonyl-(4-phenoxy-phenylamino)-methyl]-2-methyl-furan-3-carbonsäuremethylester | 409,43 | 408,3/410,2 |
| 43 | 5-[Ethoxycarbonyl-(4-iod-phenylamino)-methyl]-2-methyl-furan-3-carbonsäureethylester | 457,26 | 456,2/458,0 /459,1 |
| 44 | 5-[(2-Chlor-phenylamino)-ethoxycarbonyl-methyl]-2-methyl-furan-3-carbonsäureethylester | 365,81 | 366,1/367,0 /368,1 |
| 45 | 5-[(4-Chlor-2-methyl-phenylamino)-ethoxycarbonyl-methyl]-2-methyl-furan-3-carbonsäureethylester | 379,83 | 380,1/382,1 |
| 46 | 5-[(2-Chlor-4-fluor-phenylamino)-ethoxycarbonyl-methyl]-2-methyl-furan-3-carbonsäureethylester | 383,8 | 384,1/385,0 |
| 47 | 5-[Ethoxycarbonyl-(4-phenoxy-phenylamino)-methyl]-2-methyl-furan-3-carbonsäureethylester | 423,46 | 422,4/424,2 |
| 48 | 5-[(2,3-Dichlor-phenylamino)-ethoxycarbonyl-methyl]-2-methyl-furan-3-carbonsäureethylester | 400,25 | 400,0/402,0 |
| 49 | (4-Hydroxymethyl-furan-2-yl)-(4-iod-phenylamino)-essigsäureethylester | 401,19 | 402,2 |
| 50 | (2,4-Dichlor-phenylamino)-(4-hydroxymethyl-furan-2-yl)-essigsäureethylester | 344,19 | 344,2/346,2 |
| 51 | (4-Chlor-2-methyl-phenylamino)-(4-hydroxymethyl-furan-2-yl)-essigsäureethylester | 323,77 | 324,3 |
| 52 | (4-Hydroxymethyl-furan-2-yl)-(4-phenoxy-phenylamino)-essigsäureethylester | 367,4 | 368,3 |
| 53 | (2,3-Dichlor-phenylamino)-(4-hydroxymethyl-furan-2-yl)-essigsäureethylester | 344,19 | 344,1/346,1 |
| 54 | (2,3-Dichlor-phenylamino)-furan-2-yl-essigsäureethylester | 314,16 | 314,4/316,3 |
| 55 | 5-[Carboxy-(3,5-dibrom-6-methyl-pyridin-2-ylamino)-methyl]-2-methyl-furan-3-carbonsäuremethylester | 462,1 | 460,9/462,9 /464,9 |
| 56 | (5-Methylsulfanylmethyl-furan-2-yl)-(pyrazin-2-ylamino)-essigsäure | 279,31 | 280,0 |
| 57 | (3,5-Dibrom-pyridin-2-ylamino)-(5-methylsulfanylmethyl-furan-2-yl)-essigsäure | 436,13 | 436,8/438,8 |
| 58 | (3,5-Dibrom-6-methyl-pyridin-2-ylamino)-(5-methylsulfanylmethyl-furan-2-yl)-essigsäure | 450,15 | 448,9/450,8 /452,8 |
| 59 | 5-[(4-Brom-2-chlor-phenylamino)-carboxy-methyl]-2-methyl-furan-3-carbonsäuremethylester | 402,63 | (M-CO₂) 356,4/358,4 /360,3 |
| 60 | 5-[Carboxy-(4-cyano-phenylamino)-methyl]-2-methyl-furan-3-carbonsäuremethylester | 314,29 | (M-CO₂) 269,4 |
| 61 | 5-[Carboxy-(3,5-dichlor-phenylamino)-methyl]-2-methyl-furan-3-carbonsäuremethylester | 358,17 | 358,0/360,0 (M-CO₂) 312,4 |
| 62 | 5-[Carboxy-(2-phenoxy-phenylamino)-methyl]-2,4-dimethyl-furan-3-carbonsäureethylester | 409,43 | 410,1 (M-CO₂) 364,4 |
| 63 | 5-[(4-Brom-2-chlor-phenylamino)-carboxy-methyl]-2,4-dimethyl-furan-3-carbonsäureethylester | 430,68 | (M-CO2) 384,3/386,2 /388,2 |
| 64 | 5-[Carboxy-(4-cyano-phenylamino)-methyl]-2,4-dimethyl-furan-3-carbonsäureethylester | 342,35 | (M-CO₂) 297,4 |
| 65 | 5-[Carboxy-(3,5-dichlor-phenylamino)-methyl]-2,4-dimethyl-furan-3-carbonsäureethylester | 386,23 | (M-CO₂) 340,3/342,3 |
| 66 | 5-[(4-Brom-2-chlor-phenylamino)-carboxy-methyl]-2-methyl-furan-3-carbonsäureethylester | 416,65 | 418,0, (M-CO₂) 370,4/372,3 |
| 67 | 5-[Carboxy-(4-cyano-phenylamino)-methyl]-2-methyl-furan-3-carbonsäureethylester | 328,32 | (M-CO₂) 283,4 |
| 68 | 5-[Carboxy-(3,5-dichlor-phenylamino)-methyl]-2-methyl-furan-3-carbonsäureethylester | 372,2 | 372,0/374,0 (M-CO₂) 326,3 |
| 69 | (5-*tert*-Butyl-1H-pyrazol-3-ylamino)-(4-hydroxymethyl-furan-2-yl)-essigsäure | 293,32 | 294,5 |
| 70 | (4-Brom-2-chlor-phenylamino)-(3-methyl-thiophen-2-yl)-essigsäure | 360,66 | (M-CO₂) 314,4/316,3 /318,3 |
| 71 | (4-Cyano-phenylamino)-(3-methyl-thiophen-2-yl)-essigsäure | 272,32 | (M-CO₂) 227,4 |
| 72 | (3,5-Dichlor-phenylamino)-(3-methyl-thiophen-2-yl)-essigsäure | 316,2 | (M-CO₂) 270,4/272,4 |
| 73 | (3,5-Dichlor-phenylamino)-furan-2-yl-essigsäure | 286,11 | 286,3/288,3 (M-CO₂) 240,6/242,5 |
| 74 | [5-(Furan-2-ylmethylsulfanylmethyl)-furan-2-yl]-(4-phenoxy-phenylamino)-essigsäureethylester | 463,55 | 464,0 |
| 75 | (5-Ethoxycarbonylmethyl-thiophen-2-yl)-(5-hydroxy-4-phenylazo-1 H-pyrazol-3-ylamino)-essigsäureethylester | 457,5 | 458,0 |
| 76 | {5-[1-(4-Cyano-1H-pyrazol-3-ylamino)-3-methoxy-2-oxo-propyl]-thiophen-2-yl}-essigsäureethylester | 362,4 | 363,0 |
| 77 | (4-Ethyl-phenylamino)-(5-methylsulfanylmethyl-furan-2-yl)-essigsäure | 305,39 | 306,1, (M-CO₂) 260,2 |
| 78 | (3-Chlor-2-methyl-phenylamino)-(5-methylsulfanylmethyl-furan-2-yl)-essigsäure | 325,81 | 326,0/328,0 , (M-CO₂) 280,2 |
| 79 | (4-Chlor-phenylamino)-(5-methylsulfanylmethyl-furan-2-yl)-essigsäure | 311,78 | 311,9/314,0 , (M-CO₂) 266,1 |
| 80 | (5-Methylsulfanylmethyl-furan-2-yl)-o-tolylamino-essigsäure | 291,36 | 292,1, (M-CO₂) 246,2 |
| 81 | 5-[Carboxy-(4-chlor-3-trifluormethyl-phenylamino)-methyl]-2-methyl-furan-3-carbonsäureethylester | 405,75 | (M-CO₂) 360,2/362,1 |
| 82 | 5-[Carboxy-(2-chlor-phenylamino)-methyl]-2-methyl-furan-3-carbonsäureethylester | 337,75 | 338,0, (M-CO2) 292,2 |
| 83 | 5-[Carboxy-(2-chlor-4-fluor-phenylamino)-methyl]-2-methyl-furan-3-carbonsäureethylester | 355,74 | 356,8/358,9 , (M-CO2) 310,2/312,2 |
| 84 | 5-[Carboxy-(4-chlor-2-fluor-phenylamino)-methyl]-2-methyl-furan-3-carbonsäureethylester | 355,74 | (M-CO₂) 310,2/312,2 |
| 85 | 5-[Carboxy-(2,3-dichlor-phenylamino)-methyl]-2-methyl-furan-3-carbonsäureethylester | 372,2 | (M-CO₂) 310,2/312,2 |
| 86 | (2,4-Dichlor-phenylamino)-(4-methyl-thiophen-2-yl)-essigsäure | 316,2 | (M-CO₂) 270,3/272,3 |
| 87 | (4-Chlor-3-trifluormethyl-phenylamino)-(4-methyl-thiophen-2-yl)-essigsäure | 349,75 | (M-CO₂) 304,4/306,3 |
| 88 | (2,4-Dichlor-phenylamino)-furan-2-yl-essigsäure | 286,11 | 286,3/288,1 , (M-CO₂) 240,4/242,4 |
| 89 | (4-Chlor-3-trifluormethyl-phenylamino)-furan-2-yl-essigsäure | 319,66 | (M-CO₂) 274,5/276,5 |
| 90 | (4-lod-phenylamino)-(5-methylsulfanylmethyl-furan-2-yl)-essigsäure | 403,23 | 403,9, (M-CO₂) 358,1 |
| 91 | (2,4-Dichlor-phenylamino)-(5-methylsulfanylmethyl-furan-2-yl)-essigsäure | 346,23 | (M-CO₂) 300,2/302,1 |
| 92 | (4-Chlor-3-trifluormethyl-phenylamino)-(5-methylsulfanylmethyl-furan-2-yl)-essigsäure | 379,78 | (M-CO₂) 334,1/336,1 |
| 93 | (2-Chlor-phenylamino)-(5-methylsulfanylmethyl-furan-2-yl)-essigsäure | 311,78 | 312,0/314,0 , (M-CO₂) 366,2/368,2 |
| 94 | (4-Chlor-2-methyl-phenylamino)-(5-methylsulfanylmethyl-furan-2-yl)-essigsäure | 325,81 | 326,0/328,0 , (M-CO₂) 280,2/282,2 |
| 95 | (2-Chlor-4-fluor-phenylamino)-(5-methylsulfanylmethyl-furan-2-yl)-essigsäure | 329,77 | 330,0, (M-CO₂) 284,2/286,2 |
| 96 | (2-Chlor-4-methyl-phenylamino)-(5-methylsulfanylmethyl-furan-2-yl)-essigsäure | 325,81 | 326,0/328,0 , (M-CO₂) 280,2 |
| 97 | (2,3-Dichlor-phenylamino)-(5-methylsulfanylmethyl-furan-2-yl)-essigsäure | 346,23 | (M-CO₂) 300,2/302,1 |
| 98 | [5-(Furan-2-ylmethy)sulfanylmethyl)-furan-2-yl]-(4-iodo-phenylamino)-essigsäure | 469,29 | 470,1/471,7 |
| 99 | (4-Chlor-3-trifluormethyl-phenylamino)-[5-(furan-2-ylmethylsulfanylmethyl)-furan-2-yl]-essigsäure | 445,84 | 446,7/447,6 (M-CO₂) 400,6/401,5 |
| 100 | (4-Chlor-2-methyl-phenylamino)-[5-(furan-2-ylmethylsulfanylmethyl)-furan-2-yl]-essigsäure | 391,87 | 392,5/393,4 |
| 101 | (2-Chlor-4-methyl-phenylamino)-[5-(furan-2-ylmethylsulfanylmethyl)-furan-2-yl]-essigsäure | 391,87 | 392,2/394,8 |
| 102 | (4-Chlor-3-trifluormethyl-phenylamino)-(5-ethoxycarbonylmethyl-thiophen-2-yl)-essigsäure | 421,82 | (M-CO₂) 376,4/378,4 |
| 103 | (4-Acetyl-3,5-dimethyl-furan-2-yl)-(2-chlor-4-methyl-phenylamino)-essigsäure | 335,78 | (M-CO₂) 290,3/292,2 |
| 104 | (4-Acetyl-3,5-dimethyl-furan-2-yl)-(2,3-dichlor-phenylamino)-essigsäure | 356,2 | (M-CO₂) 310,3/312,2 |
| 106 | (3,5-Dichlor-phenylamino)-(5-methylsulfanylmethyl-furan-2-yl)-essigsäure | 346,23 | 345,9/347,9 ; 300,0/302,0 (M-CO₂) |
| 113 | (3-Chlor-phenylamino)-(5-mercaptomethyl-furan-2-yl)-essigsäure | 297,8 | 297,9 (MH) |
| 114 | (3,4-Dichlor-phenylamino)-(5-mercaptomethyl-furan-2-yl)-essigsäure | 332,2 | 332,0 (MH) |

### Allgemeine Arbeitsvorschrift 2 (AAV 2)

3,5-Dichloranilin (10 mmol), Glyoxylsäure-Hydrat (11 mmol) und das Furan-Derivat (IV-A) bzw. Thiophen-Derivat (IV-B) (30 mmol) wurden in 10 ml Acetonitril gelöst und 14 Stunden bei 40°C gerührt. Der Reaktionsansatz wurde eingeengt und mittels RP4-Kieselgel-MPLC (Polygoprep 60-130 C4, Macherey-Nagel, Postfach 101352, D-52313 Düren, Deutschland) mit Methanol/Wasser-Gemischen unterschiedlicher Zusammensetzung und einem Zusatz von 0,1 %igem Eisessig gereinigt. Nach Einrotieren der Produktfraktionen wurde das Produkt als Öl isoliert.

### Allgemeine Arbeitsvorschrift 3 (AAV 3) - Mikrowellen-Bestrahlung

Die Umsetzungen unter Mikrowellen-Bestrahlung wurden in einer Labor-Mikrowelle des Fabrikats MLS ETHOS 600 der Firma MLS-GmbH (D-88299 Leutkirch, Auenweg 37, Deutschland) durchgeführt.

Zur Synthese wurden die Reaktionskomponenten (II), (III) und (IV-A) bzw. (IV-B) sowie das Lösungsmittel in ein druckstabiles Teflongefäß gegeben, das mit einem Teflondeckel verschlossen und in eine Sicherungshalterung zum Ablassen von Überdruck eingespannt wurde. Die Kontrolle der Reaktionstemperatur erfolgte in einem zweiten Teflongefäß, welches das gleiche Lösungsmittel enthielt, das bei der Synthese verwendet wurde. Die Temperatursteuerung erfolgte über eine interne Glasfaseroptik, die in einem Quarzrohr geführt und extern von einem Steuerungscomputer kontrolliert wurde.

3,5-Dichloranilin (10 mmol), Glyoxylsäure-Hydrat (11 mmol) und Furan-Derivat (IV-A) bzw. Thiophen-Derivat (IV-B) (30 mmol) wurden in 10 ml Acetonitril gelöst, in der Mikrowelle innerhalb einer Minute auf 50°C erhitzt und fünf Minuten bei dieser Temperatur belassen. Die Mikrowellenstrahlung wurde mit einer Leistung von 800 Watt eingestrahlt, die durch einen Steuerungsrechner kontrolliert und nachreguliert wurde. Nach dem Abkühlen wurde das Gefäß in Eiswasser gestellt, vorsichtig geöffnet und das Lösungsmittel am Rotationsverdampfer entfernt.

Die Reinigung erfolgte mittels RP4-Kieselgel-MPLC (Polygoprep 60-130 C4, Macherey-Nagel, Postfach 101352, D-52313 Düren, Deutschland) mit Methanol/Wasser-Gemischen unterschiedlicher Zusammensetzung und einem Zusatz von 0,1 %igem Eisessig. Nach Einrotieren der Produktfraktionen wurde das Produkt als Öl isoliert.

Für die Herstellung des Hydrochlorid-Salzes wurde das Rohprodukt in Butan-2-on gelöst, unter Eiskühlung und Rühren mit äquimolarer Menge an Wasser und dann mit äquimolarer Menge an TMSCI versetzt. Nach Stehenlassen über Nacht wurde das ausgefallene HCI-Salz abgesaugt und getrocknet, oder die Fällung wurde durch Entfernen der Lösungsmittel im Vakuum herbeigeführt.

Nachfolgend werden die ¹H-NMR-Daten einiger der nach den AAV 2 und 3 hergestellten Verbindungen wiedergegeben; die NMR-Daten wurden bei 600 MHz erhalten, wobei als Lösungsmittel *d₆*-DMSO verwendet wurde:

### (3,5-Dichlor-phenylamino)-(thiophen-2-yl)-essigsäureethylester (115)

δ = 1,20 ppm (t, 3H, CH₃); 4,16 ppm (q, 2H, OCH₂); 5,65 ppm (d, 1H, α-CH); 6,65 ppm (s, 1 H, Aryl-4-H); 6,80 pm (s, 2H, Aryl-2-H und Aryl-6-H); 7,10 ppm (m, 1 H, Thiophen-H); 7,20 ppm (d, 1 H, Thiophen-H); 7,50 ppm (d, 1 H, Thiophen-H).

### (4-Bromfuran-2-yl)-(3,5-dichlorphenylamino)-essigsäureethylester (116)

δ = 1,15 ppm (t, 3H, CH₃); 4,15 ppm (q, 2H, OCH₂); 5,50 pp (d, 1 H, α-CH); 6,60 ppm (s, 1 H, Furan-H); 6,70 ppm (s, 1 H, Furan-H); 6,75 ppm (s, 1 H, Aryl-4-H); 6,80 ppm (s, 2H, Aryl-2-H und Aryl-6H); 7,80 ppm (d, 1 H, α-NH).

### (3,5-Dichlorphenylamino)-(5-propylthiophen-2-yl)-essigsäureethylester-Hydrochlorid (117-HCl)

δ = 0,95 ppm (t, 3H, CH₃); 1,20 ppm (t, 3H, CH₃); 1,60 ppm (q, 2H, CH₂); 2,70 ppm (t, 2H, CH₂); 4,15 ppm (q, 2H, OCH₂); 4,80 ppm (s, 2H, α-NH₂⁺ und 1H, α-CH); 5,50 ppm (d, 1 H, Thiophen-H); 6,40 ppm (d, 1 H, Thiophen-H); 6,40 ppm (s, 1 H, Aryl-4-H); 6,72 ppm (s, 2H, Aryl-2-H und Aryl-6-H).

### (3,5-Dichlorphenylamino)-(3-methylthiophen-2-yl)-essigsäureethylester (118)

δ = 1,15 ppm (t, 3H, CH₃); 2,20 ppm (s, 3H, 2-CH₃-Thiophen); 4,10 ppm (q, 2H, OCH₂); 5,50 ppm (d, 1 H, α-CH); 6,65 ppm (s, 2H, Aryl-2-H und Aryl-6H); 6,80 ppm (d, 1 H, Thiophen-H); 7,28 ppm (d, 1 H, Thiophen-H).

### (5-tert-Butylfuran-2-yl)-(3,5-dichlorphenylamino)-essigsäure (119)

δ = 1,22 ppm (s, 9H, *tert*-Bu); 5,24 ppm (d, 1 H, α-CH); 5,99 ppm (d, 1 H, Furan-H); 6,26 ppm (d, 1 H, Furan-H); 6,54 ppm (s, 1 H, Aryl-4-CH); 6,73 ppm (s, 2H, Aryl-2H und Aryl-6H); 8,27 ppm (d, 1 H, α-NH); 13,80 ppm (s (breit), 1H, CO₂H).

### (5-tert-Butyl-furan-2-yl)-3,5-dichlorphenylamino)-essigsäure-Hydrochlorid (119-HCl)

δ = 1,25 ppm (s, 9H, tert-Butyl); 5,30 ppm (m, 1 H, α-CH); 6,00 ppm (d, 1 H, Furyl-H); 6,30 ppm (d, 1H, Furyl-H); 6,65 ppm (m, 1 H, α-NH); 6,70 ppm (s, 1 H, Aryl-H); 6,75 ppm (s, 2H, Aryl-H).

### (3,5-Dichlorphenylamino)-[5-(methylsulfanyl)methyl-furan-2-yl]-essigsäure (106)

δ = 2,02 ppm (s, 3H, SCH₃); 3,69 ppm (s, 2H, CH₂S); 5,30 ppm (d, 1H, α-CH); 6,22 ppm (s, 1 H, Furan-H); 6,37 ppm (s, 1 H, Furan-H); 6,63 ppm (s, 1 H, Aryl-4-H); 6,73 ppm (s, 2H, Aryl-2-H und Aryl-4H); 6,79 ppm (d, 1 H, α-NH); 13,17 ppm (s (breit), 1 H, CO₂H).

### (3,5-Dichlorphenylamion)-[5-(methylsulfanyl)methyl-furan-2-yl]-essigsäure-Hydrochlorid (106-HCl)

δ = 2,08 ppm (s, 3H, SCH₃); 2,50 ppm (2, 2H, CH₂S); 3,70 ppm (s, 2H, α-NH₂⁺); 5,37 ppm (m, 1 H, α-CH); 6,25 ppm (d, 1 H, Furan-H); 6,40 ppm (d, 1 H, Furan-H); 6,80 ppm (s, 1 H, Aryl-4-H); 6,78 pm (s, 2H, Aryl-2-H und Aryl-6-H)_{.}

### (3,5-Dichlorphenylamino)-(5-methylsulfanylmethylfuran-2-yl)-essigsäure Natriumsalz (106-Na)

δ = 2,99 ppm (s, 3H, SCH₃); 3,65 ppm (s, 2H, CH₂S); 4,45 ppm (m, 1 H, α-CH); 6,10 ppm (m, 3H, Aryl-H); 6,45 ppm (m,1 H, α-NH); 6,50 ppm (d, 1 H, Thiophen-H); 6,55 ppm (d, 2H, Thiophen-H).

### (3,5-Dichlorphenylamino)-[5-(furan-2-ylmethylsulfanylmethyl)-furan-2-yl]-essigsäure (122)

δ = 3,68 ppm (s, 2H, CH₂S); 3,73 (s, 2H, CH₂S); 5,33 (d, 1 H, α-CH); 5,72 ppm (m, 1 H, Furan-H); 6,26 ppm (d, 1 H, Furan-H); 6,27 ppm (d, 1 H, Furan-H); 6,36 ppm (d, 1 H, Furan-H); 6,39 ppm (d, 1 H, Furan-H); 6,64 ppm (s, 1 H, Aryl-4-H); 6,74 ppm (s, 2H, Aryl-2-H und Aryl-6-H); 7,55 ppm (d, 1 H, α-NH); 13,23 ppm (s (breit), 1 H, CO₂H).

### (3,5-Dichlorphenylamino)-[5-(furan-2-ylmethyldisulfanylmethyl)-furan-2-yl]-essigsäure (120)

δ = 3,73 ppm (s, 2H, CH₂S); 3,80 ppm (s, 2H, CH₂S); 5,35 ppm (d, 1 H, α-CH); 6,24 ppm (m, 1 H, Furan-H); 6,24 (d, 1 H, Furan-H); 6,30 ppm (d, 1 H, Furan-H); 6,38 ppm (s, 1 H, Furan-H); 6,43 ppm (d, 1 H, Furan-H); 6,43 ppm (s, 1H, Aryl-4-H); 6,74 ppm (s, 2H, Aryl-2-H und Aryl-6-H); 7,58 ppm (d, 1H, α-NH); 13,20 ppm (s (breit), 1H, CO₂H).

### (3,5-Dichlorphenylamino)-(5-methyldisulfanylmethylfuran-2-yl)-essigsäure (121)

δ = 2,08 ppm (s, 3H, SCH₃); 3,96 ppm (s, 2H, CH₂S); 5,30 (d, 1 H, α-CH); 6,32 ppm (d, 1 H, Furan-H); 6,41 ppm (d, 1 H, Furan-H); 6,61 ppm (s, 1 H, Aryl-4-H); 6,73 ppm (s, 2H, Aryl-2-H und Aryl-6-H); 8,25 ppm (d, 1 H, α-NH); 13,19 ppm (s (breit), 1 H, CO₂H).

### (5-Acetylsulfanylmethyl-furan-2-yl)-(3,5-dichlorphenylamino)-essigsäure (110)

δ = 2,35 ppm (s, 3H, COCH₃); 4,13 ppm (s, 2H, CH₂S); 5,28 ppm (d, 1 H, α-CH); 6,24 ppm (s, 1 H, Furan-H); 6,35 (d, 1 H, Furan-H); 6,63 ppm (s, 1 H, Aryl-4-H); 6,71 ppm (s, 2H, Aryl-2-H und Aryl-6-H); 8,29 ppm (d, 1 H, α-NH); 13,13 ppm (s (breit), 1 H, CO₂H).

### (5-Acetoxymethylfuran-2-yl)-(3,5-dichlorphenylamino)-essigsäure (123) Molmasse (ber.): 358,18 g/mol; gef. 356,0.

δ = 2,05 ppm (s, 3H, COCH₃); 5,00 ppm (s, 2H, CH₂O); 5,20 ppm (m, 1 H, α-CH); 6,40 - 6,50 ppm (m, 2H, Furyl-H); 6,60 ppm (s, 1 H, Aryl-H); 6,65 ppm (s, 2H, Aryl-H); 6,80 ppm (d, 1 H, α-NH).

Weitere nach AAV 2 und AAV 3 hergestellte Verbindungen sind in Tabelle 2 wiedergegeben.

**Tabelle 2**

| **Beispiel-Nr.** | **Verbindung** | **berechnete Molmasse** | **gefundene Molmasse** |
|---|---|---|---|
| 106 | (3,5-Dichlor-phenylamino)-(5-methylsulfanylmethyl-furan-2-yl)-essigsäure | 412,3 | 410,2 (M-H) 366,2 (M-CO2) |
| 107 | (3,5-Dichlor-phenylamino)-(5-mercaptomethyl-furan-2-yl)-essigsäure | 332,2 | 330,1 (M-H) 286,1(M-CO2) |
| 108 | (3,5-Dichlor-phenylamino)-(5-methyl-thiophen-2-yl)-essigsäure | 316,2 | 314,0 (M-H) 270,2 (M-CO2) |
| 109 | (3,5-Dichlor-phenylamino)-(5-hydroxymethyl-thiophen-2-yl)-essigsäure | 332,2 | 331,9 (M-H) 286,1 (M-CO2) |
| 110 | (5-Acetylsulfanylmethyl-furan-2-yl)-(3,5-dichlorphenylamino)-essigsäure | 374,2 | 372,3 (M-H) 327,6 (M-CO2) |
| 111 | (3,5-Dichlor-phenylamino)-(5-ethyl-thiophen-2-yl)-essigsäure | 330,2 | 328,0 (M-H) 284,2 (M-CO2) |
| 112 | (3,5-Dichlor-phenylamino)-(5-n-propyl-thiophen-2-yl)-essigsäure | 344,3 | 342,0 (M-H) 298,2 (M-CO2) |

### Allgemeine Arbeitsvorschrift 4 (AAV 4)

Die Synthesen erfolgten analog zu dem von N. A. Petasis et al., Tetrahedron (1997), 16463-16470, beschriebenen Synthese-Verfahren: In 50 ml Dichlormethan wurden 10 mmol Glyoxylsäure-Hydrat gelöst, unter Rühren 10 mmol Anilin-Komponente (II) und 10 mmol der Boronsäure des Furan- bzw. Thiophen-Derivats (IV-A) bzw. (IV-B) zugegeben und über Nacht bei Raumtemperatur gerührt. Das ausgefallene Produkt wurde abgesaugt, mit wenig kaltem Dichlormethan nachgewaschen und die Substanz im Hochvakuum getrocknet. Es wurden farblose Feststoffe erhalten.

Nachfolgend werden die ¹H-NMR-Daten einiger der nach der AAV 4 hergestellten Verbindungen wiedergegeben; die NMR-Daten wurden bei 300 MHz bzw. 600 MHz erhalten, wobei als Lösungsmittel *d₆*-DMSO verwendet wurde:

### (3,5-Dichlorphenylamino)-thiophen-3-yl)-essigsäure (124)

Molmasse (ber.): 302,18 g/mol; gef. 301,9.
δ = 5,25 ppm (m, 1 H, α-CH); 6,65 ppm (s, 1 H, Aryl-H); 6,70 ppm (s, 2H, Aryl-H); 7,85 ppm (m, 1H, α-NH); 7,15 ppm (d, 1H, Thiophen-H); 7,50 - 7,60 ppm (dd, 2H, Thiophen-H); 13,00 (s (breit), 1H, COOH).

### (3,5-Dichlorphenylamino)-(4-methylthiophen-2-yl)-essigsäure (126)

Molmasse (ber.): 358,18 g/mol; gef. 317,1.
δ = 2,20 ppm (2, 3H, CH₃); 5,40 ppm (d, 1 H, α-CH); 6,65 ppm (s, 1 H, Aryl-H); 6,75 ppm (s, 2H, Aryl-H); 6,90 ppm (d, 1 H, α-NH); 7,00 - 7,10 ppm (s, 2H, Thiophen-H); 13,30 ppm (s (breit), 1 H, COOH).

### (3,5-Dichlorphenylamino)-(5-methylthiophen-2-yl)-essigsäure (125)

Molmasse (ber.): 358,18 g/mol; gef. 316,2.
δ = 2,40 ppm (s, 3H, CH₃); 5,40 ppm (d, 1 H, α-CH); 6,60 - 6,70 ppm (m, 2H, α-NH und Aryl-H); 6,70 ppm (s, 2H, Aryl-H); 6,90 ppm (d, 1H, Thiophen-H); 6,95 ppm (d, 1 H, Thiophen-H); 13,30 ppm (s (breit), 1 H, COOH).

### (3,5-Dichlorphenylamino)-(5-methylthiophen-2-yl)-essigsäure-Natriumsalz (125-Na)

δ = 2,40 ppm (s, 3H, CH₃); 5,30 ppm (d, 1 H, α-CH); 6,60 - 6,80 ppm (m, 4H, α-NH und Aryl-H); 6,85 ppm (d, 1 H, Thiophen-H); 6,90 ppm (d, 1 H, Thiophen-H).

### (5-Chlorthiophen-2-yl)-(3,5-dichlorphenylamino)-essigsäure (127)

Molmasse (ber.): 336,63 g/mol; gef. 335,7.
δ = 5,50 ppm (d, 1 H, α-CH); 6,65 ppm (s, 1 H, α-NH); 6,70 ppm (s, 1 H, Aryl-H); 6,80 ppm (s, 2H, Aryl-H); 7,00 ppm (d, 1 H, Thiophen-H); 7,05 ppm (d, 1 H, Thiophen-H); 13,50 ppm (s (breit), 1 H, COOH).

### (3,5-Dichlorphenylamino)-(5-chlorthiophen-2-yl)-essigsäure-Natriumsalz (127-Na)

δ = 4,55 ppm (d, 1 H, δ-CH); 6,55 ppm (s, 1H, Aryl-H); 6,60 ppm (s, 2H, Aryl-H); 6,75 ppm (d, 1H, Thiophen-H); 6,85 ppm (d, 1 H, Thiophen-H).

### Rezeptorbindungs-Studien (Glycin-Bindungsstelle des NMDA-Rezeptorkanals)

Die Untersuchungen zur Bestimmung der Affinität der erfindungsgemäßen Verbindungen der Formel (I) zur Glycin-Bindungsstelle des NMDA-Rezeptorkanals wurden an Hirnmembranhomogenaten (Homogenat von Cortex- und Hippocampus-Areal aus dem Hirn von männlichen Ratten, Stamm Wistar) durchgeführt (B.M. Baron , B.W. Siegel, B.L. Harrison, R.S. Gross, C. Hawes and P.Towers, Journal of Pharmacology and Experimental Therapeutics, (1996), Vol. 279, S. 62).

Hierzu wurden Cortex und Hippocampus aus frisch entnommenen Rattengehirnen freipräpariert und in 5 mmol/l TRIS-Acetatpuffer, 0,32 mol/l Saccharose pH 7,4 (10 ml/g Frischgewicht) mit einem Potter-Homogenisator (Fa. Braun/Melsungen; 10 Kolbenhübe bei 500 Upm) unter Eiskühlung homogenisiert und anschließend für 10 Minuten bei 1.000 g und 4°C zentrifugiert. Der erste Überstand wurde gesammelt und das Sediment erneut mit 5 mmol/l TRIS-Acetatpuffer, 0,32 mol/l Saccharose pH 7,4 (5 ml/g ursprüngliches Frischgewicht) mit dem Potter-Homogenisator (10 Kolbenhübe bei 500 Upm) unter Eiskühlung homogenisiert und für 10 Minuten bei 1.000 g und 4°C zentrifugiert. Der resultierende Überstand wurde mit dem Überstand aus der ersten Zentrifugation vereinigt und bei 17.000 g für 20 Minuten bei 4°C zentrifugiert. Der Überstand nach dieser Zentrifugation wurde verworfen und das Membransediment mit 5 mmol/l TRIS-Acetatpuffer pH 8,0 (20 ml/g ursprüngliches Frischgewicht) aufgenommen und mit 10 Kolbenhüben bei 500 Upm homogenisiert.

Anschließend wurde das Membranhomogenat für 1 Stunde bei 4°C inkubiert für 30 Minuten bei 50.000 g und 4°C zentrifugiert. Der Überstand wurde verworfen und das Zentrifugenröhrchen mit dem Membransediment mit Parafilm verschlossen und für 24 Stunden bei -20°C eingefroren. Am folgenden Tag wurde das Membransediment aufgetaut und mit eiskaltem 5 mmol/l TRIS-Acetatpuffer, 0,1 % Saponin (w/v) pH 7,0 (10 ml/g ursprüngliches Frischgewicht) aufgenommen und mit 10 Kolbenhüben bei 500 Upm homogenisiert und anschließend für 20 Minuten bei 50.000 g und 4°C zentrifugiert. Der resultierende Überstand wurde verworfen und das Sediment in einem kleinen Volumen mit 5 mmol/l TRIS-Acetatpuffer pH 7,0 (ca. 2 ml/g ursprüngliches Frischgewicht) aufgenommen und erneut mit 10 Kolbenhüben bei 500 Upm homogenisiert. Nach Bestimmung des Proteingehaltes wurde das Membranhomogenat mit 5 mmol/l TRIS-Acetatpuffer pH 7,0 auf eine Proteinkonzentration von 10 mg Protein/ml eingestellt und in Aliquoten bis zur Testung eingefroren.

Für den Rezeptorbindungstest wurden Aliquote aufgetaut, 1:10 mit 5 mmol/l TRIS-Acetatpuffer pH 7,0 verdünnt, mit 10 Kolbenhüben bei 500 Upm mit dem Potter-Homogenisator (10 Kolbenhübe bei 500 Upm) unter Eiskühlung homogenisiert und für 60 Minuten bei 55.000 g bei 4°C zentrifugiert. Der Überstand wurde dekantiert und das Membransediment mit eiskaltem 50 mmol/l TRIS-Acetatpuffer pH 7,0 auf eine Proteinkonzentration von 1 mg/ml eingestellt und erneut mit 10 Kolbenhüben bei 500 Upm homogenisiert und unter Rühren auf einem Magnetrührer im Eisbad in Suspension gehalten. Von diesem Membranhomogenat wurden jeweils 100 µl je 1 ml-Ansatz im Rezeptorbindungstest eingesetzt (0,1 mg Protein/ml im Endansatz).

Im Bindungstest wurde als Puffer 50 mmol/l TRIS-Acetatpuffer pH 7,0 sowie als radioaktiver Ligand 1 nmol/l (³H)-MDL 105.519 (B.M. Baron et al. 1996) eingesetzt. Der Anteil an unspezifischer Bindung wurde in Anwesenheit von 1 mmol/l Glycin bestimmt.

In weiteren Ansätzen wurden die erfindungsgemäßen Verbindungen in Konzentrationsreihen zugegeben und die Verdrängung des radioaktiven Liganden aus seiner spezifischen Bindung an die Glycin-Bindungsstelle des NMDA-Rezeptorkanals ermittelt. Die jeweiligen Dreifachansätze wurden über 120 Minuten bei 4°C inkubiert und anschließend zur Bestimmung des an das Membranhomogenat gebundenen radioaktiven Liganden mittels Filtration durch Glasfaser-Filtermatten (GF/B) geerntet. Die auf den Glasfaser-Filtern zurückgehaltene Radioaktivität wurde nach Zugabe von Szintillator im β-Counter gemessen.

Die Affinität der erfindungsgemäßen Verbindungen zur Glycin-Bindungsstelle des NMDA-Rezeptorkanals wurde als IC₅₀ (Konzentration mit 50 % Verdrängung des radioaktiven Liganden aus seiner spezifischen Bindung) nach dem Massenwirkungsgesetz mittels nichtlinearer Regression berechnet und ist in Tabelle 3 nach Umrechnung (nach der Cheng-Prussoff-Beziehung) als Ki-Wert (Mittelwert +/- Standardabweichung von 3 unabhängigen Versuchen) oder als prozentualer Anteil des gebundenen radioaktiven Liganden s.o., der bei einer Konzentration von 10 µM der zu testenden erfindungsgemäßen Substanz aus seiner spezifischen Bindung verdrängt wird, angegeben

**Tabelle 3**

| **Beispiels-Nr. und Verbindung** | | **Glycin-Bindungsstelle des NMDA-Rezeptorkanals** | |
|---|---|---|---|
| | | **Kᵢ (**µ**mol)** | **Verdrängung (%,10**µ**mol)** |
| **106** | (3,5-Dichlor-phenylamino)-(5-methylsulfanylmethyl-furan-2-yl)-essigsäure | | 65 |
| **106- HCl** | (3,5-Dichlor-phenylamino)-(5-methylsulfanylmethyl-furan-2-yl)-essigsäure-Hydrochlorid | | 91 |
| **106- Na** | (3,5-Dichlor-phenylamino)-(5-methylsulfanylmethyl-furan-2-yl)-essigsäure-Natrium | 2,980 | 78 |
| **107** | (3,5-Dichlor-phenylamino)-(5-mercaptomethyl-furan-2-yl)-essigsäure | | 101 |
| **109** | (3,5-Dichlor-phenylamino)-(5-hydroxymethyl-thiophen-2-yl)-essigsäure | | 66 |
| **110** | (5-Acetylsulfanylmethyl-furan-2-yl)-(3,5-dichlor-phenylamino)-essigsäure | 0,120 | 104 |
| **111** | (3,5-Dichlor-phenylamino)-(5-ethyl-thiophen-2-yl)-essigsäure | | 44 |
| **113** | (3-Chlor-phenylamino)-(5-mercaptomethyl-furan-2-yl)-essigsäure | | 99 |
| **114** | (3,4-Dichlor-phenylamino)-(5-mercaptomethyl-furan-2-yl)-essigsäure | | 45 |
| **119-HCl** | (5-tert.-Butylfuran-2-yl)-(3,5-dichlorphenylamino)-essigsäure-Hydrochlord | | 91 |
| **120** | (3,5-Dichlorphenylamino)-[5-(furan-2-ylmethyldisulfanylmethyl)-furan-2-yl]-essigsäure | 0,470 | 103 |
| **121** | (3,5-Dichlorphenylamino)-(5-methyldisulfanylmethylfuran-2-yl)-essigsäure | 0,290 | 106 |
| **123** | (5-Acetoxymethylfuran-2-yl)-(3,5- dichlorphenylamino)-essigsäure | 5,03 | 60 |
| **125-Na** | (3,5-Dichlorphenylamino)-(5-methylthiophen-2-yl)-essigsäure-Natriumsalz | 5,03 | 63 |
| **126** | (3,5-Dichlorphenylamino)-(4-methylthiophen-2-yl)-essigsäure | 15,38 | 52 |
| **127** | (5-Chlorthiophen-2-yl)-(3,5- dichlorphenylamino)-essigsäure | 2,57 | 68 |
| **127-Na** | (3,5-Dichlorphenylamino)-(5-chlorthiophen-2-yl)-essigsäure-Natriumsalz | | 72 |

### Pharmazeutische Formulierung eines erfindungsgemäßen Arzneimittels

1 g des Hydrochlorids von (3,5-Dichlorphenylamino)-(5-methyldisulfanyl-methylfuran-2-yl)-essigsäure **(121)** wurde in 1 l Wasser für Injektionszwecke bei Raumtemperatur gelöst und anschließend durch Zugabe von Natriumchlorid auf isotone Bedingungen eingestellt.

## Patentansprüche

1. Verbindung der allgemeinen Struktur (I), oder ein pharmazeutisch annehmbares Salz davon, 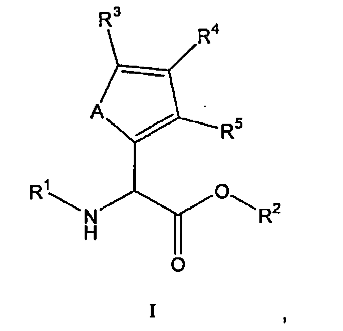 worin
A Sauerstoff oder Schwefel bedeutet,
R¹ Aryl oder Heterocyclyl² bedeutet,
R² H, Methyl, Ethyl, n-Propyl, 2-Propyl, n-Butyl, tert-Butyl, n-Hexyl bedeutet,
R³, R⁴ und R⁵ unabhängig voneinander H, OH, SH, Br, Cl, C₁₋₆-Alkyl, 2-Furyl, 3-Furyl, 2-Thienyl, 3-Thienyl, -SiR⁶R⁷R⁸, -CH₂OH, -CH₂-O-(C=O)-CH₃, -(CH₂)-Sₚ-(CH₂)_{q}-R¹⁰, p = 1 oder 2 und q = 0 oder 1, -(CH₂)ᵣ-CO₂R¹¹ mit r = 0 oder 1 oder -COR¹³ bedeuten,
R⁶, R⁷ und R⁸ unabhängig voneinander Methyl, tert-Butyl oder Phenyl bedeuten,
R¹⁰ H, Methyl, Ethyl, n-Propyl, 2-Furyl, 2-Thienyl oder -(C=O)-CH₃ bedeutet,
R¹¹ H, Methyl, Ethyl oder tert-Butyl bedeutet,
R¹³ Methyl bedeutet,
wobei
Aryl für 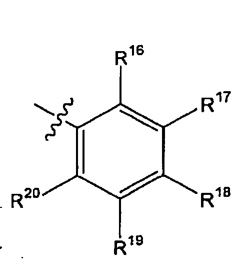 steht,
Heterocyclyl² für 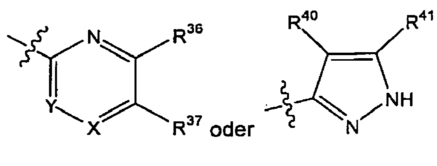 steht,
R¹⁶, R¹⁷, R¹⁸, R¹⁹ und R²⁰ unabhängig voneinander H, OR²⁸, S(O)ₜR²⁹ mit t = 0, F, Cl, Br, I, -CN, -NO₂, Methyl, Ethyl, n-Propyl, 2-Propyl, n-Butyl, 2-Butyl, iso-Butyl, tert-Butyl, n-Pentyl, Neopentyl, Isopentyl, n-Hexyl, iso-Hexyl, CF₃ oder -CO₂R³¹ bedeuten,
R²⁸, R²⁹ und R³¹ unabhängig voneinander H, Methyl, Ethyl, -CF₃ oder Phenyl bedeuten,
X-Y für CR³⁸-CR³⁹, CR³⁸-N oder N-CR³⁹ steht,
R³⁶, R³⁷, R³⁸ und R³⁹ unabhängig voneinander H, F, Cl, Br, I, -CN, -NO₂, -OH, -SH, C₁₋₆-Alkyl oder -CF₃ bedeuten,
und
R⁴⁰ und R⁴¹ unabhängig voneinander H, F, Cl, Br, I, -CN, -OH, -O-C₁₋₆-Alkyl,SH, -S-C₁₋₆-Alkyl, C₁₋₈-Alkyl, CO₂-C₁₋₆-Alkyl oder -N=N-Aryl bedeuten,
wobei (4-Methoxy-phenylamino)-thien-2-yl-essigsäure ausgenommen ist.

2. Verbindung nach Anspruch 1, oder ein pharmazeutisch annehmbares Salz davon, in Form des Racemats, in Form der reinen Enantiomeren oder Diastereomeren oder in Form von Mischungen der Enantiomeren oder Diastereomeren in einem beliebigen Mischungsverhältnis.

3. Verbindung nach einem der Ansprüche 1 oder 2, oder eines ihrer pharmazeutisch annehmbaren Salze,
**dadurch gekennzeichnet, daß**
R¹ Aryl oder Heterocyclyl² bedeutet,
R² H, Methyl, Ethyl oder tert-Butyl bedeutet,
R³ H, Cl, Methyl, Ethyl, n-Propyl, 2-Propyl, n-Butyl, tert-Butyl, CH₂OH, -CH₂SH, -CH₂-S-CH₃, -CH₂-S-CH₂-Furan-2-yl, -CH₂-O-(C=O)-CH₃, -CH₂-S-(C=O)-CH₃, -CH₂-S-S-CH₃, -CH₂-S-S-CH₂-Furan2-yl, -CH₂-CO₂Methyl oder -CH₂-CO₂Ethyl bedeutet,
R⁴ H, Br, Methyl, Ethyl, -CH₂OH, -CO₂Methyl, -CO₂Ethyl oder-C(=O)Methyl bedeutet,
R⁵ H, Methyl oder Ethyl bedeutet,
wobei
Aryl für 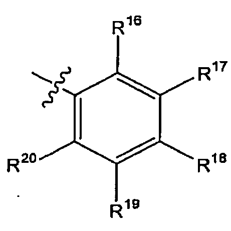 steht,
Heterocyclyl² für 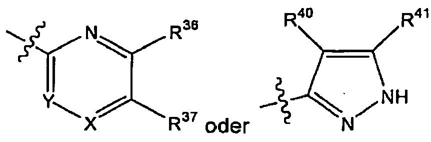 steht,
R¹⁶ H, -O-Phenyl, F, Cl, Br, Methyl, Ethyl, n-Propyl, 2-Propyl oder tert-Butyl bedeutet,
R¹⁷ H, Cl, Methyl, Ethyl oder CF₃ bedeutet,
R¹⁸ H, F, Cl, Br, I, -CN, -O-CH₃, -O-CF₃, -O-Phenyl, Methyl, Ethyl, n-Propyl, 2-Propyl, n-Butyl, 2-Butyl oder tert-ButyL bedeutet,
R¹⁹ H, Cl, Br, Methyl oder Ethyl bedeutet,
R²⁰ H oder Methyl bedeutet,
X-Y für CR³⁸-CR³⁹, CR³⁸-N oder N-CR³⁹ steht,
R³⁶ H, Methyl oder Ethyl bedeutet
R³⁷ H, NO₂, Cl, Br, Methyl oder CF₃ bedeutet;
R³⁸ H bedeutet,
R³⁹ H, Cl oder Br bedeutet,
R⁴⁰ und H, -N=N-Phenyl, -CN, CO₂H, CO₂-Methyl oder CO₂-Ethyl bedeutet,
R⁴¹ H, OH, SH, S-Methyl, Methyl, Ethyl, n-Propyl, 2-Propyl, n-Butyl oder tert-Butyl bedeutet.

4. Verbindung nach einem der Ansprüche 1 bis 3, oder eines ihrer pharmazeutisch annehmbaren Salze, **dadurch gekennzeichnet, daß**
R¹ 4-Trifluormethoxy-phenyl, 2-Phenoxy-phenyl, 4-Phenoxy-phenyl, 2-Chlor-phenyl, 4-Chlor-phenyl, 4-Iod-phenyl, 4-Cyano-phenyl, 2-Methyl-phenyl, 2-Ethyl-phenyl, 4-Ethyl-phenyl, 2-(2-Propyl)-phenyl, 4-(2-Butyl)-phenyl, 4-tert-Butyl-phenyl, 2,4-Dichlorphenyl, 2,3-Dichlorphenyl, 3,5-Dichlorphenyl, 2,4-Dibrom-phenyl, 4-Chlor-2-fluor-phenyl, 2-Chlor-4-fluor-phenyl, 4-Brom-2-chlor-phenyl, 2-Chlor-4-lod-phenyl, 3-Chlor-2-methyl-phenyl, 4-Chlor-2-methyl-phenyl, 5-Chlor-2-methyl-phenyl, 2-Chlor-4-methyl-phenyl, 4-Chlor-3-trifluormethyl-phenyl, 2,4-Dibrom-5-methyl-phenyl, 5-Nitro-pyridin-2-yl, 3,5-Dibrom-pyridin-2-yl, 3,5-Dichlor-pyridin-2-yl, 3-Chlor-5-Trifluormethyl-pyridin-2-yl, 3,5-Dibrom-6-methyl-pyridin-2-yl, Pyrazin-2-yl, 5-Brom-pyrimidin-2-yl, 4-Carboxyethyl-pyrazol-3-yl, 4-Cyano-pyrazol-3-yl, 5-tert-Butyl-pyrazol-3-yl, 5-Hydroxy-4-(4-Phenylazo)-pyrazol-3-yl oder 4-Cyano-5-methylsulfanyl-pyrazol-3-yl bedeutet,
R² H oder Ethyl bedeutet,
R³ H, Cl, Methyl, Ethyl, n-Propyl, tert-Butyl, -CH₂OH, -CH₂SH,-CH₂S-CH₃, -CH₂-S-CH₂-Furan-2-yl, -CH₂-O-(C=O)-CH₃, -CH₂-S-(C=O)-CH₃, -CH₂-S-S-CH₃, -CH₂-S-S-CH₂-Furan2-yl oder -CH₂-CO₂Ethyl bedeutet,
R⁴ H, Br, Methyl, -CH₂OH, -CO₂Methyl, -CO₂Ethyl oder -C(=O)CH₃ bedeutet,
R⁵ H oder Methyl bedeutet.

5. Verbindung nach einem der Ansprüche 1 bis 4, oder eines ihrer pharmazeutisch annehmbaren Salze, **dadurch gekennzeichnet, daß**
R¹ 3,5-Dichlorphenyl bedeutet und
R² H bedeutet.

6. Verbindung nach einem der Ansprüche 1-5, oder eines ihrer pharmazeutisch annehmbaren Salze, **dadurch gekennzeichnet, daß** die Verbindung aus der Gruppe ausgewählt ist, die
• (5-Methylsulfanylmethyl-furan-2-yl)-(5-nitro-pyridin-2-ylamino)-essigsäure
• (5-Brompyrimidin-2-ylamino)-(5-methylsulfanylmethyl-furan-2-yl)-essigsäure
• 5-[Carboxy-(3,5-dichlorpyridin-2-ylamino)-methyl]-2-methyl-furan-3-carbonsäureethylester
• 5-[Carboxy-(3,5-dibrompyridin-2-ylamino)-methyl]-2-methyl-furan-3-carbonsäureethylester
• 5-[Carboxy-(3,5-dibrom-6-methyl-pyridin-2-ylamino)-methyl]-2-methyl-furan-3-carbonsäureethylester
• (3,5-Dibrom-6-methyl-pyridin-2-ylamino)-(4-hydroxymethyl-furan-2-yl)-essigsäure
• (3,5-Dichlor-pyridin-2-ylamino)-(4-methyl-thiophen-2-yl)-essigsäure
• (2,4-Dibrom-5-methyl-phenylamino)-(4-methyl-thiophen-2-yl)-essigsäure
• (4-Methyl-thiophen-2-yl)-(5-nitro-pyridin-2-ylamino)-essigsäure
• (3,5-Dichlor-pyridin-2-ylamino)-furan-2-yl-essigsäure
• (3,5-Dibrompyridin-2-ylamino)-furan-2-yl-ssigsäure
• (3,5-Dibrom-6-methyl-pyridin-2-ylamino)-furan-2-yl-essigsäure
• (3-Chlor-5-trifluormethyl-pyridin-2-ylamino)-furan-2-yl-essigsäure
• (5-Brompyrimidin-2-ylamino)-furan-2-yl-essigsäure
• 5-[(3,5-Dichlor-phenylamino)-ethoxycarbonyl-methyl]-2-methyl-furan-3-carbonsäuremethylester
• (5-Hydroxy-4-phenylazo-1H-pyrazol-3-ylamino)-(5-methylsulfanylmethyl-furan-2-yl)-essigsäureethylester
• 3-{[Ethoxycarbonyl-(4-ethoxycarbonyl-5-methyl-furan-2-yl)-methyl]-amino}-1 H-pyrazol-4-carbosäureethylester
• 5-[(4-Cyano-5-methylsulfanyl-1H-pyrazol-3-ylamino)-ethoxycarbonyl-methyl]-2-methyl-furan-3-carbonsäureethylester
• 5-[(4-Cyano-1H-pyrazol-3-ylamino)-ethoxycarbonyl-methyl]-2-methyl-furan-3-carbonsäureethylester
• 5-[(4-Brom-2-chlor-phenylamino)-ethoxycarbonyl-methyl]-2-methyl-furan-3-carbonsäureethylester
• 5-[(4-Cyano-phenylamino)-ethoxycarbonyl-methyl]-2-methyl-furan-3-carbonsäureethylester
• (4-Hydroxymethyl-furan-2-yl)-(5-hydroxy-4-phenylazo-1H-pyrazol-3-ylamino)-essigsäureethylester
• (4-Cyano-5-methylsulfanyl-1H-pyrazol-3-ylamino)-(4-hydroxymethyl-furan-2-yl)-essigsäureethylester
• (4-Brom-2-chlor-phenylamino)-(4-hydroxymethyl-furan-2-yl)-essigsäureethylester
• (3,5-Dichlor-phenylamino)-(4-hydroxymethyl-furan-2-yl)-essigsäureethylester
• (5-Chlor-2-methyl-phenylamino)-[5-(furan-2-ylmethylsulfanylmethyl)-furan-2-yl]-essigsäureethylester
• (2,4-Dibrom-phenylamino)-(4-methyl-thiophen-2-yl)-essigsäure
• (5-Chlor-2-methyl-phenylamino)-(5-methylsulfanylmethyl-furan-2-yl)-essigsäure
• (2-Ethyl-phenylamino)-(5-methylsulfanylmethyl-furan-2-yl)-essigsäure
• (4-sec-Butyl-phenylamino)-(5-methylsulfanylmethyl-furan-2-yl)-essigsäure
• (5-Methylsulfanylmethyl-furan-2-yl)-(4-trifluormethoxy-phenylamino)-essigsäure
• (2-Isopropyl-phenylamino)-(5-methylsulfanylmethyl-furan-2-yl)-essigsäure
• (2,4-Dibrom-phenylamino)-(5-methylsulfanylmethyl-furan-2-yl)-essigsäure
• (4-*tert*-Butyl-phenylamino)-(5-methylsulfanylmethyl-furan-2-yl)-essigsäure
• (5-Chlor-2-methyl-phenylamino)-[5-(furan-2-ylmethylsulfanylmethyl)-furan-2-yl]-essigsäure
• (2-Ethyl-phenylamino)-[5-(furan-2-ylmethylsulfanylmethyl)-furan-2-yl]-essigsäure
• (4-sec-Butyl-phenylamino)-[5-(furan-2-ylmethylsulfanylmethyl)-furan-2-yl]-essigsäure
• [5-(Furan-2-ylmethylsulfanylmethyl)-furan-2-yl]-(2-isopropyl-phenylamino)-essigsäure
• (4-*tert*-Butyl-phenylamino)-[5-(furan-2-ylmethylsulfanylmethyl)-furan-2-yl]-essigsäure
• 5-[Ethoxycarbonyl-(4-iod-phenylamino)-methyl]-2-methyl-furan-3-carbonsäuremethylester
• 5-[(4-Chlor-2-methyl-phenylamino)-ethoxycarbonyl-methyl]-2-methyl-furan-3-carbonsäuremethylester
• 5-[Ethoxycarbonyl-(4-phenoxy-phenylamino)-methyl]-2-methyl-furan-3-carbonsäuremethylester
• 5-[Ethoxycarbonyl-(4-iod-phenylamino)-methyl]-2-methyl-furan-3-carbonsäureethylester
• 5-[(2-Chlor-phenylamino)-ethoxycarbonyl-methyl]-2-methyl-furan-3-carbonsäureethylester
• 5-[(4-Chlor-2-methyl-phenylamino)-ethoxycarbonyl-methyl]-2-methyl-furan-3-carbonsäureethylester
• 5-[(2-Chlor-4-fluor-phenylamino)-ethoxycarbonyl-methyl]-2-methyl-furan-3-carbonsäureethylester
• 5-[Ethoxycarbonyl-(4-phenoxy-phenylamino)-methyl]-2-methyl-furan-3-carbonsäureethylester
• 5-[(2,3-Dichlor-phenylamino)-ethoxycarbonyl-methyl]-2-methyl-furan-3-carbonsäureethylester
• (4-Hydroxymethyl-furan-2-yl)-(4-iod-phenylamino)-essigsäureethylester
• (2,4-Dichlor-phenylamino)-(4-hydroxymethyl-furan-2-yl)-essigsäureethylester
• (4-Chlor-2-methyl-phenylamino)-(4-hydroxymethyl-furan-2-yl)-essigsäureethylester
• (4-Hydroxymethyl-furan-2-yl)-(4-phenoxy-phenylamino)-essigsäureethylester
• (2,3-Dichlor-phenylamino)-(4-hydroxymethyl-furan-2-yl)-essigsäureethylester
• (2,3-Dichlor-phenylamino)-furan-2-yl-essigsäureethylester
• 5-[Carboxy-(3,5-dibrom-6-methyl-pyridin-2-ylamino)-methyl]-2-methyl-furan-3-carbonsäuremethylester
• (5-Methylsulfanylmethyl-furan-2-yl)-(pyrazin-2-ylamino)-essigsäure
• (3,5-Dibrom-pyridin-2-ylamino)-(5-methylsulfanylmethyl-furan-2-yl)-essigsäure
• (3,5-Dibrom-6-methyl-pyridin-2-ylamino)-(5-methylsulfanylmethyl-furan-2-yl)-essigsäure
• 5-[(4-Brom-2-chlor-phenylamino)-carboxy-methyl]-2-methyl-furan-3-carbonsäuremethylester
• 5-[Carboxy-(4-cyano-phenylamino)-methyl]-2-methyl-furan-3-carbonsäuremethylester
• 5-[Carboxy-(3,5-dichlor-phenylamino)-methyl]-2-methyl-furan-3-carbonsäuremethylester
• 5-[Carboxy-(2-phenoxy-phenylamino)-methyl]-2,4-dimethyl-furan-3-carbonsäureethylester
• 5-[(4-Brom-2-chlor-phenylamino)-carboxy-methyl]-2,4-dimethyl-furan-3-carbonsäureethylester
• 5-[Carboxy-(4-cyano-phenylamino)-methyl]-2,4-dimethyl-furan-3-carbonsäureethylester
• 5-[Carboxy-(3,5-dichlor-phenylamino)-methyl]-2,4-dimethyl-furan-3-carbonsäureethylester
• 5-[(4-Brom-2-chlor-phenylamino)-carboxy-methyl]-2-methyl-furan-3-carbonsäureethylester
• 5-[Carboxy-(4-cyano-phenylamino)-methyl]-2-methyl-furan-3-carbonsäureethylester
• 5-[Carboxy-(3,5-dichlor-phenylamino)-methyl]-2-methyl-furan-3-carbonsäureethylester
• (5-*tert*-Butyl-1H-pyrazol-3-ylamino)-(4-hydroxymethyl-furan-2-yl)-essigsäure
• (4-Brom-2-chlor-phenylamino)-(3-methyl-thiophen-2-yl)-essigsäure
• (4-Cyano-phenylamino)-(3-methyl-thiophen-2-yl)-essigsäure
• (3,5-Dichlor-phenylamino)-(3-methyl-thiophen-2-yl)-essigsäure
• (3,5-Dichlor-phenylamino)-furan-2-yl-essigsäure
• [5-(Furan-2-ylmethylsulfanylmethyl)-furan-2-yl]-(4-phenoxy-phenylamino)-essigsäureethylester
• (5-Ethoxycarbonylmethyl-thiophen-2-yl)-(5-hydroxy-4-phenylazo-1 H-pyrazol-3-ylamino)-essigsäureethylester
• {5-[1-(4-Cyano-1H-pyrazol-3-ylamino)-3-methoxy-2-oxo-propyl]-thiophen-2-yl}-essigsäureethylester
• (4-Ethyl-phenylamino)-(5-methylsulfanylmethyl-furan-2-yl)-essigsäure
• (3-Chlor-2-methyl-phenylamino)-(5-methylsulfanylmethyl-furan-2-yl)-essigsäure
• (4-Chlor-phenylamino)-(5-methylsulfanylmethyl-furan-2-yl)-essigsäure
• (5-Methylsulfanylmethyl-furan-2-yl)-o-tolylamino-essigsäure
• 5-[Carboxy-(4-chlor-3-trifluormethyl-phenylamino)-methyl]-2-methyl-furan-3-carbonsäureethylester
• 5-[Carboxy-(2-chlor-phenylamino)-methyl]-2-methyl-furan-3-carbonsäureethylester
• 5-[Carboxy-(2-chlor-4-fluor-phenylamino)-methyl]-2-methyl-furan-3-carbonsäureethylester
• 5-[Carboxy-(4-chlor-2-fluor-phenylamino)-methyl]-2-methyl-furan-3-carbonsäureethylester
• 5-[Carboxy-(2,3-dichlor-phenylamino)-methyl]-2-methyl-furan-3-carbonsäureethylester
• (2,4-Dichlor-phenylamino)-(4-methyl-thiophen-2-yl)-essigsäure
• (4-Chlor-3-trifluormethyl-phenylamino)-(4-methyl-thiophen-2-yl)-essigsäure
• (2,4-Dichlor-phenylamino)-furan-2-yl-essigsäure
• (4-Chlor-3-trifluormethyl-phenylamino)-furan-2-yl-essigsäure
• (4-lod-phenylamino)-(5-methylsulfanylmethyl-furan-2-yl)-essigsäure
• (2,4-Dichlor-phenylamino)-(5-methylsulfanylmethyl-furan-2-yl)-essigsäure
• (4-Chlor-3-trifluormethyl-phenylamino)-(5-methylsulfanylmethyl-furan-2-yl)-essigsäure
• (2-Chlor-phenylamino)-(5-methylsulfanylmethyl-furan-2-yl)-essigsäure
• (4-Chlor-2-methyl-phenylamino)-(5-methylsulfanylmethyl-furan-2-yl)-essigsäure
• (2-Chlor-4-fluor-phenylamino)-(5-methylsulfanylmethyl-furan-2-yl)-essigsäure
• (2-Chlor-4-methyl-phenylamino)-(5-methylsulfanylmethyl-furan-2-yl)-essigsäure
• (2,3-Dichlor-phenylamino)-(5-methylsulfanylmethyl-furan-2-yl)-essigsäure
• [5-(Furan-2-ylmethylsulfanylmethyl)-furan-2-yl]-(4-iodo-phenylamino)-essigsäure
• (4-Chlor-3-trifluormethyl-phenylamino)-[5-(furan-2-ylmethylsulfanylmethyl)-furan-2-yl]-essigsäure
• (4-Chlor-2-methyl-phenylamino)-[5-(furan-2-ylmethylsulfanylmethyl)-furan-2-yl]-essigsäure
• (2-Chlor-4-methyl-phenylamino)-[5-(furan-2-ylmethylsulfanylmethyl)-furan-2-yl]-essigsäure
• (4-Chlor-3-trifluormethyl-phenylamino)-(5-ethoxycarbonylmethyl-thiophen-2-yl)-essigsäure
• (4-Acetyl-3,5-dimethyl-furan-2-yl)-(2-chlor-4-methyl-phenylamino)-essigsäure
• (4-Acetyl-3,5-dimethyl-furan-2-yl)-(2,3-dichlor-phenylamino)-essigsäure
• (3,5-Dichlor-phenylamino)-(5-methylsulfanylmethyl-furan-2-yl)-essigsäure
• (3-Chlor-phenylamino)-(5-mercaptomethyl-furan-2-yl)-essigsäure
• (3,4-Dichlor-phenylamino)-(5-mercaptomethyl-furan-2-yl)-essigsäure
• (3,5-Dichlor-phenylamino)-(5-mercaptomethyl-furan-2-yl)-essigsäure
• (3,5-Dichlor-phenylamino)-(5-methyl-thiophen-2-yl)-essigsäure
• (3,5-Dichlor-phenylamino)-(5-hydroxymethyl-thiophen-2-yl)-essigsäure
• (5-Acetylsulfanylmethyl-furan-2-yl)-(3,5-dichlor-phenylamino)-essigsäure
• (3,5-Dichlor-phenylamino)-(5-ethyl-thiophen-2-yl)-essigsäure
• (3,5-Dichlor-phenylamino)-(5-n-propyl-thiophen-2-yl)-essigsäure
• (3,5-Dichlor-phenylamino)-[5-(furan-2-ylmethylsulfanylmethyl)-furan-2-yl]-essigsäure
• (3-Chlor-phenylamino)-(5-mercaptomethyl-furan-2-yl)-essigsäure
• (3,4-Dichlor-phenylamino)-(5-mercaptomethyl-furan-2-yl)-essigsäure
• (3,5-Dichlor-phenylamino)-(thiophen-2-yl)-essigsäureethylester
• (4-Bromfuran-2-yl-(3,5-dichlor-phenylamino)-essigsäureethylester
• (3,5-Dichlor-phenylamino)-(5-propylthiophen-2-yl)-essigsäureethylester
• (3,5-Dichlor-phenylamino)-(3-methylthiophen-2-yl)-essigsäureethylester
• (5-tert-Butylfuran-2-yl)-(3,5-dichlor-phenylamino)-essigsäure
• (3,5-Dichlorphenylamino)-[5-(furan-2-ylmethyldisulfanylmethyl)-furan-2-yl]-essigsäure
• (3,5-Dichlorphenylamino)-(5-methyldisulfanylmethylfuran-2-yl)-essigsäure
• (3,5-Dichlorphenylamino)-[5-(furan-2-ylmethylsufanylmethyl)-furan-2-yl]-essigsäure
• 5-Acetoxymethylfuran-2-yl-(3,5-dichlorphenylamino)-essigsäure
• (3,5-Dichlorphenylamino)-thiophen-3-yl-essigsäure
• (3,5-Dichlorphenylamino)-(5-methylthiophen-2-yl)-essigsäure
• (3,5-Dichlorphenylamino)-(4-methylthiophen-2-yl)-essigsäure
• (5-Chlorthiophen-2-yl)-(3,5-dichlorphenylamino)-essigsäure
enthält.

7. Verfahren zur Herstellung einer Verbindung der allgemeinen Struktur (I), oder eines ihrer pharmazeutisch annehmbaren Salze, 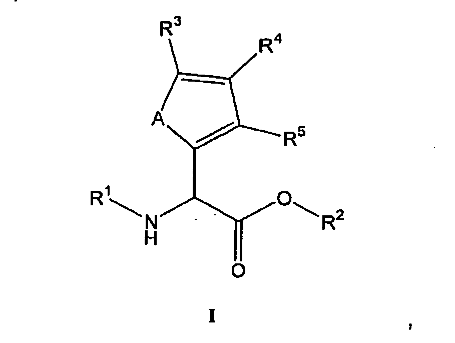 worin
A Sauerstoff oder Schwefel bedeutet,
R¹ Aryl oder Heterocyclyl² bedeutet,
R² H, Methyl, Ethyl, n-Propyl, 2-Propyl, n-Butyl, tert-Butyl, n-Hexyl bedeutet,
R³, R⁴ und R⁵ unabhängig voneinander H, OH, SH, Br, Cl, C₁₋₆-Alkyl, 2-Furyl, 3-Furyl, 2-Thienyl, 3-Thienyl, -SiR⁶R⁷R⁸, -CH₂OH, -CH₂-O-(C=O)-CH₃, -(CH₂)-Sₚ-(CH₂)_{q}-R¹⁰, p = 1 oder 2 und q = 0 oder 1, -(CH₂)ᵣ-CO₂R¹¹ mit r = 0 oder 1 oder -COR¹³ bedeuten,
R⁶, R⁷ und R⁸ unabhängig voneinander Methyl, tert-Butyl oder Phenyl bedeuten,
R¹⁰ H, Methyl, Ethyl, n-Propyl, 2-Furyl, 2-Thienyl oder -(C=O)-CH₃ bedeutet,
R¹¹ H, Methyl, Ethyl oder tert-Butyl bedeutet,
R¹³ Methyl bedeutet,
wobei
Aryl für 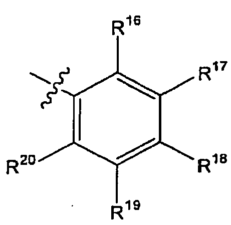 steht,
Heterocyclyl² für 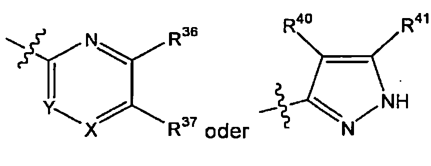 steht,
R¹⁶, R¹⁷, R¹⁸, R¹⁹ und R²⁰ unabhängig voneinander H, OR²⁸, S(O)ₜR²⁹ mit t = 0, F, Cl, Br, I, -CN, -NO₂, Methyl, Ethyl, n-Propyl, 2-Propyl, n-Butyl, 2-Butyl, iso-Butyl, tert-Butyl, n-Pentyl, Neopentyl, Isopentyl, n-Hexyl, iso-Hexyl, CF₃ oder -CO₂R³¹ bedeuten,
R²⁸, R²⁹ und R³¹ unabhängig voneinander H, Methyl, Ethyl, -CF₃ oder Phenyl bedeuten,
X-Y für CR³⁸-CR³⁹, CR³⁸-N oder N-CR³⁹ steht,
R³⁶, R³⁷, R³⁸ und R³⁹ unabhängig voneinander H, F, Cl, Br, I, -CN, -NO₂, -OH, -SH, C₁₋₆-Alkyl oder -CF₃ bedeuten,
und
R⁴⁰ und R⁴¹ unabhängig voneinander H, F, Cl, Br, I, -CN, -OH, -O-C₁₋₆-Alkyl,-SH, -S-C₁₋₆-Alkyl, C₁₋₈-Alkyl, CO₂-C₁₋₆-Alkyl oder-N=N-Aryl bedeuten.
**dadurch gekennzeichnet, daß**
ein Amin der allgemeinen Struktur (II)
R¹-NH² II,
worin R¹ wie oben in diesem Anspruch definiert ist,
mit einem Glyoxylsäurederivat der allgemeinen Struktur (III) 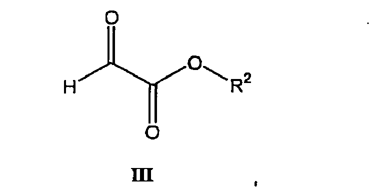 worin R² wie oben in diesem Anspruch definiert ist,
und einem Heterocyclus der allgemeinen Struktur (IV) 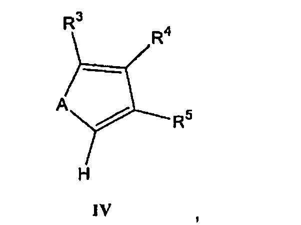 worin A, R³, R⁴ und R⁵ wie oben in diesem Anspruch definert sind,
unter Einwirken einer Säure umgesetzt wird.

8. Verfahren nach Anspruch 7, **dadurch gekennzeichnet, daß** es als Eintopf-Verfahren ausgeführt wird.

9. Verfahren nach Anspruch 7 oder 8, **dadurch gekennzeichnet, daß** die Umsetzung der Verbindungen (II), (III) und (IV) unter Einwirken von Mikrowellenstrahlung ausgeführt wird.

10. Verfahren nach einem der Ansprüche 7 bis 9, **dadurch gekennzeichnet, daß** das Verfahren in einem organischen Lösungsmittel bei einer Temperatur von 0 °C bis 100 °C, insbesondere 15 °C bis 50 °C ausgeführt wird.

11. Arzneimittel, enthaltend eine Verbindung der allgemeinen Struktur (I), oder eines ihrer pharmazeutischen Salze, 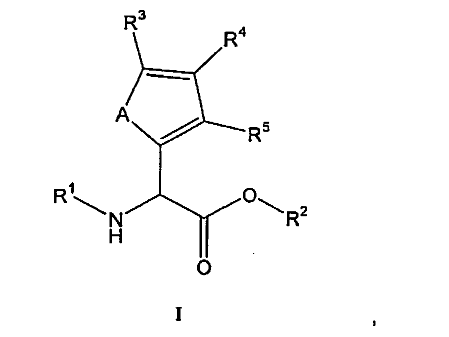 worin
A Sauerstoff oder Schwefel bedeutet,
R¹ Aryl oder Heterocyclyl² bedeutet,
R² H, Methyl, Ethyl, n-Propyl, 2-Propyl, n-Butyl, tert-Butyl, n-Hexyl bedeutet,
R³, R⁴ und R⁵ unabhängig voneinander H, OH, SH, Br, Cl C₁₋₆-Alkyl, 2-Furyl, 3-Furyl, 2-Thienyl, 3-Thienyl, -SiR⁶R⁷R⁸, -CH₂OH, CH₂-O-(C=O)-CH₃, -(CH₂)-Sₚ-(CH₂)_{q}-R¹⁰, p = 1 oder 2 und q = 0 oder 1, -(CH₂)ᵣCO₂R¹¹ mit r= 0 oder 1 oder-COR¹³ bedeuten,
R⁶, R⁷ und R⁸ unabhängig voneinander Methyl, tert-Butyl oder Phenyl bedeuten,
R¹⁰ H, Methyl, Ethyl, n-Propyl, 2-Furyl, 2-Thienyl oder -(C=O)-CH₃ bedeutet,
R¹¹ H, Methyl, Ethyl oder tert-Butyl bedeutet,
R¹³ Methyl bedeutet,
wobei
Aryl für 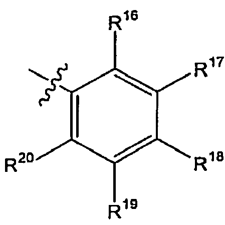 steht,
Heterocyclyl² für 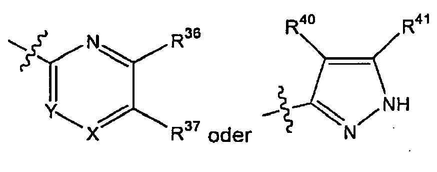 steht,
R¹⁶, R¹⁷, R¹⁸, R¹⁹ und R²⁰ unabhängig voneinander H, OR²⁸, S(O)ₜR²⁹ mit t = 0, F, Cl, Br, I, -CN, -NO₂, Methyl, Ethyl, n-Propyl, 2-Propyl, n-Butyl, 2-Butyl, iso-Butyl, tert-Butyl, n-Pentyl, Neopentyl, Isopentyl, n-Hexyl, iso-Hexyl, CF₃ oder-CO₂R³¹ bedeuten,
R²⁸, R²⁹ und R³¹ unabhängig voneinander H, Methyl, Ethyl, -CF₃ oder Phenyl bedeuten,
X-Y für CR³⁸-CR³⁹, CR³⁸-N oder N-CR³⁹ steht,
R³⁶, R³⁷, R³⁸ und R³⁹ unabhängig voneinander H, F, Cl, Br, I, -CN, -NO₂, -OH, -SH, C₁₋₆-Alkyl oder-CF₃ bedeuten,
und
R⁴⁰ und R⁴¹ unabhängig voneinander H, F, Cl, Br, I, -CN, -OH, -O-C₁₋₆-Alkyl,-SH, -S-C₁₋₆Alkyl, C₁₋₈-Alkyl, CO₂-C₁₋₆-Alkyl oder-N=N-Aryl bedeuten.

12. Verwendung einer Verbindung der allgemeinen und wie in Anspruch 1 definierten Struktur (I), oder eines ihrer pharmazeutisch annehmbaren Salze, zur Herstellung eines Medikaments zur Behandlung von Schmerz.

13. Verwendung einer Verbindung der allgemeinen und wie in Anspruch 1 definierten Struktur (I), oder eines ihrer pharmazeutisch annehmbaren Salze, zur Herstellung eines Medikaments zur Behandlung von Migräne.

14. Pharmazeutische Zusammensetzung, die mindestens eine Verbindung der allgemeinen und wie in Anspruch 1 definierten Struktur (I), oder eines ihrer pharmazeutisch annehmbaren Salze, sowie mindestens einen pharmazeutischen Hilfsstoff enthält.

## Claims

1. Compound having the general structure (I), or a pharmaceutically acceptable salt thereof, 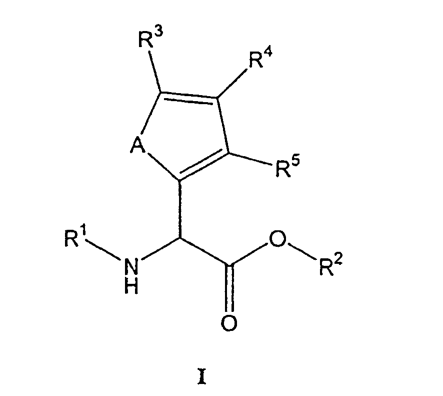 wherein
A represents oxygen or sulfur,
R¹ represents aryl or heterocyclyl²,
R² represents H, methyl, ethyl, n-propyl, 2-propyl, n-butyl, tert-butyl, n-hexyl,
R³, R⁴ and R⁵ each independently of the others represents H, OH, SH, Br, Cl, C₁₋₆-alkyl, 2-furyl, 3-furyl, 2-thienyl, 3-thienyl, -SiR⁶R⁷R⁸, -CH₂OH, -CH₂-O-(C=O)-CH₃, -(CH₂)-Sₚ-(CH₂)_{q}-R¹⁰ wherein p = 1 or 2 and q = 0 or 1, -(CH2)ᵣ-CO₂R¹¹ wherein r = 0 or 1, or -COR¹³,
R⁶, R⁷ and R⁸ each independently of the others represents methyl, tert-butyl or phenyl,
R¹⁰ represents H, methyl, ethyl, n-propyl, 2-furyl, 2-thienyl or -C(=O)-CH₃,
R¹¹ represents H, methyl, ethyl or tert-butyl,
R¹³ represents methyl,
wherein aryl represents 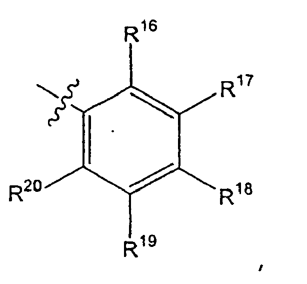
heterocyclyl² represents 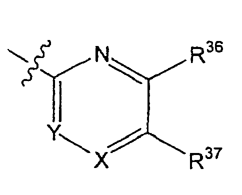 or 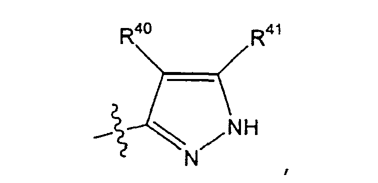
R¹⁶, R¹⁷, R¹⁸, R¹⁹ and R²⁰ each independently of the others represents H, OR²⁸, S(O)ₜR²⁹ wherein t = 0, F, Cl, Br, I, -CN, -NO₂, methyl, ethyl, n-propyl, 2-propyl, n-butyl, 2-butyl, isobutyl, tert-butyl, n-pentyl, neopentyl, isopentyl, n-hexyl, isohexyl, CF₃ or -CO₂R³¹,
R²⁸, R²⁹ and R³¹ each independently of the others represents H, methyl, ethyl, -CF₃ or phenyl,
X-Y represents CR³⁸-CR³⁹, CR³¹-N or N-CR³⁹,
R³⁶, R³⁷, R³⁸ and R³⁹ each independently of the others represents H, F, Cl, Br, I, -CN, -NO₂, -OH, -SH, C₁₋₆-alkyl or -CF₃, and
R⁴⁰ and R⁴¹ each independently of the other represents H, F, Cl, Br, I, -CN, -OH, -O-C₁₋₆-alkyl, -SH, 1-S-C₁₋₆-alkyl, C₁₋₈-alkyl, CO₂-C₁₋₆-alkyl or -N=N-aryl,
with the exception of (4-methoxy-phenylamino)-thien-2-yl-acetic acid.

2. Compound according to claim 1, or a pharmaceutically acceptable salt thereof, in the form of the racemate, in the form of the pure enantiomers or diastereoisomers or in the form of mixtures of the enantiomers or diastereoisomers in any desired mixing ratio.

3. Compound according to one of claims 1 to 2, or a pharmaceutically acceptable salt thereof,
**characterised in that**
R¹ represents aryl or heterocyclyl²,
R² represents H, methyl, ethyl or tert-butyl,
R³ represents H, Cl, methyl, ethyl, n-propyl, 2-propyl, n-butyl, tert-butyl, -CH₂OH, -CH₂SH, -CH₂-S-CH₃, -CH₂-S-CH₂-furan-2-yl, -CH₂-O-(C=O)-CH₃, -CH₂-S-(C=O)-CH₃, -CH₂-S-S-CH₃, -CH₂-S-S-CH₂-furan-2-yl, -CH₂-CO₂methyl or -CH₂-CO₂ethyl,
R⁴ represents H, Br, methyl, ethyl, -CH₂OH, -CO₂methyl, -CO₂ethyl or -C(=O)methyl,
R⁵ represents H, methyl or ethyl,
wherein aryl represents 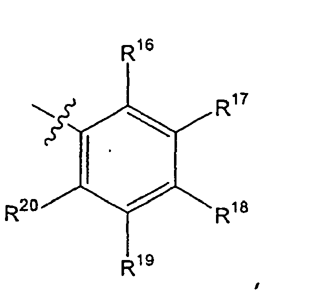
heterocyclyl² represents 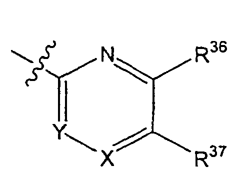 or 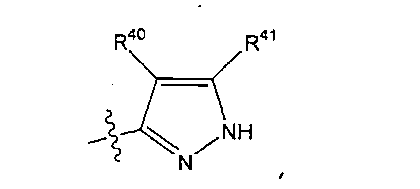
R¹⁶ represents H, -O-phenyl, F, Cl, Br, methyl, ethyl, n-propyl, 2-propyl or tert-butyl,
R¹⁷ represents H, Cl, methyl, ethyl or CF₃,
R¹⁸ represents H, F, Cl, Br, I, -CN, -O-CH₃, -O-CF₃, -O-phenyl, methyl, ethyl, n-propyl, 2-propyl, n-butyl, 2-butyl or tert-butyl,
R¹⁹ represents H, Cl, Br, methyl or ethyl,
R²⁰ represents H or methyl,
X-Y represents CR³⁸-CR³⁹, CR³⁸-N or N-CR³⁹,
R³⁶ represents H, methyl or ethyl,
R³⁷ represents H, NO₂, Cl, Br, methyl or CF₃,
R³⁸ represents H,
R³⁹ represents H, Cl or Br,
R⁴⁰ represents H, -N=N-phenyl, -CN, CO₂H, CO₂-methyl or CO₂-ethyl,
and
R⁴¹ represents H, OH, SH, S-methyl, methyl, ethyl, n-propyl, 2-propyl, n-butyl or tert-butyl.

4. Compound according to any one of claims 1 to 3, or a pharmaceutically acceptable salt thereof, **characterised in that**
R¹ represents 4-trifluoromethoxy-phenyl, 2-phenoxy-phenyl, 4-phenoxy-phenyl, 2-chloro-phenyl, 4-chloro-phenyl, 4-iodo-phenyl, 4-cyano-phenyl, 2-methyl-phenyl, 2-ethyl-phenyl, 4-ethyl-phenyl, 2-(2-propyl)-phenyl, 4-(2-butyl)-phenyl, 4-tert-butyl-phenyl, 2,4-dichlorophenyl, 2,3-dichlorophenyl, 3,5-dichlorophenyl, 2,4-dibromo-phenyl, 4-chloro-2-fluoro-phenyl, 2-chloro-4-fluoro-phenyl, 4-bromo-2-chloro-phenyl, 2-chloro-4-iodo-phenyl, 3-chloro-2-methyl-phenyl, 4-chloro-2-methyl-phenyl, 5-chloro-2-methyl-phenyl, 2-chloro-4-methyl-phenyl, 4-chloro-3-trifluoromethyl-phenyl, 2,4-dibromo-5-methyl-phenyl, 5-nitro-pyridin-2-yl, 3,5-dibromo-pyridin-2-yl, 3,5-dichloro-pyridin-2-yl, 3-chloro-5-trifluoromethyl-pyridin-2-yl, 3,5-dibromo-6-methyl-pyridin-2-yl, pyrazin-2-yl, 5-bromo-pyrimidin-2-yl, 4-carboxy-ethyl-pyrazol-3-yl, 4-cyano-pyrazol-3-yl, 5-tert-butyl-pyrazol-3-yl, 5-hydroxy-4-(4-phenylazo)-pyrazol-3-yl or 4-cyano-5-methylsulfanyl-pyrazol-3-yl,
R² represents H or ethyl,
R³ represents H, Cl, methyl, ethyl, n-propyl, tert-butyl, -CH₂OH, -CH₂SH, -CH₂S-CH₃, -CH₂-S-CH₂-furan-2-yl, -CH₂-O-(C=O)-CH₃, -CH₂-S-(C=O)-CH₃-, -CH₂-S-S-CH₃, -CH₂-S-S-CH₂-furan-2-yl or -CH₂-CO₂ethyl,
R⁴ represents H, Br, methyl, -CH₂OH, -CO₂methyl, -CO₂ethyl or -C(=O)CH₃,
R⁵ represents H or methyl.

5. Compound according to any one of claims 1 to 4, or a pharmaceutically acceptable salt thereof, **characterised in that**
R¹ represents 3,5-dichlorophenyl and
R² represents H.

6. Compound according to any one of claims 1 to 5, or a pharmaceutically acceptable salt thereof, **characterised in that** the compound is selected from the group containing
• (5-methylsulfanylmethyl-furan-2-yl)-(5-nitro-pyridin-2-ylamino)-acetic acid
• (5-bromopyrimidin-2-ylamino)-(5-methylsulfanylmethyl-furan-2-yl)-acetic acid
• 5-[carboxy-(3,5-dichloropyridin-2-ylamino)-methyl]-2-methyl-furan-3-carboxylic acid ethyl ester
• 5-[carboxy-(3,5-dibromopyridin-2-ylamino)-methyl]-2-methyl-furan-3-carboxylic acid ethyl ester
• 5-[carboxy-(3,5-dibromo-6-methyl-pyridin-2-ylamino)-methyl]-2-methyl-furan-3-carboxylic acid ethyl ester
• (3,5-dibromo-6-methyl-pyridin-2-ylamino)-(4-hydroxymethyl-furan-2-yl)-acetic acid
• (3,5-dichloro-pyridin-2-ylamino)-(4-methyl-thiophen-2-yl)-acetic acid
• (2,4-dibromo-5-methyl-phenylamino)-(4-methyl-thiophen-2-yl)-acetic acid
• (4-methyl-thiophen-2-yl)-(5-nitro-pyridin-2-ylamino)-acetic acid
• (3,5-dichloro-pyridin-2-ylamino)-furan-2-yl-acetic acid
• (3,5-dibromopyridin-2-ylamino)-furan-2-yl-acetic acid
• (3,5-dibromo-6-methyl-pyridin-2-ylamino)-furan-2-yl-acetic acid
• (3-chloro-5-trifluoromethyl-pyridin-2-ylamino)-furan-2-yl-acetic acid
• (5-bromopyrimidin-2-ylamino)-furan-2-yl-acetic acid
• 5-[(3,5-dichloro-phenylamino)-ethoxycarbonylmethyl]-2-methyl-furan-3-carboxylic acid methyl ester
• (5-hydroxy-4-phenylazo-1H-pyrazol-3-ylamino)-(5-methylsulfanylmethyl-furan-2-yl)-acetic acid ethyl ester
• 3-{[ethoxycarbonyl-(4-ethoxycarbonyl-5-methyl-furan-2-yl)-methyl]-amino}-1H-pyrazole-4-carboxylic acid ethyl ester
• 5-[(4-cyano-5-methylsulfanyl-1H-pyrazol-3-ylamino)-ethoxycarbonyl-methyl]-2-methyl-furan-3-carboxylic acid ethyl ester
• 5-[(4-cyano-1H-pyrazol-3-ylamino)-ethoxycarbonylmethyl]-2-methyl-furan-3-carboxylic acid ethyl ester
• 5-[(4-bromo-2-chloro-phenylamino)-ethoxycarbonylmethyl]-2-methyl-furan-3-carboxylic acid ethyl ester
• 5-[(4-cyano-phenylamino)-ethoxycarbonyl-methyl]-2-methyl-furan-3-carboxylic acid ethyl ester
• (4-hydroxymethyl-furan-2-yl)-(5-hydroxy-4-phenylazo-1H-pyrazol-3-ylamino)-acetic acid ethyl ester
• (4-cyano-5-methylsulfanyl-1H-pyrazol-3-ylamino)-(4-hydroxymethyl-furan-2-yl)-acetic acid ethyl ester
• (4-bromo-2-chloro-phenylamino)-(4-hydroxymethyl-furan-2-yl)-acetic acid ethyl ester
• (3,5-dichloro-phenylamino)-(4-hydroxymethyl-furan-2-yl)-acetic acid ethyl ester
• (5-chloro-2-methyl-phenylamino)-[5-(furan-2-ylmethylsulfanylmethyl)-furan-2-yl]-acetic acid ethyl ester
• (2,4-dibromo-phenylamino)-(4-methyl-thiophen-2-yl)-acetic acid
• (5-chloro-2-methyl-phenylamino)-(5-methylsulfanyl-methyl-furan-2-yl)-acetic acid
• (2-ethyl-phenylamino)-(5-methylsulfanylmethyl-furan-2-yl)-acetic acid
• (4-sec-butyl-phenylamino)-(5-methylsulfanylmethyl-furan-2-yl)-acetic acid
• (5-methylsulfanylmethyl-furan-2-yl)-(4-trifluoromethoxy-phenylamino)-acetic acid
• (2-isopropyl-phenylamino)-(5-methylsulfanylmethyl-furan-2-yl)-acetic acid
• (2,4-dibromo-phenylamino)-(5-methylsulfanylmethyl-furan-2-yl)-acetic acid
• (4-tert-butyl-phenylamino)-(5-methylsulfanylmethyl-furan-2-yl)-acetic acid
• (5-chloro-2-methyl-phenylamino)-[5-(furan-2-ylmethylsulfanylmethyl)-furan-2-yl]-acetic acid
• (2-ethyl-phenylamino)-[5-(furan-2-ylmethylsulfanyl-methyl)-furan-2-yl]-acetic acid
• (4-sec-butyl-phenylamino)-[5-(furan-2-ylmethyl-sulfanylmethyl)-furan-2-yl]-acetic acid
• [5-(furan-2-ylmethylsulfanylmethyl)-furan-2-yl]-(2-isopropyl-phenylamino)-acetic acid
• (4-tert-butyl-phenylamino)-[5-(furan-2-ylmethyl-sulfanylmethyl)-furan-2-yl]-acetic acid
• 5-[ethoxycarbonyl-(4-iodo-phenylamino)-methyl]-2-methyl-furan-3-carboxylic acid methyl ester
• 5-[(4-chloro-2-methyl-phenylamino)-ethoxycarbonylmethyl]-2-methyl-furan-3-carboxylic acid methyl ester
• 5-[ethoxycarbonyl-(4-phenoxy-phenylamino)-methyl]-2-methyl-furan-3-carboxylic acid methyl ester
• 5-[ethoxycarbonyl-(4-iodo-phenylamino)-methyl]-2-methyl-furan-3-carboxylic acid ethyl ester
• 5-[(2-chloro-phenylamino)-ethoxycarbonyl-methyl]-2-methyl-furan-3-carboxylic acid ethyl ester
• 5-[(4-chloro-2-methyl-phenylamino)-ethoxycarbonylmethyl]-2-methyl-furan-3-carboxylic acid ethyl ester
• 5-[(2-chloro-4-fluoro-phenylamino)-ethoxycarbonylmethyl]-2-methyl-furan-3-carboxylic acid ethyl ester
• 5-[ethoxycarbonyl-(4-phenoxy-phenylamino)-methyl]-2-methyl-furan-3-carboxylic acid ethyl ester
• 5-[(2,3-dichloro-phenylamino)-ethoxycarbonylmethyl]-2-methyl-furan-3-carboxylic acid ethyl ester
• (4-hydroxymethyl-furan-2-yl)-(4-iodo-phenylamino)-acetic acid ethyl ester
• (2,4-dichloro-phenylamino)-(4-hydroxymethyl-furan-2-yl)-acetic acid ethyl ester
• (4-chloro-2-methyl-phenylamino)-(4-hydroxymethyl-furan-2-yl)-acetic acid ethyl ester
• (4-hydroxymethyl-furan-2-yl)-(4-phenoxy-phenyl-amino)-acetic acid ethyl ester
• (2,3-dichloro-phenylamino)-(4-hydroxymethyl-furan-2-yl)-acetic acid ethyl ester
• (2,3-dichloro-phenylamino)-furan-2-yl-acetic acid ethyl ester
• 5-[carboxy-(3,5-dibromo-6-methyl-pyridin-2-ylamino)-methyl]-2-methyl-furan-3-carboxylic acid methyl ester
• (5-methylsulfanylmethyl-furan-2-yl)-(pyrazin-2-ylamino)-acetic acid
• (3,5-dibromo-pyridin-2-ylamino)-(5-methylsulfanyl-methyl-furan-2-yl)-acetic acid
• (3,5-dibromo-6-methyl-pyridin-2-ylamino)-(5-methyl-sulfanylmethyl-furan-2-yl)-acetic acid
• 5-[(4-bromo-2-chloro-phenylamino)-carboxy-methyl]-2-methyl-furan-3-carboxylic acid methyl ester
• 5-[carboxy-(4-cyano-phenylamino)-methyl]-2-methylfuran-3-carboxylic acid methyl ester
• 5-[carboxy-(3,5-dichloro-phenylamino)-methyl]-2-methyl-furan-3-carboxylic acid methyl ester
• 5-[carboxy-(2-phenoxy-phenylamino)-methyl]-2,4-dimethyl-furan-3-carboxylic acid ethyl ester
• 5-[(4-bromo-2-chloro-phenylamino)-carboxy-methyl]-2,4-dimethyl-furan-3-carboxylic acid ethyl ester
• 5-[carboxy-(4-cyano-phenylamino)-methyl]-2,4-dimethyl-furan-3-carboxylic acid ethyl ester
• 5-[carboxy-(3,5-dichloro-phenylamino)-methyl]-2,4-dimethyl-furan-3-carboxylic acid ethyl ester
• 5-[(4-bromo-2-chloro-phenylamino)-carboxy-methyl]-2-methyl-furan-3-carboxylic acid ethyl ester
• 5-[carboxy-(4-cyano-phenylamino)-methyl]-2-methylfuran-3-carboxylic acid ethyl ester
• 5-[carboxy-(3,5-dichloro-phenylamino)-methyl]-2-methyl-furan-3-carboxylic acid ethyl ester
• (5-tert-butyl-1H-pyrazol-3-ylamino)-(4-hydroxymethyl-furan-2-yl)-acetic acid
• (4-bromo-2-chloro-phenylamino)-(3-methyl-thiophen-2-yl)-acetic acid
• (4-cyano-phenylamino)-(3-methyl-thiophen-2-yl)-acetic acid
• (3,5-dichloro-phenylamino)-(3-methyl-thiophen-2-yl)-acetic acid
• (3,5-dichloro-phenylamino)-furan-2-yl-acetic acid
• [5-(furan-2-ylmethylsulfanylmethyl)-furan-2-yl]-(4-phenoxy-phenylamino)-acetic acid ethyl ester
• (5-ethoxycarbonylmethyl-thiophen-2-yl)-(5-hydroxy-4-phenylazo-1H-pyrazol-3-ylamino)-acetic acid ethyl ester
• {5-[1-(4-cyano-1H-pyrazol-3-ylamino)-3-methoxy-2-oxo-propyl]-thiophen-2-yl}-acetic acid ethyl ester
• (4-ethyl-phenylamino)-(5-methylsulfanylmethyl-furan-2-yl)-acetic acid
• (3-chloro-2-methyl-phenylamino)-(5-methylsulfanyl-methyl-furan-2-yl)-acetic acid
• (4-chloro-phenylamino)-(5-methylsulfanylmethyl-furan-2-yl)-acetic acid
• (5-methylsulfanylmethyl-furan-2-yl)-o-tolylamino-acetic acid
• 5-[carboxy-(4-chloro-3-trifluoromethyl-phenylamino)-methyl]-2-methyl-furan-3-carboxylic acid ethyl ester
• 5-[carboxy-(2-chloro-phenylamino)-methyl]-2-methylfuran-3-carboxylic acid ethyl ester
• 5-[carboxy-(2-chloro-4-fluoro-phenylamino)-methyl]-2-methyl-furan-3-carboxylic acid ethyl ester
• 5-[carboxy-(4-chloro-2-fluoro-phenylamino)-methyl]-2-methyl-furan-3-carboxylic acid ethyl ester
• 5-[carboxy-(2,3-dichloro-phenylamino)-methyl]-2-methyl-furan-3-carboxylic acid ethyl ester
• (2,4-dichloro-phenylamino)-(4-methyl-thiophen-2-yl)-acetic acid
• (4-chloro-3-trifluoromethyl-phenylamino)-(4-methyl-thiophen-2-yl)-acetic acid
• (2,4-dichloro-phenylamino)-furan-2-yl-acetic acid
• (4-chloro-3-trifluoromethyl-phenylamino)-furan-2-yl-acetic acid
• (4-iodo-phenylamino)-(5-methylsulfanylmethyl-furan-2-yl)-acetic acid
• (2,4-dichloro-phenylamino)-(5-methylsulfanylmethyl-furan-2-yl)-acetic acid
• (4-chloro-3-trifluoromethyl-phenylamino)-(5-methyl-sulfanylmethyl-furan-2-yl)-acetic acid
• (2-chloro-phenylamino)-(5-methylsulfanylmethyl-furan-2-yl)-acetic acid
• (4-chloro-2-methyl-phenylamino)-(5-methylsulfanyl-methyl-furan-2-yl)-acetic acid
• (2-chloro-4-fluoro-phenylamino)-(5-methylsulfanyl-methyl-furan-2-yl)-acetic acid
• (2-chloro-4-methyl-phenylamino)-(5-methylsulfanyl-methyl-furan-2-yl)-acetic acid
• (2,3-dichloro-phenylamino)-(5-methylsulfanylmethyl-furan-2-yl)-acetic acid
• [5-(furan-2-ylmethylsulfanylmethyl)-furan-2-yl]-(4-iodo-phenylamino)-acetic acid
• (4-chloro-3-trifluoromethyl-phenylamino)-[5-(furan-2-ylmethylsulfanylmethyl)-furan-2-yl]-acetic acid
• (4-chloro-2-methyl-phenylamino)-[5-(furan-2-ylmethylsulfanylmethyl)-furan-2-yl]-acetic acid
• (2-chloro-4-methyl-phenylamino)-[5-(furan-2-ylmethylsulfanylmethyl)-furan-2-yl]-acetic acid
• (4-chloro-3-trifluoromethyl-phenylamino)-(5-ethoxycarbonylmethyl-thiophen-2-yl)-acetic acid
• (4-acetyl-3,5-dimethyl-furan-2-yl)-(2-chloro-4-methyl-phenylamino)-acetic acid
• (4-acetyl-3,5-dimethyl-furan-2-yl)-(2,3-dichloro-phenylamino)-acetic acid
• (3,5-dichloro-phenylamino)-(5-methylsulfanylmethyl-furan-2-yl)-acetic acid
• (3-chloro-phenylamino)-(5-mercaptomethyl-furan-2-yl)-acetic acid
• (3,4-dichloro-phenylamino)-(5-mercaptomethyl-furan-2-yl)-acetic acid
• (3,5-dichloro-phenylamino)-(5-mercaptomethyl-furan-2-yl)-acetic acid
• (3,5-dichloro-phenylamino)-(5-methyl-thiophen-2-yl)-acetic acid
• (3,5-dichloro-phenylamino)-(5-hydroxymethyl-thiophen-2-yl)-acetic acid
• (5-acetylsulfanylmethyl-furan-2-yl)-(3,5-dichloro-phenylamino)-acetic acid
• (3,5-dichloro-phenylamino)-(5-ethyl-thiophen-2-yl)-acetic acid
• (3,5-dichloro-phenylamino)-(5-n-propyl-thiophen-2-yl)-acetic acid
• (3,5-dichloro-phenylamino)-[5-(furan-2-ylmethyl-sulfanylmethyl)-furan-2-yl]-acetic acid
• (3-chloro-phenylamino)-(5-mercaptomethyl-furan-2-yl)-acetic acid
• (3,4-dichloro-phenylamino)-(5-mercaptomethyl-furan-2-yl)-acetic acid
• (3,5-dichloro-phenylamino)-(thiophen-2-yl)-acetic acid ethyl ester
• (4-bromofuran-2-yl)-(3,5-dichloro-phenylamino)-acetic acid ethyl ester
• (3,5-dichloro-phenylamino)-(5-propylthiophen-2-yl)-acetic acid ethyl ester
• (3,5-dichloro-phenylamino)-(3-methylthiophen-2-yl)-acetic acid ethyl ester
• (5-tert-butylfuran-2-yl)-(3,5-dichloro-phenylamino)-acetic acid
• (3,5-dichlorophenylamino)-[5-(furan-2-ylmethyl-disulfanylmethyl)-furan-2-yl]-acetic acid
• (3,5-dichlorophenylamino)-(5-methyldisulfanylmethyl-furan-2-yl)-acetic acid
• (3,5-dichlorophenylamino)-[5-(furan-2-ylmethyl-sulfanylmethyl)-furan-2-yl]-acetic acid
• 5-acetoxymethylfuran-2-yl-(3,5-dichlorophenylamino)-acetic acid
• (3,5-dichlorophenylamino)-thiophen-3-yl-acetic acid
• (3,5-dichlorophenylamino)-(5-methylthiophen-2-yl)-acetic acid
• (3,5-dichlorophenylamino)-(4-methylthiophen-2-yl)-acetic acid
• (5-chlorothiophen-2-yl)-(3,5-dichlorophenylamino)-acetic acid.

7. Process for the preparation of a compound having the general structure (I), or of a pharmaceutically acceptable salt thereof, 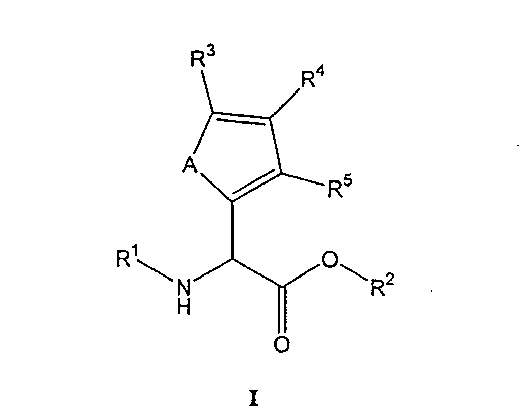 wherein
A represents oxygen or sulfur,
R¹ represents aryl or heterocyclyl²,
R² represents H, methyl, ethyl, n-propyl, 2-propyl, n-butyl, tert-butyl, n-hexyl,
R³, R⁴ and R⁵ each independently of the others represents H, OH, SH, Br, Cl, C₁₋₆-alkyl, 2-furyl, 3-furyl, 2-thienyl, 3-thienyl, -SiR⁶R⁷R⁸, -CH₂OH, -CH₂-O-(C=O)-CH₃, -(CH₂)-Sₚ-(CH₂)_{q}-R¹⁰ wherein p = 1 or 2 and q = 0 or 1, -(CH₂)ᵣ-CO₂R¹¹ wherein r = 0 or 1, or -COR¹³,
R⁶, R⁷ and R⁸ each independently of the others represents methyl, tert-butyl or phenyl,
R¹⁰ represents H, methyl, ethyl, n-propyl, 2-furyl, 2-thienyl or -C(=O)-CH₃,
R¹¹ represents H, methyl, ethyl or tert-butyl,
R¹³ represents methyl,
wherein aryl represents 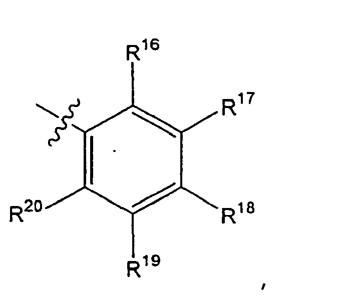
heterocyclyl² represents 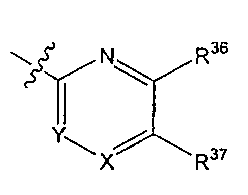 or 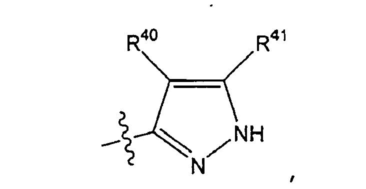
R¹⁶, R¹⁷, R¹⁸, R¹⁹ and R²⁰ each independently of the others represents H, OR²⁸, S(O)ₜR²⁹ wherein t = 0, F, Cl, Br, I, -CN, -NO₂, methyl, ethyl, n-propyl, 2-propyl, n-butyl, 2-butyl, isobutyl, tert-butyl, n-pentyl, neopentyl, isopentyl, n-hexyl, isohexyl, CF₃ or -CO₂R³¹,
R²⁸ R²⁹ and R³¹ each independently of the others represents H, methyl, ethyl, -CF₃ or phenyl,
X-Y represents CR³⁸-CR³⁹, CR³⁸-N or N-CR³⁹,
R³⁶, R³⁷, R³⁸ and R³⁹ each independently of the others represents H, F, Cl, Br, I, -CN, -NO₂, -OH, -SH, C₁₋₆-alkyl or -CF₃,
and
R⁴⁰ and R⁴¹ each independently of the other represents H, F, Cl, Br, I, -CN, -OH, -O-C₁₋₆-alkyl, -SH, -S-C₁₋₆-alkyl, C₁₋₈-alkyl, CO₂-C₁₋₆-alkyl or -N=N-aryl.
which process is **characterised in that**
an amine having the general structure (II)
R¹-NH² II,
wherein R¹ is as defined above in this claim,
is reacted, under the action of an acid, with a glyoxalic acid derivative having the general structure (III) 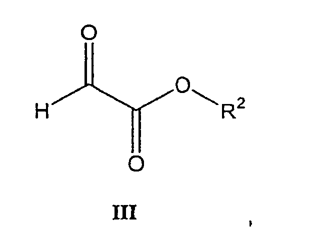 wherein R² is as defined above in this claim,
and with a heterocycle having the general structure (IV) 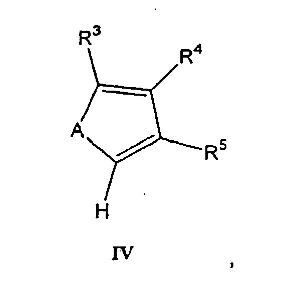 wherein A, R³, R⁴ and R⁵ are as defined above in this claim.

8. Process according to claim 7, **characterised in that** it is carried out as a one-pot process.

9. Process according to either claim 7 or claim 8, **characterised in that** the reaction of the compounds (II), (III) and (IV) is carried out under the action of microwave radiation.

10. Process according to any one of claims 7 to 9, **characterised in that** the process is carried out in an organic solvent at a temperature of from 0°C to 100°C, especially from 15°C to 50°C.

11. Medicament containing a compound having the general structure (I), or a pharmaceutically acceptable salt thereof, 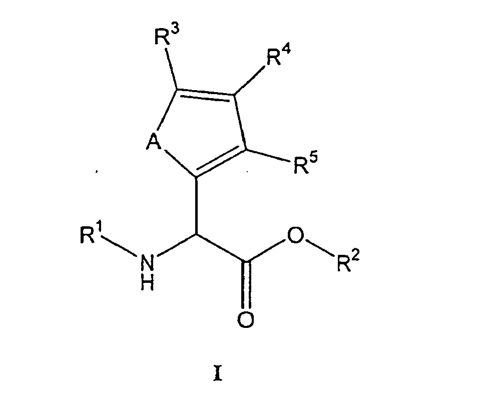 wherein
A represents oxygen or sulfur,
R¹ represents aryl or heterocyclyl²,
R² represents H, methyl, ethyl, n-propyl, 2-propyl, n-butyl, tert-butyl, n-hexyl,
R³, R⁴ and R⁵ each independently of the others represents H, OH, SH, Br, Cl, C₁₋₆-alkyl, 2-furyl, 3-furyl, 2-thienyl, 3-thienyl, -SiR⁶R⁷R⁸, -CH₂OH, -CH₂-O-(C=O)-CH₃, -(CH₂)-Sₚ-(CH₂)_{q}-R¹⁰ wherein p = 1 or 2 and q = 0 or 1, (CH₂)ᵣ-CO₂R¹¹ wherein r = 0 or 1, or -COR¹³,
R⁶, R⁷ and R⁸ each independently of the others represents methyl, tert-butyl or phenyl,
R¹⁰ represents H, methyl, ethyl, n-propyl, 2-furyl, 2-thienyl or -C(=O)-CH₃,
R¹¹ represents H, methyl, ethyl or tert-butyl,
R¹³ represents methyl,
wherein aryl represents 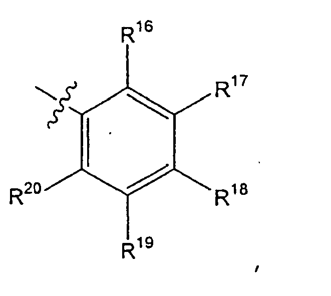
heterocyclyl² represents 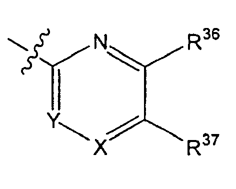 or 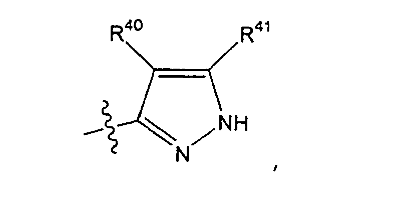
R¹⁶, R¹⁷, R¹⁸, R¹⁹ and R²⁰ each independently of the others represents H, OR²⁸, S(O)ₜR²⁹ wherein t = 0, F, Cl, Br, I, -CN, -NO₂, methyl, ethyl, n-propyl, 2-propyl, n-butyl, 2-butyl, isobutyl, tert-butyl, n-pentyl, neopentyl, isopentyl, n-hexyl, isohexyl, CF₃ or -CO₂R³¹,
R²⁸, R²⁹ and R³¹ each independently of the others represents H, methyl, ethyl, -CF₃ or phenyl,
X-Y represents CR³⁸-CR³⁹, CR³⁸-N or N-CR³⁹,
R³⁶, R³⁷, R³⁸ and R³⁹ each independently of the others represents H, F, Cl, Br, I, -CN, -NO₂, -OH, -SH, C₁₋₆-alkyl or -CF₃,
and
R⁴⁰ and R⁴¹ each independently of the other represents H, F, Cl, Br, I, -CN, -OH, -O-C₁₋₆-alkyl, -SH, -S-C₁₋₆-alkyl, C₁₋₈-alkyl, CO₂-C₁₋₆-alkyl or -N=N-aryl.

12. Use of a compound having the general structure (I) as defined in claim 1, or of a pharmaceutically acceptable salt thereof, in the preparation of a medicament for the treatment of pain.

13. Use of a compound having the general structure (I) as defined in claim 1, or of a pharmaceutically acceptable salt thereof, in the preparation of a medicament for the treatment of migraine.

14. Pharmaceutical composition containing at least one compound having the general structure (I) as defined in claim 1, or a pharmaceutically acceptable salt thereof, as well as at least one pharmaceutical excipient.

## Revendications

1. Composé de formule générale (I), ou sel pharmaceutiquement acceptable de ce composé, 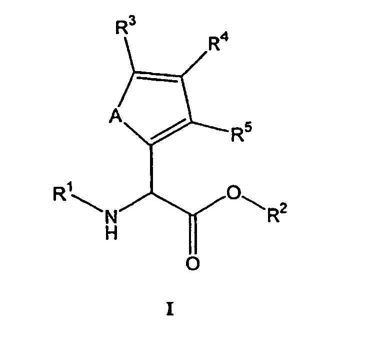 formule dans laquelle
A représente l'oxygène ou le soufre,
R¹ est un reste aryle ou hétérocyclyle²,
R² représente H, un reste méthyle, éthyle, n-propyle, 2-propyle, n-butyle, tertiobutyle, n-hexyle,
R³, R⁴ et R⁵ représentent, indépendamment les uns des autres, H, OH, SH, Br, Cl, un reste alkyle en C₁ à C₆, 2-furyle, 3-furyle, 2-thiényle, 3-thiényle, -SiR⁶R⁷R⁸, -CH₂OH, -CH₂-O-(C=O)-CH₃, -(CH₂)-Sₚ-(CH₂)_{q}-R¹⁰, p ayant la valeur 1 ou 2 et q ayant la valeur 0 ou 1, -(CH₂)ᵣ-CO₂R¹¹, r ayant la valeur 0 ou 1, ou -COR¹³,
R⁶, un R⁷ et R⁸ représentent indépendamment les uns des autres reste méthyle, tertiobutyle ou phényle,
R¹⁰ représente H, un reste méthyle, éthyle, n-propyle, 2-furyle, 2-thiényle ou un groupe -(C=O)-CH₃,
R¹¹ représente H, un reste méthyle, éthyle ou tertiobutyle,
R¹³ représente un reste méthyle,
aryle représentant un groupe 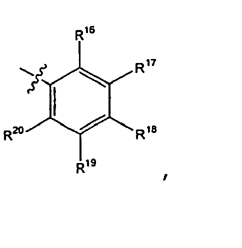
hétérocyclyle² représentant un groupe 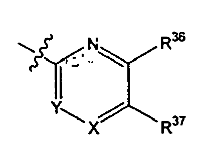 ou 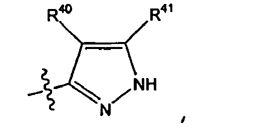
R¹⁶, R¹⁷, R¹⁹ et R²⁰ représentent, indépendamment les uns des autres , H, un groupe OR²⁸, S(O)ₜR²⁹, où t est égal à 0, F, Cl, Br, I, un groupe -CN, -NO₂, un reste méthyle, éthyle, n-propyle, 2-propyle, n-butyle, 2-butyle, isobutyle, tertiobutyle, n-pentyle, néopentyle, isopentyle, n-hexyle, isohexyle, CF₃ ou -CO₂R³¹,
R²⁸, R²⁹ et R³¹ représentent indépendamment les uns des autres H, un reste méthyle, éthyle, -CF₃ ou phényle,
X-Y représente un groupe CR³⁸-CR³⁹, CR³⁸-N ou N-CR³⁹,
R³⁶, R³⁷, R³⁸ et R³⁹ représentent, indépendamment les uns des autres H, F, Cl, Br, I, -CN, -NO₂, -OH, -SH, un reste alkyle en C₁ à C₆ ou un reste -CF₃ et
R⁴⁰ et R⁴¹ représentent, indépendamment l'un de l'autre, H, F, Cl, Br, I, -CN, -OH, -O- (alkyle en C₁ à C₆), -SH, -S-(alkyle en C₁ à C₆), alkyle en C₁ à C₈, CO₂-(alkyle en C₁ à C₆) ou -N=N-aryle,
l'acide (4-méthoxyphénylamino)-thién-2-ylacétique étant exclu.

2. Composé suivant la revendication 1, ou sel pharmaceutiquement acceptable de ce composé, sous forme du racémate, sous forme des énantiomères ou des diastéréo-isomères purs ou sous forme de mélanges des énantiomères ou des diastéréo-isomères, dans un rapport de mélange quelconque.

3. Composé suivant l'une des revendications 1 ou 2, ou l'un de ses sels pharmaceutiquement acceptables, **caractérisé en ce que**
R¹ est un reste aryle ou hétérocyclyle²,
R² représente H, un reste méthyle, éthyle ou tertiobutyle,
R³ représente H, Cl, un reste méthyle, éthyle, n-propyle, 2-propyle, n-butyle, tertiobutyle, un groupe -CH₂OH, -CH₂SH, -CH₂-S-CH₃, -CH₂-S-CH₂-furanne-2-yle, -CH₂-O-(C=O)-CH₃, -CH₂-S-(C=O)-CH₃, -CH₂-S-S-CH₃, -CH₂-S-S-CH₂-furanne-2-yle, -CH₂-CO₂-méthyle ou -CH₂-CO₂-éthyle,
R⁴ représente H, Br, un reste méthyle, éthyle, -CH₂OH, -CO₂-méthyle, -CO₂-éthyle ou -CO-méthyle,
R⁵ représente H, un reste méthyle ou éthyle,
aryle désignant un groupe 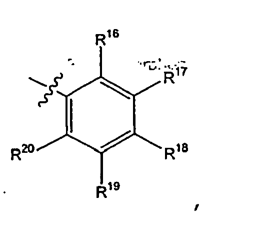
hétérocyclyle² représentant un groupe 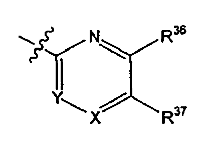 ou 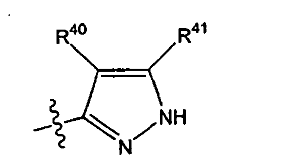
R¹⁶ représente H, un reste -O-phényle, F, Cl, Br, un reste méthyle, éthyle, n-propyle, 2-propyle ou tertiobtyle,
R¹⁷ représente H, Cl, un reste méthyle, éthyle ou CF₃,
R¹⁸ représente H, F, Cl, Br, I, un groupe -CN, -O-CH₃, -O-CF₃, -O-phényle, un reste méthyle, éthyle, n-propyle, 2-propyle, n-butyle, 2-butyle ou tertiobutyle,
R¹⁹ représente H, Cl, Br, un reste méthyle ou éthyle,
R²⁰ représente H, ou un reste méthyle,
X-Y représente un groupe CR³⁸-CR³⁹, R³⁸-N ou N-CR³⁹,
R³⁶ représente H, un reste méthyle ou éthyle,
R³⁷
ou
représente H, un groupe NO₂, Cl, Br, un reste méthyle CF₃,
R³⁸ représente H,
R³⁹ représente H, Cl ou Br,
R⁴⁰ représente H, un groupe -N=N-phényle, -CN, CO₂H, CO₂-méthyle ou CO₂-éthyle,
et
R⁴¹ représente H, OH, SH, S-méthyle, un reste méthyle, éthyle, n-propyle, 2-propyle, n-butyle ou tertiobutyle.

4. Composé suivant l'une des revendications 1 à 3, ou l'un de ses sels pharmaceutiquement acceptables, **caractérisé en ce que**
R¹ représente un groupe 4-trifluorométhoxyphényle, 2-phénoxyphényle, 4-phénoxyphényle, 2-chlorophényle, 4-chlorophényle, 4-iodophényle, 4-cyanophényle, 2-méthylphényle, 2-éthylphényle, 4-éthylphényle, 2-(2-propyl)-phényle, 4-(2-butyl)-phényle, 4-tertiobutylphényle, 2,4-dichlorophényle, 2,3-dichlorophényle, 3,5-dichlorophényle, 2,4-dibromophényle, 4-chloro-2-fluoro-phényle, 2-chloro-4-fluorophényle, 4-bromo-2-chlorophényle, 2-chloro-4-iodophényle, 3-chloro-2-méthylphényle, 4-chloro-2-méthylphényle, 5-chloro-2-méthylphényle, 2-chloro-4-méthylphényle, 4-chloro-3-trifluorométhylphényle, 2,4-dibromo-5-méthylphényle, 5-nitropyridine-2-yle, 3,5-dibromopyridine-2-yle, 3,5-dichloropyridine-2-yle, 3-chloro-5-trifluorométhylpyridine-2-yle, 3,5-dibromo-6-méthylpyridine-2-yle, pyrazine-2-yle, 5-bromopyrimidine-2-yle, 4-carboxyéthylpyÉazole-3-yle, 4-cyanopyrazole-3-yle, 5-tertiobutylpyrazole-3-yle, 5-hydroxy-4-(4-phénylazo)-pyrazole-3-yle ou 4-cyano-5-thiométhylpyrazole-3-yle,
R² représente H ou un reste éthyle,
R³ représente H, Cl, un reste méthyle, éthyle, n-propyle, tertiobutyle, un groupe -CH₂OH, -CH₂SH, -CH₂S-CH₃, -CH₂-S-CH₂-furanne-2-yle, -CH₂-O-(C=)-CH₃, -CH₂-S-(C=O)-CH₃, -CH₂-S-S-CH₃, -CH₂-S-S-CH₂-furanne-2-yle ou -CH₂-CO₂-éthyle,
R⁴ représente H, Br, un reste méthyle, -CH₂OH, -CO₂-méthyle, -CO₂-éthyle ou -C(=O)CH₃ et
R⁵ représente H ou un reste méthyle.

5. Composé suivant l'une des revendications 1 à 4, ou l'un de ses sels pharmaceutiquement acceptables, **caractérisé en ce que**
R¹ est un reste 3,5-dichlorophényle et
R² représente l'hydrogène.

6. Composé suivant l'une des revendications 1 à 5, ou l'un de ses sels pharmaceutiquement acceptables, **caractérisé en ce que** le composé est choisi dans le groupe qui comprend
• l'acide (5-méthylsulfanylméthyl-furanne-2-yl)-(5-nitro-pyridine-2-ylamino)-acétique
• l'acide (5-bromopyrimidine-2-ylamino)-(5-méthylsulfanyl-méthylfuranne-2-yl)-acétique
• l'ester éthylique d'acide 5-[carboxy-(3,5-dichloro-pyridine-2-ylamino)-méthyl]-2-méthylfuranne-3-carboxylique
• l'ester éthylique d'acide 5-[carboxy-(3,5-dibromo-pyridine-2-ylamino)-méthyl]-2-méthylfuranne-3-carboxylique
• l'ester éthylique d'acide 5-[carboxy-(3,5-dibromo-6-méthyl-pyridine-2-ylamino)-méthyl]-2-méthylfuranne-3-carboxylique
• l'acide (3,5-dibromo-6-méthyl-pyridine-2-ylamino)-(4-hydroxyméthyl-furanne-2-yl)-acétique
• l'acide (3,5-dichloropyridine-2-ylamino)-(4-méthyl-thiophène-2-yl)-acétique
• l'acide (2,4-dibromo-5-méthyl-phénylamino)-(4-méthyl-thiophène-2-yl)-acétique
• l'acide (4-méthyl-thiophène-2-yl)-(5-nitropyridine-2-yl-amino)-acétique
• l'acide (3,5-dichloropyridine-2-ylamino)-furanne-2-ylacétique
• l'acide (3,5-dibromopyridine-2-ylamino)-furanne-2-ylacétique
• l'acide (3,5-dibromo-6-méthyl-pyridine-2-ylamino)-furanne-2-ylacétique
• l'acide (3-chloro-5-trifluorométhyl-pyridine-2-ylamino)-furanne-2-ylacétique
• l'acide (5-bromopyrimidine-2-ylamino)-furanne-2-ylacétique
• l'ester méthylique d'acide 5-[(3,5-dichlorophénylamino)-éthoxycarbonyl-méthyl]-2-méthylfuranne-3-carboxylique
• l'ester éthylique d'acide (5-hydroxy-4-phénylazo-1H-pyrazole-3-ylamino)-(5-méthylsulfanylméthyl-furanne-2-yl)-acétique
• l'ester éthylique d'acide 3-{[éthoxycarbonyl-(4-éthoxycarbonyl-5-méthyl-furanne-2-yl)-méthyl]-amino}-1H-pyrazole-4-carboxylique
• l'ester éthylique d'acide 5-[(4-cyano-5-méthylsulfanyl-1H-pyrazole-3-ylamino)-éthoxycarbonyl-méthyl]-2-méthyl-furanne-3-carboxylique
• l'ester éthylique d'acide 5-[(4-cyano-1H-pyrazole-3-ylamino)-éthoxycarbonyl-méthyl]-2-méthyl-furanne-3-carboxylique
• l'ester éthylique d'acide 5-[(4-bromo-2-chlorophénylamino)-éthoxycarbonyl-méthyl]-2-méthyl-furanne-3-carboxylique
• l'ester éthylique d'acide 5-[(4-cyanophénylamino)-éthoxycarbonyl-méthyl]-2-méthyl-furanne-3-carboxylique
• l'ester éthylique d'acide (4-hydroxyméthyl-furanne-2-yl)-(5-hydroxy-4-phénylazo-1H-pyrazole-3-ylamino)-acétique
• l'ester éthylique d'acide (4-cyano-5-méthylsulfanyl-1H-pyrazole-3-ylamino)-(4-hydroxyméthyl-furanne-2-yl)-acétique
• l'ester éthylique d'acide (4-bromo-2-chlorophénylamino)-(4-hydroxyméthyl-furanne-2-yl)-acétique
• l'ester éthylique d'acide (3,5-dichlorophénylamino)-(4-hydroxyméthyl-furanne-2-yl)-acétique
• l'ester éthylique d'acide (5-chloro-2-méthyl-phénylamino)-[5-(furanne-2-ylméthylsulfanylméthyl)-furanne-2-yl]-acétique
• l'acide (2,4-dibromophénylamino)-(4-méthyl-thiophène-2-yl)-acétique
• l'acide (5-chloro-2-méthyl-phénylamino)-(5-méthylsulfanylméthyl-furanne-2-yl)-acétique
• l'acide (2-éthyl-phénylamino)-(5-méthylsulfanylméthyl-furanne-2-yl)-acétique
• l'acide (4-sec.-butyl-phénylamino)-(5-méthylsulfanylméthyl-furanne-2-yl)-acétique
• l'acide (5-méthylsulfanylméthyl-furanne-2-yl)-(4-trifluorométhoxyphénylamino)-acétique
• l'acide (2-isopropyl-phénylamino)-(5-méthylsulfanylméthyl-furanne-2-yl)-acétique
• l'acide (2,4-dibromophénylamino)-(5-méthylsulfanylméthyl-furanne-2-yl)-acétique
• l'acide (4-tertiobutyl-phénylamino)-(5-méthylsulfanyl-méthyl-furanne-2-yl)-acétique
• l'acide (5-chloro-2-méthyl-phénylamino)-[5-(furanne-2-ylméthylsulfanylméthyl)-furanne-2-yl]-acétique
• l'acide (2-éthyl-phénylamino)-[5-(furanne-2-ylméthylsulfanylméthyl)-furanne-2-yl]-acétique
• l'acide (4-sec.-butyl-phénylamino)-[5-(furanne-2-ylméthylsulfanylméthyl)-furanne-2-yl]-acétique
• l'acide [5-(furanne-2-ylméthylsulfanylméthyl)-furanne-2-yl]-(2-isopropyl-phénylamino)-acétique
• l'acide (4-tertiobutyl-phénylamino)-[5-(furanne-2-ylméthylsulfanylméthyl)-furanne-2-yl]-acétique
• l'ester méthylique d'acide 5-[éthoxycarbonyl-(4-iodo-phénylamino)-méthyl]-2-méthyl-furanne-3-carboxylique
• l'ester méthylique d'acide 5-[(4-chloro-2-méthyl-phénylamino)-éthoxycarbonyl-méthyl]-2-méthyl-furanne-3-carboxylique
• l'ester méthylique d'acide 5-[éthoxycarbonyl-(4-phénoxyphénylamino)-méthyl]-2-méthyl-furanne-3-carboxylique
• l'ester éthylique d'acide 5-[éthoxycarbonyl-(4-iodo-phénylamino)-méthyl]-2-méthyl-furanne-3-carboxylique
• l'ester éthylique d'acide 5-[(2-chlorophénylamino)-éthoxycarbonyl-méthyl]-2-méthyl-furanne-3-carboxylique
• l'ester éthylique d'acide 5-[(4-chloro-2-méthyl-phénylamino)-éthoxycarbonyl-méthyl]-2-méthyl-furanne-3-carboxylique
• l'ester éthylique d'acide 5-[(2-chloro-4-fluoro-phénylamino)-éthoxycarbonyl-méthyl]-2-méthyl-furanne-3-carboxylique
• l'ester éthylique d'acide 5-[éthoxycarbonyl-(4-phénoxyphénylamino)-méthyl]-2-méthyl-furanne-3-carboxylique
• l'ester éthylique d'acide 5-[(2,3-dichlorophénylamino)-éthoxycarbonyl-méthyl]-2-méthyl-furanne-3-carboxylique
• l'ester éthylique d'acide (4-hydroxyméthyl-furanne-2-yl)-(4-iodophénylamino)-acétique
• l'ester éthylique d'acide (2,4-dichlorophénylamino)-(4-hydroxyméthyl-furanne-2-yl)-acétique
• l'ester éthylique d'acide (4-chloro-2-méthyl-phénylamino)-(4-hydroxyméthyl-furanne-2-yl)-acétique
• l'ester éthylique d'acide (4-hydroxyméthyl-furanne-2-yl)-(4-phénoxy-phénylamino)-acétique
• l'ester éthylique d'acide (2,3-dichlorophénylamino)-(4-hydroxyméthyl-furanne-2-yl)-acétique
• l'ester éthylique d'acide (2,3-dichlorophénylamino)-furanne-2-ylacétique
• l'ester méthylique d'acide 5-[carboxy-(3,5-dibromo-6-méthyl-pyridine-2-ylamino)-méthyl]-2-méthyl-furanne-3-carboxylique
• l'acide (5-méthylsulfanylméthyl-furanne-2-yl)-(pyrazine-2-ylamino)-acétique
• l'acide (3,5-dibromopyridine-2-ylamino)-(5-méthylsulfanylméthyl-furanne-2-yl)-acétique
• l'acide (3,5-dibromo-6-méthyl-pyridine-2-ylamino)-(5-méthylsulfanylméthyl-furanne-2-yl)-acétique
• l'ester méthylique d'acide 5-[(4-bromo-2-chlorophénylamino)-carboxyméthyl]-2-méthyl-furanne-3-carboxylique
• l'ester méthylique d'acide 5-[carboxy-(4-cyanophénylamino)-méthyl]-2-méthyl-furanne-3-carboxylique
• l'ester méthylique d'acide 5-[carboxy-(3,5-dichlorophénylamino)-méthyl]-2-méthyl-furanne-3-carboxylique
• l'ester éthylique d'acide 5-[carboxy-(2-phénoxyphénylamino)-méthyl]-2,4-diméthyl-furanne-3-carboxylique
• l'ester éthylique d'acide 5-[(4-bromo-2-chlorophénylamino)-carboxyméthyl]-2,4-diméthyl-furanne-3-carboxylique
• l'ester éthylique d'acide 5-[carboxy-(4-cyanophénylamino)-méthyl]-2,4-diméthyl-furanne-3-carboxylique
• l'ester éthylique d'acide 5-[carboxy-(3,5-dichlorophénylamino)-méthyl]-2,4-diméthyl-furanne-3-carboxylique
• l'ester éthylique d'acide 5-[(4-bromo-2-chlorophénylamino)-carboxyméthyl]-2-méthyl-furanne-3-carboxylique
• l'ester éthylique d'acide 5-[carboxy-(4-cyano-phénylamino)-méthyl]-2-méthyl-furanne-3-carboxylique
• l'ester éthylique d'acide 5-[carboxy-(3,5-dichlorophénylamino)-méthyl]-2-méthyl-furanne-3-carboxylique
• l'acide (5-tertiobutyl-1H-pyrazole-3-ylamino)-(4-hydroxyméthyl-furanne-2-yl)-acétique
• l'acide (4-bromo-2-chlorophénylamino)-(3-méthylthiophène-2-yl)-acétique
• l'acide (4-cyano-phénylamino)-(3-méthyl-thiophène-2-yl)-acétique
• l'acide (3,5-dichlorophénylamino)-(3-méthyl-thiophène-2-yl)-acétique
• l'acide (3,5-dichlorophénylamino)-furanne-2-ylacétique
• l'ester éthylique d'acide [5-(furanne-2-ylméthylsulfanylméthyl)-furanne-2-yl]-(4-phénoxyphénylamino)-acétique
• l'ester éthylique d'acide (5-éthoxycarbonylméthylthiophène-2-yl)-(5-hydroxy-4-phénylazo-1H-pyrazole-3-ylamino)-acétique
• l'acide {5-[1-(4-cyano-1H-pyrazole-3-ylamino)-3-méthoxy-2-oxo-propyl]-thiophène-2-yl}-acétique
• l'acide (4-éthyl-phénylamino)-(5-méthylsulfanylméthylfuranne-2-yl)-acétique
• l'acide (3-chloro-2-méthyl-phénylamino)-(5-méthylsulfanylméthyl-furanne-2-yl)-acétique
• l'acide (4-chlorophénylamino)-(5-méthylsulfanylméthylfuranne-2-yl)-acétique
• l'acide (5-méthylsulfanylméthyl-furanne-2-yl)-o-tolyl-amino-acétique
• l'ester éthylique d'acide 5-[carboxy-(4-chloro-3-trifluorométhyl-phénylamino)-méthyl]-2-méthyl-furanne-3-carboxylique
• l'ester éthylique d'acide 5-[carboxy-(2-chlorophénylamino)-méthyl]-2-méthyl-furanne-3-carboxylique
• l'ester éthylique d'acide 5-[carboxy-(2-chloro-4-fluorophénylamino)-méthyl]-2-méthyl-furanne-3-carboxylique
• l'ester éthylique d'acide 5-[carboxy-(4-chloro-2-fluorophénylamino)-méthyl]-2-méthyl-furanne-3-carboxylique
• l'ester éthylique d'acide 5-[carboxy-(2,3-dichlorophénylamino)-méthyl]-2-méthyl-furanne-3-carboxylique
• l'acide (2,4-dichlorophénylamino)-(4-méthyl-thiophène-2-yl)-acétique
• l'acide (4-chloro-3-trifluorométhyl-phénylamino)-(4-méthyl-thiophène-2-yl)-acétique
• l'acide (2,4-dichlorophénylamino)-furanne-2-ylacétique
• l'acide (4-chloro-3-trifluorométhyl-phénylamino)-furanne-2-ylacétique
• l'acide (4-iodophénylamino)-(5-méthylsulfanylméthylfuranne-2-yl)-acétique
• l'acide (2,4-dichlorophénylamino)-(5-méthylsulfanylméthyl-furanne-2-yl)-acétique
• l'acide (4-chloro-3-trifluorométhyl-phénylamino)-(5-méthylsulfanylméthyl-furanne-2-yl)-acétique
• l'acide (2-chlorophénylamino)-(5-méthylsulfanylméthylfuranne-2-yl)-acétique
• l'acide (4-chloro-2-méthyl-phénylamino)-(5-méthylsulfanylméthyl-furanne-2-yl)-acétique
• l'acide (2-chloro-4-fluorophénylamino)-(5-méthylsulfanylméthyl-furanne-2-yl)-acétique
• l'acide (2-chloro-4-méthyl-phénylamino)-(5-méthylsulfanylméthyl-furanne-2-yl)-acétique
• l'acide (2,3-dichlorophénylamino)-(5-méthylsulfanylméthyl-furanne-2-yl)-acétique
• l'acide [5-(furanne-2-ylméthylsulfanylméthyl)-furanne-2-yl]-(4-iodophénylamino)-acétique
• l'acide (4-chloro-3-trifluorométhyl-phénylamino)-[5-(furanne-2-ylméthylsulfanylméthyl)-furanne-2-yl]-acétique
• l'acide (4-chloro-2-méthyl-phénylamino)-[5-(furanne-2-ylméthylsulfanylméthyl)-furanne-2-yl]-acétique
• l'acide (2-chloro-4-méthyl-phénylamino)-[5-(furanne-2-ylméthylsulfanylméthyl)-furanne-2-yl]-acétique
• l'acide (4-chloro-3-trifluorométhyl-phénylamino)-(5-éthoxycarbonylméthyl-thiophène-2-yl)-acétique
• l'acide (4-acétyl-3,5-diméthyl-furanne-2-yl)-(2-chloro-4-méthyl-phénylamino)-acétique
• l'acide (4-acétyl-3,5-diméthyl-furanne-2-yl)-(2,3-dichlorophénylamino)-acétique
• l'acide (3,5-dichlorophénylamino)-(5-méthylsulfanylméthyl-furanne-2-yl)-acétique
• l'acide (3-chlorophénylamino)-(5-mercaptométhyl-furanne-2-yl)-acétique
• l'acide (3,4-dichlorophénylamino)-(5-mercaptométhyl-furanne-2-yl)-acétique
• l'acide (3,5-dichlorophénylamino)-(5-mercaptométhyl-furanne-2-yl)-acétique
• l'acide (3,5-dichlorophénylamino)-(5-méthyl-thiophène-2-yl)-acétique
• l'acide (3,5-dichlorophénylamino)-(5-hydroxyméthyl-thiophène-2-yl)-acétique
• l'acide (5-acétylsulfanylméthyl-furanne-2-yl)-(3,5-dichlorophénylamino)-acétique
• l'acide (3,5-dichlorophénylamino)-(5-éthyl-thiophène-2-yl)-acétique
• l'acide (3,5-dichlorophénylamino)-(5-n-propyl-thiophène-2-yl)-acétique
• l'acide (3,5-dichlorophénylamino)-[5-(furanne-2-ylméthylsulfanylméthyl)-furanne-2-yl]-acétique
• l'acide (3-chlorophénylamino)-(5-mercaptométhyl-furanne-2-yl)-acétique
• l'acide (3,4-dichlorophénylamino)-(5-mercaptométhyl-furanne-2-yl)-acétique
• l'ester éthylique d'acide (3,5-dichlorophénylamino)-(thiophène-2-yl)-acétique
• l'ester éthylique d'acide (4-bromofuranne-2-yl)-(3,5-dichlorophénylamino)-acétique
• l'ester éthylique d'acide (3,5-dichlorophénylamino)-(5-propylthiophène-2-yl)-acétique
• l'ester éthylique d'acide (3,5-dichlorophénylamino)-(3-méthylthiophène-2-yl)-acétique
• l'acide (5-tertiobutylfuranne-2-yl)-(3,5-dichlorophénylamino)-acétique
• l'acide (3,5-dichlorophénylamino)-[5-(furanne-2-yl-méthyl-disulfanylméthyl)-furanne-2-yl]-acétique
• l'acide (3,5-dichlorophénylamino)-(5-méthyldisulfanylméthylfuranne-2-yl)-acétique
• l'acide (3,5-dichlorophénylamino)-[5-(furanne-2-ylméthylsulfanylméthyl)-furanne-2-yl]-acétique
• l'acide 5-acétoxyméthylfuranne-2-yl-(3,5-dichlorophénylamino)-acétique
• l'acide (3,5-dichlorophénylamino)-thiophène-3-ylacétique
• l'acide (3,5-dichlorophénylamino)-(5-méthylthiophène-2-yl)-acétique
• l'acide (3,5-dichlorophénylamino)-(4-méthylthiophène-2-yl)-acétique
• l'acide (5-chlorothiophène-2-yl)-(3,5-dichlorophénylamino)-acétique.

7. Procédé de production d'un composé de formule générale (I) ou de l'un de ses sels pharmaceutiquement acceptables 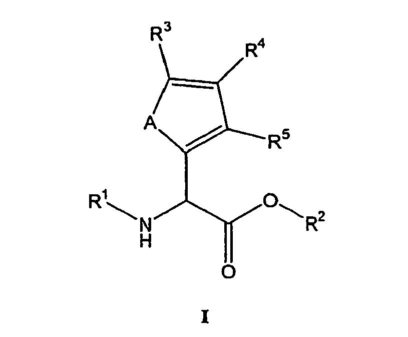 formule dans laquelle
A représente l'oxygène ou le soufre,
R¹ est un reste aryle ou hétérocyclyle²,
R² représente H, un reste méthyle, éthyle, n-propyle, 2-propyle, n-butyle, tertiobutyle, n-hexyle,
R³, R⁴ et R⁵ représentent, indépendamment les uns des autres, H, OH, SH, Br, Cl, un reste alkyle en C₁ à C₆, 2-furyle, 3-furyle, 2-thiényle, 3-thiényle, -SiR⁶R⁷R⁸, -CH₂OH, -CH₂-O-(C=O)-CH₃, -(CH₂)-Sₚ-(CH₂)_{q}-R¹⁰, p ayant la valeur 1 ou 2 et q ayant la valeur 0 ou 1, -(CH₂)ᵣ-CO₂R¹¹, r ayant la valeur 0 ou 1, ou -COR¹³,
R⁶, R⁷ et R⁸ représentent indépendamment les uns des autres un reste méthyle, tertiobutyle ou phényle,
R¹⁰ représente H, un reste méthyle, éthyle, n-propyle, 2-furyle, 2-thiényle ou un groupe -(C=O)-CH₃,
R¹¹ représente H, un reste méthyle, éthyle ou tertiobutyle,
R¹³ représente un reste méthyle,
aryle représentant un groupe 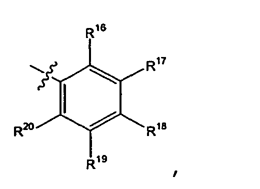
hétérocyclyle² représentant un groupe 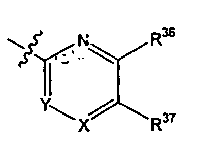 ou 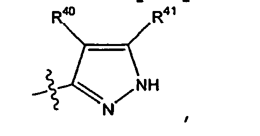
R¹⁶, R¹⁷, R¹⁹ et R²⁰ représentent, indépendamment les uns des autres , H, un groupe OR²⁸, S(O)ₜR²⁹, où t est égal à 0, F, Cl, Br, I, un groupe -CN, -NO₂, un reste méthyle, éthyle, n-propyle, 2-propyle, n-butyle, 2-butyle, isobutyle, tertiobutyle, n-pentyle, néopentyle, isopentyle, n-hexyle, isohexyle, CF₃ ou -CO₂R³¹,
R²⁸, R²⁹ et R³¹ représentent indépendamment les uns des autres H, un reste méthyle, éthyle, -CF₃ ou phényle,
X-Y représente un groupe CR³⁸-CR³⁹, CR³⁸-N ou N-CR³⁹,
R³⁶, R³⁷, R³⁸ et R³⁹ représentent, indépendamment les uns des autres H, F, Cl, Br, I, -CN, -NO₂, -OH, -SH, un reste alkyle en C₁ à C₆ ou un reste -CF₃ et
R⁴⁰ et R⁴¹ représentent, indépendamment l'un de l'autre, H, F, Cl, Br, I, -CN, -OH, -O-(alkyle en C₁ à C₆), -SH, -S-(alkyle en C₁ à C₆), alkyle en C₁ à C₈, CO₂-(allyle en C₁ à C₆) ou -N=N-aryle,
**caractérisé en ce qu'**une amine de formule générale (II)
R¹-NH² II
dans laquelle R¹ est tel que défini ci-dessus dans cette revendication,
est amenée à réagir avec un dérivé d'acide glyoxylique de formule générale (III) 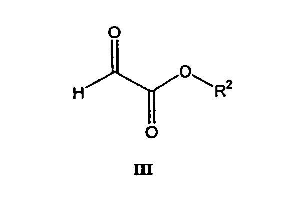 dans laquelle R² est tel que défini ci-dessus dans cette revendication, et un hétérocycle de formule générale (IV) 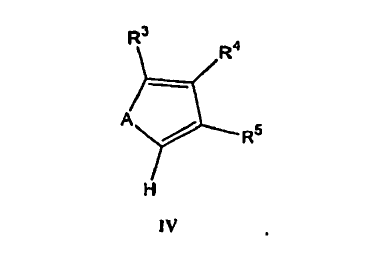 dans laquelle A, R³, R⁴ et R⁵ sont tels que définis ci-dessus dans cette revendication, sous l'action d'un acide.

8. Procédé suivant la revendication 7, **caractérisé ce qu'**il est mis en oeuvre comme procédé en récipient unique.

9. Procédé suivant la revendication 7 ou 8, **caractérisé en ce que** la réaction des composés (II) (III) et (IV) est conduite sous l'action d'un rayonnement de micro-ondes.

10. Procédé suivant l'une des revendications 7 à 9, **caractérisé en ce qu'**il est mis en oeuvre dans un solvant organique à une température de 0°C à 100°C, en particulier de 15°C à 50°C.

11. Médicament contenant un composé de formule générale (I), ou l'un de ses sels pharmaceutiques, 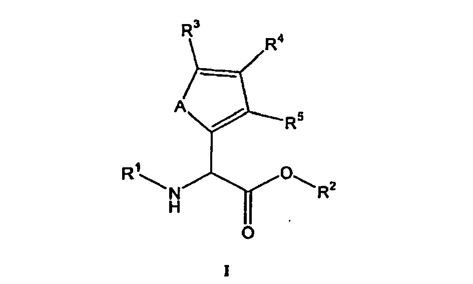 formule dans laquelle
A représente l'oxygène ou le soufre,
R¹ est un reste aryle ou hétérocyclyle²,
R² représente H, un reste méthyle, éthyle, n-propyle, 2-propyle, n-butyle, tertiobutyle, n-hexyle,
R³, R⁴ et R⁵ représentent, indépendamment les uns des autres, H, OH, SH, Br, Cl, un reste alkyle en C₁ à C₆, 2-furyle, 3-furyle, 2-thiényle, 3-thiényle, -SiR⁶R⁷R⁸, -CH₂OH, -CH₂-O-(C=O)-CH₃, -(CH₂)-Sₚ-(CH₂)_{q}-R¹⁰, p ayant la valeur 1 ou 2 et q ayant la valeur 0 ou 1, -(CH₂)ᵣ-CO₂R¹¹, r ayant la valeur 0 ou 1, ou -COR¹³,
R⁶, un R⁷ et R⁸ représentent indépendamment les uns des autres reste méthyle, tertiobutyle ou phényle,
R¹⁰ représente H, un reste méthyle, éthyle, n-propyle, 2-furyle, 2-thiényle ou un groupe -(C=O)-CH₃,
R¹¹ représente H, un reste méthyle, éthyle ou tertiobutyle,
R¹³ représente un reste méthyle,
aryle représentant un groupe 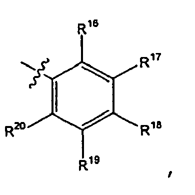
hétérocyclyle² représentant un groupe 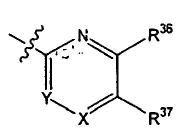 ou 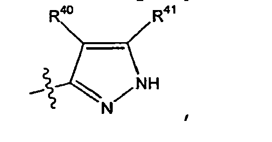
R¹⁶, R¹⁷, R¹⁹ et R²⁰ représentent, indépendamment les uns des autres , H, un groupe OR²⁸, S(O)ₜR²⁹, où t est égal à 0, F, Cl, Br, I, un groupe -CN, -NO₂, un reste méthyle, éthyle, n-propyle, 2-propyle, n-butyle, 2-butyle, isobutyle, tertiobutyle, n-pentyle, néopentyle, isopentyle, n-hexyle, isohexyle, CF₃ ou -CO₂R³¹,
R²⁸, R²⁹ et R³¹ représentent indépendamment les uns des autres H, un reste méthyle, éthyle, -CF₃ ou phényle,
X-Y représente un groupe CR³⁸-CR³⁹, CR³⁸-N ou N-CR³⁹,
R³⁶, R³⁷, R³⁸ et R³⁹ représentent, indépendamment les uns des autres H, F, Cl, Br, I, -CN, -NO₂, -OH, -SH, un reste alkyle en C₁ à C₆ ou un reste -CF₃ et
R⁴⁰ et R⁴¹ représentent, indépendamment l'un de l'autre, H, F, Cl, Br, I, -CN, -OH, -O-(alkyle en C₁ à C₆), -SH, -S-(alkyle en C₁ à C₆), alkyle en C₁ à C₈, CO₂-(alkyle en C₁ à C₆) ou -N=N-aryle.

12. Utilisation d'un composé de formule générale (I) telle que définie dans la revendication 1, ou de l'un de ses sels pharmaceutiquement acceptables, pour la préparation d'un médicament destiné au traitement de la douleur.

13. Utilisation d'un composé de formule générale (I) telle que définie dans la revendication 1, ou de l'un de ses sels pharmaceutiquement acceptables pour la préparation d'un médicament destiné au traitement de la migraine.

14. Composition pharmaceutique, qui contient au moins un composé de formule générale (I) telle que définie dans la revendication 1, ou l'un de ses sels pharmaceutiquement acceptables, ainsi qu'au moins une substance auxiliaire pharmaceutique.
